(19) Europäisches Patentamt
European Patent Office
Office européen des brevets

(11) Publication number: 0 035 046
B1

(12) # EUROPEAN PATENT SPECIFICATION

(45) Date of publication of patent specification: 02.10.85

(21) Application number: 80101038.0

(22) Date of filing: 03.03.80

(51) Int. Cl.$^4$: C 07 D 257/04, C 07 D 401/12, C 07 D 403/12, C 07 D 405/12, C 07 D 409/12, C 07 D 413/12, C 07 D 417/12, A 61 K 31/41

(54) **Novel tetrazole derivatives, process for the preparation thereof, and anti-ulcer composition containing the same.**

(43) Date of publication of application:
09.09.81 Bulletin 81/36

(45) Publication of the grant of the patent:
02.10.85 Bulletin 85/40

(84) Designated Contracting States:
BE CH DE FR GB IT NL SE

(56) References cited:
EP-A-0 035 228
DD-A- 105 224
DD-A- 106 645
DE-A-1 670 183
DE-A-2 748 207
US-A-3 374 145
US-A-3 743 646
US-A-3 767 667
US-A-4 044 144
US-A-4 110 338

The file contains technical information submitted after the application was filed and not included in this specification

(73) Proprietor: OTSUKA PHARMACEUTICAL CO., LTD.
9, Kandatsukasacho 2-chome
Chiyoda-ku Tokyo 101 (JP)

(72) Inventor: Uchida, Minoru
No.11, Oohayashi-cho Aza-honson
Komatsujima-shi Tokushima-Ken (JP)
Inventor: Nakagawa, Kazuyuki
No.774, Kawauchi-cho Oomatsu
Tokushima-shi Tokushima-ken (JP)
Inventor: Nishi, Takao No. 2-28, Kitajima-cho
Tarohachisu Aza-sotobiraki
Itano-gun Tokushima-ken (JP)

(74) Representative: von Kreisler, Alek, Dipl.-Chem. et al
Deichmannhaus am Hauptbahnhof
D-5000 Köln 1 (DE)

(56) References cited:
J. Org. Chem. 21, 311-315 (1956)
Can. J. Chem. 47, 813-819 (1969)
J. Org. Chem. 21, 767 (1956)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 035 046 B1

Courier Press, Leamington Spa, England.

**Description**

The present invention relates to novel tetrazole derivatives, process for the preparation of the tetrazole derivatives, and a pharmaceutical composition for treating peptic and duodenal ulcers which contains as the essential ingredient the tetrazole derivatives.

The compounds of the present invention are tetrazole derivatives of the formula:

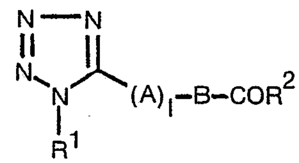

[I]

wherein $R^1$ is hydrogen, an alkyl having 1 to 6 carbon atoms, a cycloalkyl having 3 to 12 carbon atoms or phenyl; A is sulfur or an alkylene-thio wherein the alkylene moiety has 1 to 6 carbon atoms and the thio group bonds to the group B; l is 0 or 1; B is an alkylene having 1 to 6 carbon atoms; $R^2$ is hydroxy, an alkoxy having 1 to 6 carbon atoms, or a group:

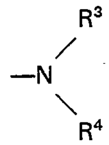

wherein $R^3$and $R^4$are the same or different and are each hydrogen, an alkyl having 1 to 6 carbon atoms, a cycloalkyl having 3 to 12 carbon atoms, phenyl, a cycloalkylalkyl wherein the cycloalkyl moiety has 3 to 8 carbon atoms and the alkyl moiety has 1 to 6 carbon atoms, a phenyl-alkyl wherein the alkyl moiety has 1 to 6 carbon atoms, a hydroxyalkyl having 1 to 6 carbon atoms, a saturated or unsaturated heterocyclic group selected from the group consisting of pyridyl, pyrrolyl, pyrrolidinyl, 3-pyrrolinyl, dihydropyridyl, piperidinyl, 2-pyrazolinyl, 2-imidazolinyl, imidazolyl, imidazolidinyl, pyrazolyl, pyrimidinyl, pyridazinyl, pyrazinyl, piperazinyl, oxazolyl, oxazolidinyl, isoxazolyl, 4H-1,4-oxazinyl, morpholinyl, thiazolyl, thiazolidinyl, isothiazolyl, 4H-1,4-thiazinyl, furyl, tetrahydrofuryl, 2H-pyranyl, 4H-pyranyl, 3,4-dihydro-2H-pyranyl, tetrahydropyranyl, thienyl, tetrahydrothienyl, thianyl and 1,4-dithianyl, or an alkyl having 1 to 6 carbon atoms which is substituted by said saturated or unsaturated heterocyclic group, or the $R^3$ and $R^4$ may combine together with the nitrogen atom to which they are joined with or without being intervened with oxygen or nitrogen to form a saturated or unsaturated heterocyclic group selected from the group consisting of piperidino, pyrrolidino, piperazino, 1-imidazolyl, 1-pyrazolyl, 4,5-dihydropyrazol-1-yl, 1-pyrrolyl, 4H-1,4-oxazin-4-yl, and 3-tetrahydrooxazolyl which heterocyclic group may be substituted with an alkyl having 1 to 6 carbon atoms or an alkanoyl having 1 to 6 carbon atoms; said cycloalkyl, phenyl and phenyl-alkyl in the substituents $R^1$, $R^3$ and $R^4$ may have on the cycloalkyl or phenyl ring one or two substituents selected from the group consisting of an alkoxy having 1 to 6 carbon atoms, an alkyl having 1 to 6 carbon atoms, a halogen, an N,N-dialkyamino wherein each alkyl moiety has 1 to 6 carbon atoms, nitro, aminosulfonyl, hydroxy and an alkanoyloxy having 1 to 6 carbon atoms; provided that

a) when l is 0 and $R^1$ is hydrogen or an alkyl having 1 to 6 carbon atoms, $R^2$ is other than the groups selected from hydroxy, an alkoxy having 1 to 6 carbon atoms and amino;

b) when l is 0 and $R^1$ is n-butyl, B is not methylene and $R^2$ is not ethylamino;

c) when l is 0 and $R^1$ is phenyl and B is methylene, $R^2$ is other than the groups selected from hydroxy, ethoxy, amino, methylamino and benzylamino;

d) when l is 0 and $R^1$ is phenyl and B is ethylmethylene, $R^2$ is not hydroxy, ethoxy or amino;

e) when l is 0 and $R^1$ is cyclohexyl and B is ethylene or methylmethylene, $R^2$ is not amino;

f) when l is 0 and $R^1$ is cyclohexyl and B is dimethylmethylene, $R^2$ is other than the groups selected from amino, diethylamino, benzylamino and morpholino; and

g) when l is 1 and $R^1$ is hydrogen, an alkyl group having 1 to 6 carbon atoms, a cycloalkyl group having 3 to 12 carbon atoms or phenyl and A is S and B is alkylene having 1 to 6 carbon atoms and $R^2$ is a

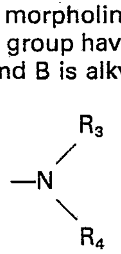

—group, $R^3$ and $R^4$ are not combined together with the nitrogen atom to which they are joined to form a 1-imidazolyl- or 1-pyrrolyl-group, and a pharmaceutically acceptable salt thereof.

US—A 3 767 667 discloses a process for preparing 1H-tetrazole compounds, in which the nitrogen in 1-position and the only carbon atom in the 5-membered heterocyclic ring can be substituted by numerous

groups. The tetrazole compounds are disclosed to be useful as starting materials for preparing antibiotics.

In US—A 3 743 646, there are described tetrazole derivatives, in which the nitrogen in the 2-position is substituted by a monohalophenyl-residue, whereby the carbon atom in the heterocyclic ring is substituted by a propionic acid amide residue. These amides show antiinflammatory properties, and, in addition possess the property of producing less gastric irritation than the corresponding acids.

US—A 3 374 145 discloses a pharmaceutical preparation having antiinflammatory activity containing 5-amino-1-phenyl-tetrazole. This preparation is useful in treating various inflammatory diseases.

J. Org. Chem. *21*, 311 (1956) describes the preparation of 1- and 5-tetrazolyl acidic acids, wherein several 1-substituted and 5-tetrazolyl acidic esters and acids as well as corresponding amides have been prepared.

In Can. J. Chem. *47*, 813 (1969) there is described the synthesis of (inter alia) 5-tetrazolyl acetic acids and their methyl esters, which may be substituted by lower alkyl groups at the nitrogen atom of the heterocyclic ring in 1-position. These compounds are described as precursors for penicillin derivatives.

The present invention does not include 5-tetrazolyl derivatives which are mentioned in one of the documents above.

In J. Org. Chem. *21*, 715 (1956) there is described a preparation of a series of 55 tetrazol acid derivatives, most of them having no significant physiologic action other than toxic manifestations at the higher dose levels. Only few compounds were physiologically acitive insofar as they produce signs of central nervous system depression.

The compounds of the present invention have prophylactic or therapeutic activities agaist peptic and/or duodenal ulcers and are useful as a medicine for treating peptic and/or duodenal ulcers in human and other animals. The compounds have also an anti-inflammatory activity and are useful as an anti-inflammatory drug.

In the present specification, the term "alkyl having 1 to 6 carbon atoms" denotes a straight or branched alkyl group and includes, for example, methyl, ethyl, propyl, isopropyl, butyl, tert-butyl, pentyl and hexyl. The term "cycloalkyl" denotes a cycloalkyl group having 3 to 12 carbon atoms and includes, for example, cyclopropyl, cyclobutyl, cyclopentyl, cyclohexyl, cycloheptyl, cyclooctyl, cyclononyl, cyclodecanyl, cycloundecanyl, and cyclododecanyl. The term "alkylenethio having 1 to 6 carbon atoms" denotes a group formed by combining a straight or branched lower alkylene having 1 to 6 carbon atoms and a sulfur atom, wherein one terminal carbon atom of the alkylene is bonded to the carbon atom at 5-position of tetrazole ring and another terminal sulfur atom is bonded to the carbon atom at the terminal of the alkylene group "B" and includes, for example, methylenethio, ethylenethio, trimethylenethio, tetramethylenethio, pentamethylenethio, hexamethylenethio, 2-methyltrimethylenethio, 2,2-dimethyltrimethylenethio, and 1-methyltrimethylenethio. The term "alkylene having 1 to 6 carbon atoms" denotes a straight or branched alkylene and includes, for example, methylene, ethylene, trimethylene, tetramethylene, pentamethylene, hexamethylene, methylmethylene, 2-methyltrimethylene, 2,2-dimethyltrimethylene, and 1-methyltrimethylene.

The term "cycloalkyl alkyl" denotes a group formed by combining a straight or branched alkylene having 1 to 6 carbon atoms with a cycloalkyl having 3 to 8 carbon atoms and includes, for example, cyclopropylmethyl, cyclobutylmethyl, cyclopentylmethyl, cyclohexylmethyl, cycloheptylmethyl, cyclooctylmethyl, 2-cyclohexylethyl, 1-cyclohexylethyl, 3-cyclohexylpropyl, 1,1-dimethyl-2-cyclohexylethyl, 4-cyclohexylbutyl, 5-cyclohexylpentyl, and 6-cyclohexylhexyl. The term "phenylalkyl" denotes a group formed by combining a straight or branched alkylene having 1 to 6 carbon atoms with a phenyl group and includes, for example, benzyl, 1-phenylethyl, 2-phenylethyl, 3-phenylpropyl, 4-phenylbutyl, 1,1-dimethyl-2-phenylethyl, 5-phenylpentyl, and 6-phenylhexyl. The term "hydroxyalkyl" denotes a group formed by combining a straight or branched lower alkyl having 1 to 6 carbon atoms with a hydroxy group and includes, for example, hydroxymethyl, 2-hydroxyethyl, 3-hydroxypropyl, 2-hydroxypropyl, 4-hydroxybutyl, 1,1-dimethyl-2-hydroxyethyl, 5-hydroxypentyl, and 6-hydroxyhexyl.

The term "heterocyclic group" denotes a 5- or 6-membered saturated or unsaturated heterocyclic group containing one or two hetero atoms selected from the group consisting of nitrogen, oxygen and sulfur and includes, for example, pyridyl, pyrrolyl, pyrrolidinyl, 3-pyrrolinyl, dihydropyridyl, piperidinyl, 2-pyrazolinyl, 2-imidazolinyl, imidazolyl, imidazolidinyl, pyrazolyl, pyrimidinyl, pyridazinyl, pyrazinyl, piperazinyl, oxazolyl, oxazolidinyl, isoxazolyl, 4H-1,4-oxazinyl, morpholinyl, thiazolyl, thiazolidinyl, isothiazolyl, 4H-1,4-thiazinyl, furyl, tetrahydrofuryl, 2H-pyranyl, 4H-pyranyl, 3,4-dihydro-2H-pyranyl, tetrahydropyranyl, thienyl, tetrahydrothienyl, thianyl, and 1,4-dithianyl.

The term "alkyl substituted by said saturated or unsaturated heterocyclic group" denotes a group formed by combining a straight or branched alkylene having 1 to 6 carbon atoms with a 5- or 6-membered saturated or unsaturated heterocyclic group containing one or two hetero atoms selected from the group consisting of nitrogen, oxygen and sulfur and includes, for example, 2-pyridylmethyl, 2-(3-pyridyl)ethyl, 4-(4-pyridyl)butyl, 2-thienylmethyl, 2-(3-thienyl)ethyl, 2-pyrimidinylmethyl, 2-tetrahydropyranylmethyl, 3-pyrrolylmethyl, 2-(2-pyrrolyl)ethyl, 3-pyrrolidinylmethyl, 2-(2-pyrrolidinyl)ethyl, 2-(3-pyrrolinyl)methyl, 4-piperidinylmethyl, 2-(2-piperidinyl)ethyl, 4-dihydropyridylmethyl, 3-piperidinylmethyl, piperidinomethyl, 2-pyrazinylmethyl, 3-pyrazolylmethyl, 2-(4-pyrazolyl)ethyl, 2-imidazolylmethyl, 4-(2-imidazolyl)butyl, 2-(4-imidazolyl)ethyl, 2-imidazolidinylmethyl, 3-(4-imidazolidinyl)propyl, 3-pyridazinylmethyl, 2-(4-pyridazinyl)ethyl, 2-pyrazinylmethyl, 4-(2-pyrazinyl)-butyl, 2-(4-pyrimidinyl)ethyl, 6-(2-pyrimidinyl)hexyl, 2-pyrimidinylmethyl, 4-piperazinylmethyl, 3-(2-piperazinyl)propyl, piperazinomethyl, 2-oxazolylmethyl, 2-(4-oxazolyl)ethyl, 2-oxazolidinylmethyl, 4-isoxazolylmethyl, 2-(4H-1,4-oxazinyl)methyl, 3-morpholinylmethyl, morpholinomethyl, 2-thiazolylmethyl, 4-(4-thiazolyl)butyl, 2-thiazolidinylmethyl, 2-(4-thiazolidinyl)ethyl, 3-isothiazolylmethyl, 2-(4H-1,4-thiazinyl)methyl, furfuryl, 2-(3-furyl)-ethyl, 2-tetrahydrofurylmethyl, 2H-pyran-2-ylmethyl, 2H-pyran-4-ylmethyl, 4H-pyran-4-ylmethyl, 4H-pyran-3-ylmethyl, 2-(2-tetrahydropyranyl)ethyl, 2-(3-tetrahydropyranyl)ethyl, 4-(4-tetrahydropyranyl)butyl, 2-thienylmethyl, 2-(3-thienyl)ethyl, 2-(2-tetrahydrothienyl)ethyl, 3-thienylmethyl, 2-thianylmethyl, 2-(3-thianyl)ethyl, 4-thianylmethyl, and 1,4-dithian-2ylmethyl.

The "saturated or unsaturated heterocyclic group formed by combining the groups $R^3$ and $R^4$ together with the nitrogen atom to which they are joined with or without being intervened with oxygen or nitrogen" is a 5- or 6-membered saturated or unsaturated heterocyclic group and includes, for example, piperidino, pyrrolidino, piperazino, morpholino, 1-imidazolyl, 1-pyrazolyl, 4,5-dihydropyrazol-1-yl, 1-pyrrolyl, 4H-1,4-oxazin-4-yl, and 3-tetrahydrooxazolyl.

The term "alkoxy" denotes a straight or branched alkoxy group having 1 to 6 carbon atoms and includes, for example, methoxy, ethoxy, propoxy, isopropoxy, butoxy, tert-butoxy, pentyloxy, and hexyloxy. The term "halogen" includes fluorine, chlorine, bromine, and iodine. The term "N,N-dialkylamino" denotes an N,N-dialkylamino having 1 to 6 carbon atoms in each alkyl moiety and includes, for example, N,N-dimethylamino, N,N-diethylamino, N-methyl-N-ethylamino, N,N-dipropylamino, N,N-diisopropylamino, N,N-dibutylamino, N-methyl-N-tert-butylamino, N,N-dipentylamino, and N,N-dihexylamino. The term "alkanoyloxy" denotes an alkanoyloxy group having 1 to 6 carbon atoms and includes, for example, acetyloxy, propionyloxy, butyryloxy, isobutyryloxy, pentanoyloxy, and hexanoyloxy. The term "alkanoyl" denotes an alkanoyl group having 1 to 6 carbon atoms and includes, for example, acetyl, propionyl, butyryl, isobutyryl, pentanoyl, and hexanoyl. The cycloalkyl, phenyl and phenyl alkyl having on the cycloalkyl or phenyl ring one or two substituents selected from the group consisting of a alkoxy having 1 to 6 carbon atoms, a alkyl having 1 to 6 carbon atoms, a halogen, an N,N-dialkylamino having 1 to 6 carbon atoms in the alkyl moiety, nitro, aminosulfonyl, hydroxy or a alkanoyloxy having 1 to 6 carbon atoms in the alkanoyloxy moiety include, for example, 2-hydroxycyclohexyl, 3-hydroxycyclohexyl, 4-hydroxycyclohexyl, 2,5-dihydroxycyclohexyl, 3,4-dihydroxycyclohexyl, 2-hydroxycyclopentyl, 4-hydroxycyclooctyl, 2-acetyloxycyclohexyl, 4-butyryloxycyclohexyl, 3,4-diacetyloxycyclohexyl, 2-methylcyclohexyl, 3-ethylcyclohexyl, 4-tert-butylcyclohexyl, 3,4-dimethylcyclohexyl, 2,5-dimethylcyclohexyl, 2-methoxycyclohexyl, 3-methoxycyclohexyl, 4-methoxycyclohexyl, 3,4-dimethoxycyclohexyl, 2,5-dimethoxycyclohexyl, 4-tert-butoxycyclohexyl, 3-methylcycloheptyl, 2-chlorocyclohexyl, 3-fluorocyclohexyl, 4-bromocyclohexyl, 3,4-difluorocyclohexyl, 2,5-dichlorocyclohexyl, 4-(N,N-dimethylamino)cyclohexyl, 3-(N,N-diethylamino)cyclohexyl, 2-nitrocyclohexyl, 3-nitrocyclooctyl, 4-nitrocyclohexyl, 3,5-dinitrocyclohexyl, 2-aminosulfonylcyclohexyl, 4-aminosulfonylcyclohexyl, 2-hydroxy-3-chlorocyclohexyl, 2-methyl-3-chlorocyclohexyl, 2-hydroxyphenyl, 3-hydroxyphenyl, 4-hydroxyphenyl, 2,5-dihydroxyphenyl, 3,4-dihydroxyphenyl, 2-acetyloxyphenyl, 4-isobutyryloxyphenyl, 3,4-diacetyloxyphenyl, 2-methylphenyl, 3-methylphenyl, 4-ethylphenyl, 3,4-dimethylphenyl, 2,5-diethylphenyl, 2-methoxyphenyl, 3-methoxyphenyl, 4-methoxyphenyl, 3,4-dimethoxyphenyl, 2,5-diethoxyphenyl, 4-isopropoxyphenyl, 2-chlorophenyl, 3-bromophenyl, 4-fluorophenyl, 2,5-dichlorophenyl, 3,4-dichlorophenyl, 4-(N,N-dimethylamino)phenyl, 2-(N,N-dimethylamino)phenyl, 3-(N,N-diethylamino)phenyl, 2-nitrophenyl, 3-nitrophenyl, 4-nitrophenyl, 2,4-dinitrophenyl, 2-aminosulfonylphenyl, 4-aminosulfonylphenyl, 2-hydroxy-3-chlorophenyl, 4-methoxy-2-chlorophenyl, 2-methyl-3-chlorophenyl, 2-hydroxybenzyl, 2-(4-hydroxyphenyl)ethyl, 4-(3-hydroxyphenyl)butyl, 2-(3,4-dihydroxyphenyl)ethyl, 1-(2,5-dihydroxyphenyl)ethyl, 2-acetyloxybenzyl, 2-(3-acetyloxyphenyl)ethyl, 2-(3,4-diacetyloxyphenyl)ethyl, 1,1-dimethyl-2-(3,4-diacetyloxyphenyl)ethyl, 2-methylbenzyl, 2-(4-methylphenyl)ethyl, 2-(3,4-dimethylphenyl)ethyl, 6-(2,5-dimethylphenyl)hexyl, 2-chlorobenzyl, 3-fluorobenzyl, 4-bromobenzyl, 2-(2-chlorophenyl)ethyl, 1-(4-chlorophenyl)ethyl, 2-(3,4-dichlorophenyl)ethyl, 5-(2,5-dichlorophenyl)pentyl, 4-(N,N-dimethylamino)benzyl, 2-[2-(N,N-dimethylamino)phenyl]ethyl, 2-nitrobenzyl, 2-(2,4-dinitrophenyl)ethyl, 1-(4-nitrophenyl)ethyl, 2-aminosulfonylbenzyl, 3-aminosulfonylbenzyl, 4-aminosulfonylbenzyl, 2-(4-aminosulfonylphenyl)ethyl, 2-hydroxy-3-chlorobenzyl, 4-methoxy-2-chlorobenzyl, 2-(2-methyl-3-chlorophenyl)ethyl, 2-methoxybenzyl, 4-methoxybenzyl, 2-(3,4-dimethoxyphenyl)ethyl, 1-(3,4-dimethoxyphenyl)ethyl, 2-(2,5-diethoxyphenyl)ethyl, 1,1-dimethyl-2-(3,4-dimethoxyphenyl)ethyl, and 6-(3,4-dimethoxyphenyl)hexyl.

Representative compounds of the present invention are as follows.

N-Ethyl-N-cyclohexyl-4-(1-methyl-1,2,3,4-tetrazol-5-yl)thio-butyramide.
N-Butyl-N-cyclooctyl-4-(1-methyl-1,2,3,4-tetrazol-5-yl)thio-butyramide
N-Methyl-N-cyclopentyl-4-(1-methyl-1,2,3,4-tetrazol-5-yl)thio-butyramide
N-Isopropyl-N-cyclohexyl-4-(1-methyl-1,2,3,4-tetrazol-5-yl)thio-butyramide
N-Hexyl-N-cyclohexyl-4-(1-methyl-1,2,3,4-tetrazol-5-yl)thio-butyramide
N-Methyl-N-cyclododecanyl-4-(1-methyl-1,2,3,4-tetrazol-5-yl)thio-butyramide
N-Methyl-N-cyclohexyl-4-(1-ethyl-1,2,3,4-tetrazol-5-yl)thio-butyramide
N-Ethyl-N-cyclohexyl-4-(1-tert-butyl-1,2,3,4-tetrazol-5-yl)thio-butyramide
N-Methyl-N-cyclohexyl-4-(1-hexyl-1,2,3,4-tetrazol-5-yl)thio-butyramide
N-Cyclohexyl-4-(1-methyl-1,2,3,4-tetrazol-5-yl)thio-butyramide

N-Cyclododecanyl-4-(1-methyl-1,2,3,4-tetrazol-5-yl)thio-butyramide
N-Methyl-N-cyclohexyl-3-(1-methyl-1,2,3,4-tetrazol-5-yl)thio-propionamide
N-Methyl-N-cyclohexyl-2-(1-methyl-1,2,3,4-tetrazol-5-yl)thio-acetamide
N-Methyl-N-cyclohexyl-5-(1-methyl-1,2,3,4-tetrazol-5-yl)thio-valeramide
N-Methyl-N-cyclohexyl-7-(1-methyl-1,2,3,4-tetrazol-5-yl)thio-heptanamide
N-Methyl-N-cyclohexyl-4-(1-methyl-1,2,3,4-tetrazol-5-yl)thio-3-methylbutyramide
N-Ethyl-N-cyclohexyl-4-(1-ethyl-1,2,3,4-tetrazol-5-yl)thio-3,3-dimethylbutyramide
N-Ethyl-N-cyclohexyl-4-(1-cyclohexyl-1,2,3,4-tetrazol-5-yl)thio-butyramide
N-Ethyl-N-cyclohexyl-4-(1-cyclooctyl-1,2,3,4-tetrazol-5-yl)thio-butyramide
N-Cyclohexyl-4-(1-cyclohexyl-1,2,3,4-tetrazol-5-yl)thio-butyramide
N-Methyl-N-cyclohexyl-3-(1-cyclohexyl-1,2,3,4-tetrazol-5-yl)thio-propionamide
N-Methyl-N-cyclohexyl-4-(1-cyclohexyl-1,2,3,4-tetrazol-5-yl)thio-3-methylbutyramide
N-Methyl-N-cyclohexyl-4-(1-phenyl-1,2,3,4-tetrazol-5-yl)thio-butyramide
N-Ethyl-N-cyclohexyl-3-(1-phenyl-1,2,3,4-tetrazol-5-yl)thio-propionamide
N-Methyl-N-cyclohexyl-4-(1-phenyl-1,2,3,4-tetrazol-5-yl)thio-3-methylbutyramide
N,N-Dicyclohexyl-4-(1-methyl-1,2,3,4-tetrazol-5-yl)thio-butyramide
N-Cyclohexyl-N-cyclooctyl-4-(1-methyl-1,2,3,4-tetrazol-5-yl)thio-butyramide
N,N-Dicyclohexyl-5-(1-phenyl-1,2,3,4-tetrazol-5-yl)thio-valeramide
N,N-Dicyclohexyl-4-(1-cyclohexyl-1,2,3,4-tetrazol-5-yl)thio-butyramide
N-Ethyl-N-cyclohexyl-4-[1-(4-hydroxycyclohexyl)-1,2,3,4-tetrazol-5-yl]thio-butyramide
N-Methyl-N-cyclohexyl-4-[1-(3,4-dihydroxycyclohexyl)-1,2,3,4-tetrazol-5-yl]thio-butyramide
N-Methyl-N-cyclohexyl-4-[1-(4-acetyloxycyclohexyl)-1,2,3,4-tetrazol-5-yl]thio-butyramide
N-Methyl-N-cyclohexyl-4-[1-(4-methylcyclohexyl)-1,2,3,4-tetrazol-5-yl]thio-butyramide
N-Methyl-N-cyclohexyl-4-[1-(2,5-dimethylcyclohexyl)-1,2,3,4-tetrazol-5-yl]thio-butyramide
N-Methyl-N-cyclohexyl-4-[1-(2-methoxycyclohexyl)-1,2,3,4-tetrazol-5-yl]thio-butyramide
N-Cyclohexyl-4-[1-(4-methoxycyclohexyl)-1,2,3,4-tetrazol-5-yl]thio-butyramide
N-Methyl-N-cyclohexyl-4-[1-(3,4-dimethoxycyclohexyl)-1,2,3,4-tetrazol-5-yl]thio-butyramide
N-Methyl-N-cyclohexyl-4-[1-(2-chlorocyclohexyl)-1,2,3,4-tetrazol-5-yl]thio-butyramide
N-Ethyl-N-cyclohexyl-4-[1-(3-fluorocyclohexyl)-1,2,3,4-tetrazol-5-yl]thio-butyramide
N-Ethyl-N-cyclohexyl-4-[1-(4-bromocyclohexyl)-1,2,3,4-tetrazol-5-yl]thio-butyramide
N-Methyl-N-cyclohexyl-4-[1-(2,5-dichlorocyclohexyl)-1,2,3,4-tetrazol-5-yl]thio-butyramide
N-Methyl-N-cyclohexyl-4-[1-(4-(N,N-dimethylamino)cyclohexyl)-1,2,3,4-tetrazol-5-yl]thio-butyramide
N-Ethyl-N-cyclohexyl-4-[1-(2-nitrocyclohexyl)-1,2,3,4-tetrazol-5-yl]thio-butyramide
N-Methyl-N-cyclohexyl-4-[1-(4-aminosulfonylcyclohexyl)-1,2,3,4-tetrazol-5-yl]thio-butyramide
N-Ethyl-N-(2-hydroxycyclohexyl)-4-(1-methyl-1,2,3,4-tetrazol-5-yl)thio-butyramide
N-Methyl-N-(3-hydroxycyclohexyl)-4-(1-methyl-1,2,3,4-tetrazol-5-yl)thio-butyramide
N-Ethyl-N-(4-hydroxycyclohexyl)-4-(1-methyl-1,2,3,4-tetrazol-5-yl)thio-butyramide
N-Methyl-N-(3,4-dihydroxycyclohexyl)-4-(1-methyl-1,2,3,4-tetrazol-5-yl)thio-butyramide
N-Ethyl-N-(4-hydroxycyclohexyl)-4-(1-cyclohexyl-1,2,3,4-tetrazol-5-yl)thio-butyramide
N-Methyl-N-(2-hydroxycyclohexyl)-4-(1-phenyl-1,2,3,4-tetrazol-5-yl)thio-butyramide
N-Methyl-N-(4-hydroxycyclohexyl)-4-[1-(4-hydroxycyclohexyl)-1,2,3,4-tetrazol-5-yl)thio-butyramide
N-(4-Hydroxycyclohexyl)-4-(1-methyl-1,2,3,4-tetrazol-5-yl)thio-butyramide
N-(2-Hydroxycyclohexyl)-4-(1-cyclohexyl-1,2,3,4-tetrazol-5-yl)thio-butyramide
N-(3-Hydroxycyclohexyl)-4-(1-phenyl-1,2,3,4-tetrazol-5-yl)thio-butyramide
N-Methyl-N-(4-acetyloxycyclohexyl)-4-(1-ethyl-1,2,3,4-tetrazol-5-yl)thio-butyramide
N-Ethyl-N-(3-acetyloxycyclohexyl)-4-(1-phenyl-1,2,3,4-tetrazol-5-yl)thio-butyramide
N-Ethyl-N-(3-acetyloxycyclohexyl)-4-(1-cyclohexyl-1,2,3,4-tetrazol-5-yl)thio-butyramide
N-Methyl-N-(3,4-diacetyloxycyclohexyl)-4-(1-methyl-1,2,3,4-tetrazol-5-yl)thio-butyramide
N-(2-Acetyloxycyclohexyl)-4-(1-cyclohexyl-1,2,3,4-tetrazol-5-yl)thio-butyramide
N,N-Di(4-hydroxycyclohexyl)-4-(1-methyl-1,2,3,4-tetrazol-5-yl)thio-butyramide
N-Methyl-N-(2-methylcyclohexyl)-4-(1-methyl-1,2,3,4-tetrazol-5-yl)thio-butyramide
N-Ethyl-N-(4-methylcyclohexyl)-4-(1-cyclohexyl-1,2,3,4-tetrazol-5-yl)thio-butyramide
N-Methyl-N-(3-methylcyclohexyl)-4-(1-phenyl-1,2,3,4-tetrazol-5-yl)thio-butyramide
N-Methyl-N-(3,4-dimethylcyclohexyl)-4-(1-methyl-1,2,3,4-tetrazol-5-yl)thio-butyramide
N-Methyl-N-(4-methoxycyclohexyl)-4-(1-methyl-1,2,3,4-tetrazol-5-yl)thio-butyramide
N-Ethyl-N-(2-methoxycyclohexyl)-4-(1-cyclohexyl-1,2,3,4-tetrazol-5-yl)thio-butyramide
N-Methyl-N-(3-methoxycyclohexyl)-4-(1-phenyl-1,2,3,4-tetrazol-5-yl)thio-butyramide
N-Methyl-N-(3,4-dimethoxycyclohexyl)-4-(1-methyl-1,2,3,4-tetrazol-5-yl)thio-butyramide
N-(4-Methoxycyclohexyl)-4-(1-cyclohexyl-1,2,3,4-tetrazol-5-yl)thio-butyramide
N-Methyl-N-(4-methoxycyclohexyl)-4-[1-(4-hydroxycyclohexyl)-1,2,3,4-tetrazol-5-yl]thio-butyramide
N,N-Di(4-methoxycyclohexyl)-4-(1-methyl-1,2,3,4-tetrazol-5-yl)thio-butyramide
N-Methyl-N-(2-chlorocyclohexyl)-4-(1-methyl-1,2,3,4-tetrazol-5-yl)thio-butyramide
N-Methyl-N-(3-fluorocyclohexyl)-4-(1-cyclohexyl-1,2,3,4-tetrazol-5-yl)thio-butyramide
N-Methyl-N-(4-bromocyclohexyl)-4-(1-phenyl-1,2,3,4-tetrazol-5-yl)thio-butyramide
N-Methyl-N-(3,4-dichlorocyclohexyl)-4-(1-methyl-1,2,3,4-tetrazol-5-yl)thio-butyramide

N-Methyl-N-[4-(N,N-dimethylamino)cyclohexyl]-4-(1-methyl-1,2,3,4-tetrazol-5-yl)thio-butyramide
N-Methyl-N-(4-nitrocyclohexyl)-4-(1-methyl-1,2,3,4-tetrazol-5-yl)thio-butyramide
N-Methyl-N-(4-aminosulfonylcyclohexyl)-4-(1-methyl-1,2,3,4-tetrazol-5-yl)thio-butyramide
N-Methyl-N-(2-hydroxy-3-chlorocyclohexyl)-4-(1-methyl-1,2,3,4-tetrazol-5-yl)thio-butyramide
N-Methyl-N-(2-hydroxyphenyl)-4-(1-methyl-1,2,3,4-tetrazol-5-yl)thio-butyramide
N-(4-Hydroxyphenyl)-4-(1-methyl-1,2,3,4-tetrazol-5-yl)thio-butyramide
N-Methyl-N-(2-acetyloxyphenyl)-4-(1-methyl-1,2,3,4-tetrazol-5-yl)thio-butyramide
N-Ethyl-N-phenyl-4-(1-methyl-1,2,3,4-tetrazol-5-yl)thio-butyramide
N-Methyl-N-phenyl-4-(1-phenyl-1,2,3,4-tetrazol-5-yl)thio-butyramide
N-Methyl-N-phenyl-4-(1-cyclohexyl-1,2,3,4-tetrazol-5-yl)thio-butyramide
N-Phenyl-4-(1-phenyl-1,2,3,4-tetrazol-5-yl)thio-butyramide
N-Methyl-N-phenyl-5-(1-methyl-1,2,3,4-tetrazol-5-yl)thio-valeramide
N-Methyl-N-(2-methylphenyl)-4-(1-methyl-1,2,3,4-tetrazol-5-yl)thio-butyramide
N-(2-Methyl-3-chlorophenyl)-4-(1-methyl-1,2,3,4-tetrazol-5-yl)thio-butyramide
N-Ethyl-N-(2-methylphenyl)-4-[1-(3,4-dihydroxyphenyl)-1,2,3,4-tetrazol-5-yl]thio-butyramide
N-Methyl-N-(2,5-dimethylphenyl)-4-(1-methyl-1,2,3,4-tetrazol-5-yl)thio-butyramide
N-Methyl-N-(2-methoxyphenyl)-4-(1-methyl-1,2,3,4-tetrazol-5-yl)thio-butyramide
N-Methyl-N-(3-methoxyphenyl)-5-(1-phenyl-1,2,3,4-tetrazol-5-yl)thio-valeramide
N-Methyl-N-(3,4-dimethoxyphenyl)-4-(1-methyl-1,2,3,4-tetrazol-5-yl)thio-butyramide
N-Methyl-N-(3,4-dimethoxyphenyl)-4-[1-(4-hydroxycyclohexyl)-1,2,3,4-tetrazol-5-yl]thio-butyramide
N-(2-Methoxyphenyl)-4-(1-methyl-1,2,3,4-tetrazol-5-yl)thio-butyramide
N-(3,4-Dimethoxyphenyl)-4-(1-methyl-1,2,3,4-tetrazol-5-yl)thio-butyramide
N-Methyl-N-(2-chlorophenyl)-4-(1-methyl-1,2,3,4-tetrazol-5-yl)thio-butyramide
N-Methyl-N-(4-fluorophenyl)-4-(1-phenyl-1,2,3,4-tetrazol-5-yl)thio-butyramide
N-(4-Chlorophenyl)-4-(1-methyl-1,2,3,4-tetrazol-5-yl)thio-butyramide
N-[4-(N,N-Dimethylamino)phenyl]-4-(1-methyl-1,2,3,4-tetrazol-5-yl)thio-butyramide
N-(4-Nitrophenyl)-4-(1-methyl-1,2,3,4-tetrazol-5-yl)thio-butyramide
N-(4-Aminosulfonylphenyl)-4-(1-methyl-1,2,3,4-tetrazol-5-yl)thio-butyramide
N-Methyl-N-cyclohexyl-4-[1-(4-hydroxyphenyl)-1,2,3,4-tetrazol-5-yl]thio-butyramide
N-Methyl-N-cyclohexyl-4-[1-(2-methoxyphenyl)-1,2,3,4-tetrazol-5-yl]thio-butyramide
N-Ethyl-N-cyclohexyl-4-[1-(4-chlorophenyl)-1,2,3,4-tetrazol-5-yl]thio-butyramide
N-Methyl-N-cyclohexyl-4-[1-(4-N,N-dimethylamino)phenyl-1,2,3,4-tetrazol-5-yl]thio-butyramide
N-Methyl-N-cyclohexyl-4-[1-(4-aminosulfonylphenyl)-1,2,3,4-tetrazol-5-yl]thio-butyramide
N-Methyl-N-cyclohexyl-4-[1-(4-nitrophenyl)-1,2,3,4-tetrazol-5-yl]thio-butyramide
N,N-Dimethyl-4-(1-methyl-1,2,3,4-tetrazol-5-yl)thio-butyramide
N,N-Diethyl-4-(1-methyl-1,2,3,4-tetrazol-5-yl)thio-butyramide
N-N-Diisopropyl-4-(1-methyl-1,2,3,4-tetrazol-5-yl)thio-butyramide
N,N-Dihexyl-4-(1-methyl-1,2,3,4-tetrazol-5-yl)thio-butyramide
N-Methyl-N-ethyl-4-(1-methyl-1,2,3,4-tetrazol-5-yl)thio-butyramide
N,N-Diethyl-5-(1-ethyl-1,2,3,4-tetrazol-5-yl)thio-valeramide
N,N-Dimethyl-4-(1-methyl-1,2,3,4-tetrazol-5-yl)thio-3-methylbutyramide
N,N-Diethyl-4-(1-cyclohexyl-1,2,3,4-tetrazol-5-yl)thio-butyramide
N,N-Dimethyl-4-(1-phenyl-1,2,3,4-tetrazol-5-yl)thio-butyramide
N,N-Diethyl-4-[1-(4-hydroxycyclohexyl)-1,2,3,4-tetrazol-5-yl]thio-butyramide
N,N-Diethyl-3-[1-(2-hydroxyphenyl)-1,2,3,4-tetrazol-5-yl]thio-propionamide
N,N-Diethyl-4-[1-(3,4-dimethoxyphenyl)-1,2,3,4-tetrazol-5-yl]thio-butyramide
N,N-Dimethyl-4-[1-(4-methylcyclohexyl)-1,2,3,4-tetrazol-5-yl]thio-butyramide
N,N-Diethyl-4-[1-(2-chlorophenyl)-1,2,3,4-tetrazol-5-yl]thio-butyramide
N,N-Dimethyl-4-[1-(4-chlorocyclohexyl)-1,2,3,4-tetrazol-5-yl]thio-butyramide
N-tert-Butyl-4-(1-phenyl-1,2,3,4-tetrazol-5-yl)thio-butyramide
N-Ethyl-4-[1-(3,4-dimethoxyphenyl)-1,2,3,4-tetrazol-5-yl]thio-butyramide
4-(1-Methyl-1,2,3,4-tetrazol-5-yl)thio-butyramide
4-(1-Cyclohexyl-1,2,3,4-tetrazol-5-yl)thio-butyramide
N-Ethyl-N-cyclohexylmethyl-4-(1-methyl-1,2,3,4-tetrazol-5-yl)thio-butyramide
N-Ethyl-N-(2-cyclohexylethyl)-4-(1-cyclohexyl-1,2,3,4-tetrazol-5-yl)thio-butyramide
N-Methyl-N-cyclooctylmethyl-4-(1-phenyl-1,2,3,4-tetrazol-5-yl)thio-butyramide
N-Cyclohexylmethyl-4-(1-methyl-1,2,3,4-tetrazol-5-yl)thio-butyramide
N-Phenyl-N-cyclohexylmethyl-4-(1-methyl-1,2,3,4-tetrazol-5-yl)thio-butyramide
N,N-Dicyclohexylmethyl-4-(1-cyclohexyl-1,2,3,4-tetrazol-5-yl)thio-butyramide
N-Cyclohexyl-N-cyclohexylmethyl-4-(1-methyl-1,2,3,4-tetrazol-5-yl)thio-butyramide
N-Methyl-N-cyclohexylmethyl-4-[1-(4-hydroxycyclohexyl)-1,2,3,4-tetrazol-5-yl]thio-butyramide
N-Cyclohexylmethyl-N-(4-hydroxycyclohexyl)-4-(1-methyl-1,2,3,4-tetrazol-5-yl)thio-butyramide
N-Ethyl-N-benzyl-4-(1-methyl-1,2,3,4-tetrazol-5-yl)thio-butyramide
N,N-Dibenzyl-4-(1-methyl-1,2,3,4-tetrazol-5-yl)thio-butyramide
N-Methyl-N-benzyl-4-(1-cyclohexyl-1,2,3,4-tetrazol-5-yl)thio-butyramide

N-Ethyl-N-benzyl-4-(1-phenyl-1,2,3,4-tetrazol-5-yl)thio-butyramide
N-(1-Phenylethyl)-4-(1-methyl-1,2,3,4-tetrazol-5-yl)thio-butyramide
N-Cyclohexyl-N-benzyl-4-(1-methyl-1,2,3,4-tetrazol-5-yl)thio-butyramide
N-Phenyl-N-benzyl-4-(1-phenyl-1,2,3,4-tetrazol-5-yl)thio-butyramide
N-Methyl-N-benzyl-4-[1-(4-hydroxycyclohexyl)-1,2,3,4-tetrazol-5-yl]thio-butyramide
N-Ethyl-N-benzyl-4-[1-(3,4-dimethoxyphenyl)-1,2,3,4-tetrazol-5-yl]thio-butyramide
N-Ethyl-N-[2-(3,4-dimethoxyphenyl)ethyl]-4-(1-methyl-1,2,3,4-tetrazol-5-yl)thio-butyramide
N-Benzyl-N-[2-(3,4-dimethoxyphenyl)ethyl]-4-(1-methyl-1,2,3,4-tetrazol-5-yl)thio-butyramide
N-Methyl-N-[2-(4-methoxyphenyl)ethyl]-4-(1-methyl-1,2,3,4-tetrazol-5-yl)thio-butyramide
N-Methyl-N-[2-(3,4-dihydroxyphenyl)ethyl]-4-(1-methyl-1,2,3,4-tetrazol-5-yl)thio-butyramide
N-Cyclohexyl-N-(2-hydroxybenzyl)-4-(1-cyclohexyl-1,2,3,4-tetrazol-5-yl)thio-butyramide
N-Methyl-N-(2-acetyloxybenzyl)-4-(1-methyl-1,2,3,4-tetrazol-5-yl)thio-butyramide
N-Ethyl-N-[2-(3,4-diacetyloxyphenyl)ethyl]-4-(1-cyclohexyl-1,2,3,4-tetrazol-5-yl)thio-butyramide
N-Ethyl-N-(2-chlorobenzyl)-4-(1-methyl-1,2,3,4-tetrazol-5-yl)thio-butyramide
N-Cyclohexyl-N-[2-(3,4-dichlorophenyl)ethyl]-4-(1-methyl-1,2,3,4-tetrazol-5-yl)thio-butyramide
N-Methyl-N-(2-methylbenzyl)-5-(1-methyl-1,2,3,4-tetrazol-5-yl)thio-butyramide
N-Methyl-N-[2-(4-(N,N-dimethylamino)phenyl)ethyl]-4-(1-methyl-1,2,3,4-tetrazol-5-yl)thio-butyramide
N-Methyl-N-cyclohexyl-4-(1,2,3,4-tetrazol-5-yl)thio-butyramide
N,N-Dicyclohexyl-4-(1,2,3,4-tetrazol-5-yl)thio-butyramide
N-Methyl-N-phenyl-4-(1,2,3,4-tetrazol-5-yl)thio-butyramide
N-Methyl-N-cyclohexylmethyl-3-(1,2,3,4-tetrazol-5-yl)thio-propionamide
N,N-Dicyclohexylmethyl-4-(1,2,3,4-tetrazol-5-yl)thio-butyramide
N-Methyl-N-benzyl-4-(1,2,3,4-tetrazol-5-yl)thio-butyramide
4-(1,2,3,4-tetrazol-5-yl)thio-butyramide
N-(2-Hydroxyethyl)-4-(1-methyl-1,2,3,4-tetrazol-5-yl)thio-butyramide
N-Cyclohexyl-N-(2-hydroxyethyl)-4-(1-methyl-1,2,3,4-tetrazol-5-yl)thio-butyramide
N-Methyl-N-(2-hydroxyethyl)-4-(1-methyl-1,2,3,4-tetrazol-5-yl)thio-butyramide
N-Cyclohexyl-N-(2-hydroxyethyl)-4-(1-cyclohexyl-1,2,3,4-tetrazol-5-yl)thio-butyramide
N-Ethyl-N-(2-hydroxyethyl)-4-(1-phenyl-1,2,3,4-tetrazol-5-yl)thio-butyramide
N-Phenyl-N-(2-hydroxyethyl)-4-(1-methyl-1,2,3,4-tetrazol-5-yl)thio-butyramide
N-Methyl-N-(4-hydroxybutyl)-4-(1-methyl-1,2,3,4-tetrazol-5-yl)thio-butyramide
N,N-Di(2-hydroxyethyl)-4-(1-methyl-1,2,3,4-tetrazol-5-yl)thio-butyramide
N-(2-Hydroxyethyl)-N-(4-hydroxycyclohexyl)-4-(1-methyl-1,2,3,4-tetrazol-5-yl)thio-butyramide
N-(2-Hydroxyethyl-N-benzyl-4-(1-methyl-1,2,3,4-tetrazol-5-yl)thio-butyramide
N-(2-Hydroxyethyl)-N-cyclohexylmethyl-4-(1-cyclohexyl-1,2,3,4-tetrazol-5-yl)thio-butyramide
N-(2-Hydroxyethyl)-N-[2-(3,4-dimethoxyphenyl)-ethyl]-4-(1-methyl-1,2,3,4-tetrazol-5-yl)thio-butyramide
5-(3-Morpholinocarbonylpropylthio)-1-methyl-1,2,3,4-tetrazole
5-(3-Piperidinocarbonylpropylthio)-1-methyl-1,2,3,4-tetrazole
5-(4-Pyrrolidinocarbonylbutylthio)-1-methyl-1,2,3,4-tetrazole
5-(3-Piperazinocarbonylpropylthio)-1-cyclohexyl-1,2,3,4-tetrazole
5-[3-(1-Imidazolyl)carbonylpropylthio]-1-methyl-1,2,3,4-tetrazole
5-[3-(1-Pyrazolyl)carbonylpropylthio]-1-methyl-1,2,3,4-tetrazole
5-[3-(1-pyrrolyl)carbonylpropylthio]-1-methyl-1,2,3,4-tetrazole
5-[3-(4-methylpiperazino)carbonylpropylthio]-1-methyl-1,2,3,4-tetrazole
5-[4-(1-Imidazolyl)carbonylbutylthio]-1-cyclohexyl-1,2,3,4-tetrazole
5-[3-(1-Pyrazolyl)carbonylpropylthio]-1-phenyl-1,2,3,4-tetrazole
5-[3-(4-Acetylpiperazino)carbonylpropylthio]-1-methyl-1,2,3,4-tetrazole
N-(2-Pyridyl)-4-(1-methyl-1,2,3,4-tetrazol-5-yl)thio-butyramide
N-Ethyl-N-(2-pyridyl)-4-(1-methyl-1,2,3,4-tetrazol-5-yl)thio-butyramide
N-Cyclohexyl-N-(4-pyridyl)-4-(1-methyl-1,2,3,4-tetrazol-5-yl)thio-butyramide
N-Methyl-N-(3-pyridyl)-4-(1-cyclohexyl-1,2,3,4-tetrazol-5-yl)thio-butyramide
N-Methyl-N-(2-pyridylmethyl)-4-(1-methyl-1,2,3,4-tetrazol-5-yl)thio-butyramide
N-Methyl-N-(2-pyrrolyl)-4-(1-methyl-1,2,3,4-tetrazol-5-yl)thio-butyramide
N-Methyl-N-(3-pyrrolidinylmethyl)-4-(1-phenyl-1,2,3,4-tetrazol-5-yl)thio-butyramide
N-(3-Pyrrolin-2-yl)-4-(1-methyl-1,2,3,4-tetrazol-5-yl)thio-butyramide
N-Methyl-N-(3-pyrrolin-2-ylmethyl)-4-(1-cyclohexyl-1,2,3,4-tetrazol-5-yl)thio-butyramide
N-Methyl-N-(2-piperidinyl)-4-(1-methyl-1,2,3,4-tetrazol-5-yl)thio-butyramide
N-Cyclohexyl-N-(4-piperidinylmethyl)-4-(1-cyclohexyl-1,2,3,4-tetrazol-5-yl)thio-butyramide
N-Methyl-N-(2-pyrazolin-4-yl)-4-(1-methyl-1,2,3,4-tetrazol-5-yl)thio-butyramide
N-Methyl-N-(2-imidazolyl)-4-(1-methyl-1,2,3,4-tetrazol-5-yl)thio-butyramide
N-Cyclohexyl-N-(2-imidazolylmethyl)-4-(1-methyl-1,2,3,4-tetrazol-5-yl)thio-butyramide
N-(2-Imidazolidinyl)-5-(1-methyl-1,2,3,4-tetrazol-5-yl)thio-valeramide
N-Cyclohexyl-N-(3-pyrazolylmethyl)-4-(1-methyl-1,2,3,4-tetrazol-5-yl)thio-butyramide
N-(2-Pyrimidinyl)-4-(1-methyl-1,2,3,4-tetrazol-5-yl)thio-butyramide

N-Cyclohexyl-N-(3-pyridazinylmethyl)-4-(1-cyclohexyl-1,2,3,4-tetrazol-5-yl)thio-butyramide
N-(2-Pyrazinyl)-4-(1-cyclohexyl-1,2,3,4-tetrazol-5-yl)thio-butyramide
N-Methyl-N-(2-pyradinylmethyl)-4-(1-methyl-1,2,3,4-tetrazol-5-yl)thio-butyramide
N-Cyclohexyl-N-(2-oxazolyl)-4-(1-cyclohexyl-1,2,3,4-tetrazol-5-yl)thio-butyramide
N-Methyl-N-(2-oxazolylmethyl)-4-(1-methyl-1,2,3,4-tetrazol-5-yl)thio-butyramide
N-Methyl-N-(4-isoxazolyl)-4-(1-methyl-1,2,3,4-tetrazol-5-yl)thio-butyramide
N-(2-Thiazolyl)-4-(1-methyl-1,2,3,4-tetrazol-5-yl)thio-butyramide
N-Methyl-N-(2-furyl)-4-(1-cyclohexyl-1,2,3,4-tetrazol-5-yl)thio-butyramide
N-Furfuryl-4-(1-methyl-1,2,3,4-tetrazol-5-yl)thio-butyramide
N-Methyl-N-(2-tetrahydrofuryl)-4-(1-methyl-1,2,3,4-tetrazol-5-yl)thio-butyramide
N-Methyl-N-(2H-pyran-2-yl)-4-(1-methyl-1,2,3,4-tetrazol-5-yl)thio-butyramide
N-Methyl-N-(2-tetrahydropyranylmethyl)-4-(1-methyl-1,2,3,4-tetrazol-5-yl)thio-butyramide
N-(2-Thienyl)-4-(1-phenyl-1,2,3,4-tetrazol-5-yl)thio-butyramide
N-Methyl-N-(2-thienylmethyl)-4-(1-methyl-1,2,3,4-tetrazol-5-yl)thio-butyramide
N-Methyl-N-(4-thianyl)-4-(1-methyl-1,2,3,4-tetrazol-5-yl)thio-butyramide
N-Ethyl-N-cyclohexyl-5-(1-methyl-1,2,3,4-tetrazol-5-yl)valeramide
N-Butyl-N-cyclooctyl-5-(1-methyl-1,2,3,4-tetrazol-5-yl)valeramide
N-Ethyl-N-cyclohexyl-4-(1-tert-butyl-1,2,3,4-tetrazol-5-yl)butyramide
N-Methyl-N-cyclohexyl-7-(1-methyl-1,2,3,4-tetrazol-5-yl)heptanamide
N-Methyl-N-cyclohexyl-4-(1-methyl-1,2,3,4-tetrazol-5-yl)-3-methylbutyramide
N-Methyl-N-cyclohexyl-4-(1-cyclotridecanyl-1,2,3,4-tetrazol-5-yl)butyramide
N-Ethyl-N-cyclohexyl-5-(1-cyclohexyl-1,2,3,4-tetrazol-5-yl)valeramide
N-Ethyl-N-cyclohexyl-5-(1-phenyl-1,2,3,4-tetrazol-5-yl)valeramide
N,N-Dicyclohexyl-5-(1-phenyl-1,2,3,4-tetrazol-5-yl)valeramide
N,N-Dicyclohexyl-4-(1-cyclohexyl-1,2,3,4-tetrazol-5-yl)butyramide
N-Ethyl-N-cyclohexyl-4-[1-(4-hydroxycyclohexyl)-1,2,3,4-tetrazol-5-yl]butyramide
N-Methyl-N-cyclohexyl-5-[1-(4-acetyloxycyclohexyl)-1,2,3,4-tetrazol-5-yl]valeramide
N-Methyl-N-cyclohexyl-5-[1-(4-methylcyclohexyl)-1,2,3,4-tetrazol-5-yl]valeramide
N-Methyl-N-cyclohexyl-5-[1-(3,4-dimethoxycyclohexyl)-1,2,3,4-tetrazol-5-yl]valeramide
N-Methyl-N-cyclohexyl-4-[1-(2-chlorocyclohexyl)-1,2,3,4-tetrazol-5-yl)butyramide
N-Methyl-N-cyclohexyl-5-[1-(4-(N,N-dimethylamino)cyclohexyl)-1,2,3,4-tetrazol-5-yl]valeramide
N-Ethyl-N-(4-hydroxycyclohexyl)-4-(1-cyclohexyl-1,2,3,4-tetrazol-5-yl)butyramide
N-Methyl-N-(2-hydroxycyclohexyl)-4-(1-phenyl-1,2,3,4-tetrazol-5-yl)butyramide
N-(4-Hydroxycyclohexyl)-5-(1-phenyl-1,2,3,4-tetrazol-5-yl)valeramide
N-Methyl-N-(4-acetyloxycyclohexyl)-4-(1-ethyl-1,2,3,4-tetrazol-5-yl)butyramide
N-Ethyl-N-(2-acetyloxycyclohexyl)-5-(1-phenyl-1,2,3,4-tetrazol-5-yl)valeramide
N-Ethyl-N-(3-acetyloxycyclohexyl)-4-(1-cyclohexyl-1,2,3,4-tetrazol-5-yl)butyramide
N-Ethyl-N-(4-methylcyclohexyl)-4-(1-cyclohexyl-1,2,3,4-tetrazol-5-yl)butyramide
N-Methyl-N-(3,4-dimethylcyclohexyl)-5-(1-phenyl-1,2,3,4-tetrazol-5-yl)valeramide
N-Ethyl-N-(2-methoxycyclohexyl)-5-(1-cyclohexyl-1,2,3,4-tetrazol-5-yl)valeramide
N-Methyl-N-(3,4-dimethoxycyclohexyl)-5-(1-phenyl-1,2,3,4-tetrazol-5-yl)valeramide
N,N-Di(4-hydroxycyclohexyl)-4-[1-(4-hydroxycyclohexyl)-1,2,3,4-tetrazol-5-yl]butyramide
N-Methyl-N-(2-chlorocyclohexyl)-5-(1-methyl-1,2,3,4-tetrazol-5-yl)valeramide
N-Methyl-N-[4-(N,N-dimethylamino)cyclohexyl]-4-(1-phenyl-1,2,3,4-tetrazol-5-yl)butyramide
N-Methyl-N-(4-nitrocyclohexyl)-4-(1-methyl-1,2,3,4-tetrazol-5-yl)butyramide
N-Methyl-N-(4-aminosulfonylcyclohexyl)-5-(1-cyclohexyl-1,2,3,4-tetrazol-5-yl)valeramide
N-Methyl-N-(4-hydroxyphenyl)-5-(1-phenyl-1,2,3,4-tetrazol-5-yl)valeramide
N-Ethyl-N-phenyl-4-(1-methyl-1,2,3,4-tetrazol-5-yl)butyramide
N-Methyl-N-phenyl-5-(1-phenyl-1,2,3,4-tetrazol-5-yl)valeramide
N,N-Diphenyl-5-(1-phenyl-1,2,3,4-tetrazol-5-yl)valeramide
N-Phenyl-4-(1-phenyl-1,2,3,4-tetrazol-5-yl)butyramide
N-Methyl-N-4-methylphenyl)-5-(1-phenyl-1,2,3,4-tetrazol-5-yl)valeramide
N-Methyl-N-(2,5-dimethylphenyl)-4-(1-phenyl-1,2,3,4-tetrazol-5-yl)butyramide
N-Methyl-N-(3,4-dimethoxyphenyl)-4-(1-phenyl-1,2,3,4-tetrazol-5-yl)butyramide
N-Methyl-N-(4-methoxyphenyl)-4-[1-(4-methoxyphenyl)-1,2,3,4-tetrazol-5-yl]butyramide
N-Methyl-N-(3,4-dichlorophenyl)-4-(1-cyclohexyl-1,2,3,4-tetrazol-5-yl)butyramide
N-Methyl-N-cyclohexyl-4-[1-(2-methoxyphenyl)-1,2,3,4-tetrazol-5-yl]butyramide
N-Ethyl-N-cyclohexyl-4-[1-(4-chlorophenyl)-1,2,3,4-tetrazol-5-yl]butyramide
N-Methyl-N-phenyl-5-[1-(4-methylphenyl)-1,2,3,4-tetrazol-5-yl]valeramide
N-Methyl-N-cyclohexyl-4-[1-(4-(N,N-dimethylamino)phenyl)-1,2,3,4-tetrazol-5-yl]butyramide
N-Methyl-N-cyclohexyl-4-[1-(4-aminosulfonylphenyl)-1,2,3,4-tetrazol-5-yl]butyramide
N,N-Diethyl-5-(1-ethyl-1,2,3,4-tetrazol-5-yl)valeramide
N-Methyl-N-tert-butyl-4-(1-methyl-1,2,3,4-tetrazol-5-yl)butyramide
N,N-Diethyl-5-(1-cyclohexyl-1,2,3,4-tetrazol-5-yl)valeramide
N,N-Diethyl-5-(1-phenyl-1,2,3,4-tetrazol-5-yl)valeramide

N,N-Diethyl-4-[1-(4-hydroxycyclohexyl)-1,2,3,4-tetrazol-5-yl]butyramide
N,N-Diethyl-5-[1-(3,4-dimethloxyphenyl)-1,2,3,4-tetrazol-5-yl]valeramide
N-tert-Butyl-4-(1-phenyl-1,2,3,4-tetrazol-5-yl)butyramide
5-(1-Phenyl-1,2,3,4-tetrazol-5-yl)valeramide
N-Ethyl-N-cyclohexylmethyl-5-(1-methyl-1,2,3,4-tetrazol-5-yl)valeramide
N,N-Dicyclohexylmethyl-5-(1-cyclohexyl-1,2,3,4-tetrazol-5-yl)valeramide
N-Cyclohexyl-N-cyclohexylmethyl-4-(1-cyclohexyl-1,2,3,4-tetrazol-5-yl)butyramide
N-Cyclohexylmethyl-N-(2-cyclohexylethyl)-4-(1-cyclohexyl-1,2,3,4-tetrazol-5-yl)butyramide
N-Ethyl-N-benzyl-4-(1-phenyl-1,2,3,4-tetrazol-5-yl)butyramide
N-Cyclohexyl-N-benzyl-5-(1-cyclohexyl-1,2,3,4-tetrazol-5-yl)valeramide
N-Ethyl-N-[2-(3,4-dimethoxyphenyl)ethyl]-4-(1-cyclohexyl-1,2,3,4-tetrazol-5-yl)butyramide
N-Benzyl-N-[2-(3,4-dimethoxyphenyl)ethyl]-4-(1-phenyl-1,2,3,4-tetrazol-5-yl)butyramide
N-Phenyl-N-[2-(3,4-dimethoxyphenyl)ethyl]-4-(1-phenyl-1,2,3,4-tetrazol-5-yl)butyramide
N-Ethyl-N-[2-(3,4-diacetyloxyphenyl)ethyl]-4-(1-cyclohexyl-1,2,3,4-tetrazol-5-yl)butyramide
N-Cyclohexyl-N-[2-(3,4-dichlorophenyl)ethyl]-4-(1-cyclohexyl-1,2,3,4-tetrazol-5-yl)butyramide
N-Ethyl-N-[1-(4-nitrophenyl)ethyl]-4-(1-methyl-1,2,3,4-tetrazol-5-yl)butyramide
N-Methyl-N-cyclohexyl-5-(1,2,3,4-tetrazol-5-yl)valeramide
N-Phenyl-N-cyclohexyl-4-(1,2,3,4-tetrazol-5-yl)butyramide
5-(1,2,3,4-Tetrazol-5-yl)valeramide
N-Cyclohexyl-N-(2-hydroxyethyl)-4-(1-cyclohexyl-1,2,3,4-tetrazol-5-yl)butyramide
N-Methyl-N-(2-hydroxyethyl)-5-(1-phenyl-1,2,3,4-tetrazol-5-yl)valeramide
5-(4-Piperidinocarbonylbutyl)-1-phenyl-1,2,3,4-tetrazole
5-(4-Piperidinocarbonylbutyl)-1-methyl-1,2,3,4-tetrazole
5-[3-(1-Imidazolyl)carbonylpropyl]-1-cyclohexyl-1,2,3,4-tetrazole
5-[4-(4-Methylpiperazino)carbonylbutyl]-1-phenyl-1,2,3,4-tetrazole
5-[3-(4-Acetylpiperazino)carbonylpropyl]-1-methyl-tetrazole
N-(2-Pyridyl)-5-(1-methyl-1,2,3,4-tetrazol-5-yl)valeramide
N-Ethyl-N-(2-pyridyl)-4-(1-phenyl-1,2,3,4-tetrazol-5-yl)butyramide
N-Methyl-N-(2-pyridylmethyl)-4-(1-cyclohexyl-1,2,3,4-tetrazol-5-yl)butyramide
N-Methyl-N-[2-(3-pyrrolinyl)methyl]-4-(1-cyclohexyl-1,2,3,4-tetrazol-5-yl)butyramide
N-Cyclohexyl-N-(4-piperidinylmethyl)-5-(1-cyclohexyl-1,2,3,4-tetrazol-5-yl)valeramide
N-Methyl-N-(2-imidazolyl)-5-(1-cyclohexyl-1,2,3,4-tetrazol-5-yl)valeramide
N-Cyclohexyl-N-(3-pyrazolylmethyl)-4-(1-methyl-1,2,3,4-tetrazol-5-yl)butyramide
N-(2-Pyrimidinyl)-4-(1-cyclohexyl-1,2,3,4-tetrazol-5-yl)thio-butyramide
N-Cyclohexyl-N-(3-pyridazinylmethyl)-4-(1-cyclohexyl-1,2,3,4-tetrazol-5-yl)butyramide
N-Methyl-N-(2-oxazolylmethyl)-4-(1-phenyl-1,2,3,4-tetrazol-5-yl)butyramide
N-Cyclohexyl-N-(morpholinomethyl)-4-(1-cyclohexyl-1,2,3,4-tetrazol-5-yl)butyramide
N-(2-Thiazolyl)-4-(1-methyl-1,2,3,4-tetrazol-5-yl)butyramide
N-Methyl-N-(2-furyl)-5-(1-cyclohexyl-1,2,3,4-tetrazol-5-yl)valeramide
N-Methyl-N-(2-tetrahydrofurylmethyl)-4-(1-cyclohexyl-1,2,3,4-tetrazol-5-yl)butyramide
N-(2-Thienyl)-4-(1-phenyl-1,2,3,4-tetrazol-5-yl)butyramide
N-Ethyl-N-[1-(4-nitrophenyl)ethyl]-4-(1-methyl-1,2,3,4-tetrazol-5-yl)thio-butyramide
N-Methyl-N-[2-(4-aminosulfonylphenyl)ethyl]-4-(1-methyl-1,2,3,4-tetrazol-5-yl)thio-butyramide
N-Ethyl-N-cyclohexyl-4-[1-(2-nitrocyclohexyl)-1,2,3,4-tetrazol-5-yl]butyramide
N-Methyl-N-cyclohexyl-4-[1-(4-aminosulfonylcyclohexyl)-1,2,3,4-tetrazol-5-yl]butyramide
N-Phenyl-N-[2-(3,4-dimethylphenyl)ethyl]-4-(1-phenyl-1,2,3,4-tetrazol-5-yl)butyramide
N-Methyl-N-[2-(4-(N,N-dimethylamino)phenyl)ethyl]-4-(1-cyclohexyl-1,2,3,4-tetrazol-5-yl)butyramide
N-Methyl-N-[2-(4-aminosulfonylphenyl)ethyl]-4-(1-phenyl-1,2,3,4-tetrazol-5-yl)butyramide
N,N-Diethyl-3-(1-methyl-1,2,3,4-tetrazol-5-yl)methylthio-propionamide
N,N-Diethyl-3-(1-phenyl-1,2,3,4-tetrazol-5-yl)methylthio-propionamide
N,N-Diethyl-3-(1-cyclohexyl-1,2,3,4-tetrazol-5-yl)methylthio-propionamide
N,N-Diethyl-3-[2-(1-methyl-1,2,3,4-tetrazol-5-yl)ethylthio]propionamide
N-Ethyl-N-cyclohexyl-3-(1-methyl-1,2,3,4-tetrazol-5-yl)methylthio-propionamide
N-Ethyl-N-cyclohexyl-3-(1-phenyl-1,2,3,4-tetrazol-5-yl)methylthio-propionamide
N-Ethyl-N-cyclohexyl-3-(1-cyclohexyl-1,2,3,4-tetrazol-5-yl)methylthio-propionamide
N-Ethyl-N-cyclohexyl-3-[2-methyl-3-(1-methyl-1,2,3,4-tetrazol-5-yl)propylthio]propionamide
N,N-dicyclohexyl-3-(1-cyclohexyl-1,2,3,4-tetrazol-5-yl)methylthio-propionamide
N-Ethyl-N-phenyl-3-(1-methyl-1,2,3,4-tetrazol-5-yl)methylthio-propionamide
N-Ethyl-N-phenyl-3-(1-cyclohexyl-1,2,3,4-tetrazol-yl)methylthio-propionamide
N-Ethyl-N-phenyl-3-(1-phenyl-1,2,3,4-tetrazol-5-yl)methylthio-propionamide
N,N-Diphenyl-3-(1-phenyl-1,2,3,4-tetrazol-5-yl)methylthio-propionamide
N-Ethyl-N-cyclohexyl-4-(1-methyl-1,2,3,4-tetrazol-5-yl)methylthio-butyramide
N-Ethyl-N-benzyl-3-(1-methyl-1,2,3,4-tetrazol-5-yl)methylthio-propionamide
N-Ethyl-N-cyclohexylmethyl-3-(1-methyl-1,2,3,4-tetrazol-5-yl)methylthio-propionamide
N-Ethyl-N-(4-hydroxycyclohexyl)-3-(1-methyl-1,2,3,4-tetrazol-5-yl)methylthio-propionamide

N-Ethyl-N-[2-(3,4-dimethoxyphenyl)ethyl]-3-(1-methyl-1,2,3,4-tetrazol-5-yl)methylthio-propionamide
N-Ethyl-N-(4-methylphenyl)-3-(1-methyl-1,2,3,4-tetrazol-5-yl)methylthio-propionamide
N-Ethyl-N-cyclohexyl-3-(1-ethyl-1,2,3,4-tetrazol-5-yl)methylthio-propionamide
N,N-Diethyl-3-[1-(4-ethylphenyl)-1,2,3,4-tetrazol-5-yl]methylthio-propionamide
N-Ethyl-N-cyclohexyl-3-[1-(4-hydroxycyclohexyl)-1,2,3,4-tetrazol-5-yl]methylthio-propionamide
N-Ethyl-N-cyclohexyl-3-[1-(3,4-dimethoxyphenyl-1,2,3,4-tetrazol-5-yl]methylthio-propionamide
N-Ethyl-N-cyclohexyl-3-(1,2,3,4-tetrazol-5-yl)methylthio-propionamide
3-(1-Methyl-1,2,3,4-tetrazol-5-yl)methylthio-propionamide
N-Ethyl-N-(2-hydroxyethyl)-3-(1-methyl-1,2,3,4-tetrazol-5-yl)methylthio-propionamide
N-Ethyl-N-(2-pyridyl)-3-(1-methyl-1,2,3,4-tetrazol-5-yl)methylthio-propionamide
N-Ethyl-N-(2-pyridylmethyl)-3-(1-methyl-1,2,3,4-tetrazol-5-yl)methylthio-propionamide
N-Ethyl-3-(1-methyl-1,2,3,4-tetrazol-5-yl)methylthio-propionamide
N-Cyclohexyl-3-(1-methyl-1,2,3,4-tetrazol-5-yl)methylthio-propionamide
N-Phenyl-3-(1-methyl-1,2,3,4-tetrazol-5-yl)methylthio-propionamide
N-Furyl-3-(1-methyl-1,2,3,4-tetrazol-5-yl)methylthio-propionamide
N-Ethyl-N-(2-thienylmethyl)-3-(1-methyl-1,2,3,4-tetrazol-5-yl)methylthio-propionamide
5-[(2-Piperidinocarbonylethyl)thiomethyl]-1-methyl-1,2,3,4-tetrazole
5-[(2-Morpholinocarbonylethyl)thiomethyl]-1-methyl-1,2,3,4-tetrazole
Methyl 4-(1-methyl-1,2,3,4-tetrazol-5-yl)thio-butyrate
Methyl 4-(1-isopropyl-1,2,3,4-tetrazol-5-yl)thio-butyrate
Methyl 4-(1-phenyl-1,2,3,4-tetrazol-5-yl)thio-butyrate
Ethyl 4-(1-cyclohexyl-1,2,3,4-tetrazol-5-yl)thio-butyrate
Ethyl 4-(1-methyl-1,2,3,4-tetrazol-5-yl)thio-butyrate
Isopropyl 3-(1-ethyl-1,2,3,4-tetrazol-5-yl)thio-propionate
Ethyl 4-(1-cyclooctyl-1,2,3,4-tetrazol-5-yl)thio-butyrate
Methyl 5-(1-phenyl-1,2,3,4-tetrazol-5-yl)valerate
Methyl 5-(1-cyclohexyl-1,2,3,4-tetrazol-5-yl)valerate
Methyl 5-(1-methyl-1,2,3,4-tetrazol-5-yl)valerate
4-(1-Methyl-1,2,3,4-tetrazol-5-yl)thio-butyric acid
4-(1-Phenyl-1,2,3,4-tetrazol-5-yl)thio-butyric acid
4-(1-Cyclohexyl-1,2,3,4-tetrazol-5-yl)thio-butyric acid
5-(1-Methyl-1,2,3,4-tetrazol-5-yl)valeric acid
5-(1-Phenyl-1,2,3,4-tetrazol-5-yl)valeric acid
5-(1-Cyclohexyl-1,2,3,4-tetrazol-5-yl)valeric acid
Methyl 3-(1-methyl-1,2,3,4-tetrazol-5-yl)methylthio-propionate
Ethyl 3-(1-phenyl-1,2,3,4-tetrazol-5-yl)methylthio-propionate
Methyl 3-(1-cyclohexyl-1,2,3,4-tetrazol-5-yl)methylthio-propionate
Methyl 3-[2-(1-methyl-1,2,3,4-tetrazol-5-yl)ethylthio]propionate
Methyl 4-(1-methyl-1,2,3,4-tetrazol-5-yl)methylthio-butyrate
Methyl 3-[2-methyl-3-(1-methyl-1,2,3,4-tetrazol-5-yl)propylthio]propionate
3-(1-Methyl-1,2,3,4-tetrazol-5-yl)methylthio-propionic acid
3-[1-(2-Methoxyphenyl)-1,2,3,4-tetrazol-5-yl]methylthio-propionic acid
3-(1-Phenyl-1,2,3,4-tetrazol-5-yl)methylthio-propionic acid
3-(1-Cyclohexyl-1,2,3,4-tetrazol-5-yl)methylthio-propionic acid
3-[2-(1-Methyl-1,2,3,4-tetrazol-5-yl)ethylthio]propionic acid
4-(1-Methyl-1,2,3,4-tetrazol-5-yl)methylthio-butyric acid
Methyl 3-[1-(4-ethylphenyl)-1,2,3,4-tetrazol-5-yl]methylthio-propionate
Methyl 3-[1-(3,4-dimethoxyphenyl)-1,2,3,4-tetrazol-5-yl]methylthio-propionate
Methyl 3-[1-(4-chlorophenyl)-1,2,3,4-tetrazol-5-yl]methylthio-propionate
Methyl 3-[1-(3,4-dihydroxyphenyl-1,2,3,4-tetrazol-5-yl]methylthio-propionate
Methyl 3-[1-(2-methylcyclohexyl)-1,2,3,4-tetrazol-5-yl]methylthio-propionate
Methyl 3-[1-(4-hydroxycyclohexyl)-1,2,3,4-tetrazol-5-yl]methylthio-propionate
Methyl 3-[1-(4-methoxycyclohexyl)-1,2,3,4-tetrazol-5-yl]methylthio-propionate
Methyl 3-[1-(4-chlorocyclohexyl)-1,2,3,4-tetrazol-5-yl]methylthio-propionate
3-[1-(4-Ethylphenyl)-1,2,3,4-tetrazol-5-yl]methylthio-propionic acid
3-[1-(2-Methoxycyclohexyl)-1,2,3,4-tetrazol-5-yl]methylthio-propionic acid
3-[1-(4-Hydroxyphenyl)-1,2,3,4-tetrazol-5-yl]methylthio-propionic acid
3-[1-(4-Methylcyclohexyl)-1,2,3,4-tetrazol-5-yl]methylthio-propionic acid
3-[1-(4-Hydroxycyclohexyl)-1,2,3,4-tetrazol-5-yl]methylthio-propionic acid
3-[1-(2-Chlorocyclohexyl)-1,2,3,4-tetrazol-5-yl]methylthio-propionic acid
Methyl 4-[1-(4-methylphenyl)-1,2,3,4-tetrazol-5-yl]thio-butyrate
Methyl 4-[1-(3,4-dimethoxyphenyl)-1,2,3,4-tetrazol-5-yl]thio-butyrate
Methyl 4-[1-(2-hydroxyphenyl)-1,2,3,4-tetrazol-5-yl]thio-butyrate
Methyl 4-[1-(2-methylcyclohexyl)-1,2,3,4-tetrazol-5-yl]thio-butyrate
Methyl 4-[1-(4-hydroxycyclohexyl)-1,2,3,4-tetrazol-5-yl]thio-butyrate

Methyl 4-[1-(3,4-dimethoxycyclohexyl)-1,2,3,4-tetrazol-5-yl]thio-butyrate
4-[1-(2-Methylphenyl)-1,2,3,4-tetrazol-5-yl]thio-butyric acid
4-[1-(4-Methoxyphenyl)-1,2,3,4-tetrazol-5-yl]thio-butyric acid
4-[1-(3,4-Dimethoxyphenyl)-1,2,3,4-tetrazol-5-yl]thio-butyric acid
4-[1-(4-Methylcyclohexyl)-1,2,3,4-tetrazol-5-yl]thio-butyric acid
4-[1-(2-Hydroxycyclohexyl)-1,2,3,4-tetrazol-5-yl]thio-butyric acid
4-[1-(3,4-Dimethoxycyclohexyl)-1,2,3,4-tetrazol-5-yl]thio-butyric acid
5-[1-(2-Methoxyphenyl)-1,2,3,4-tetrazol-5-yl]valeric acid
5-[1-(4-Hydroxycyclohexyl)-1,2,3,4-tetrazol-5-yl]valeric acid
5-[1-(4-Methylphenyl)-1,2,3,4-tetrazol-5-yl]valeric acid
5-[1-(2-Chlorocyclohexyl)-1,2,3,4-tetrazol-5-yl]valeric acid
4-[1-(4-(N,N-Dimethylamino)phenyl)-1,2,3,4-tetrazol-5-yl]thio-butyric acid
4-[1-(4-Nitrophenyl)-1,2,3,4-tetrazol-5-yl]thio-butyric acid
4-[1-(4-Aminosulfonylphenyl)-1,2,3,4-tetrazol-5-yl]thio-butyric acid
4-[1-(2-Acetyloxyphenyl)-1,2,3,4-tetrazol-5-yl]thio-butyric acid
5-(1,2,3,4-Tetrazol-5-yl)valeric acid
Methyl 5-(1,2,3,4-tetrazol-5-yl)valerate
Methyl 4-(1,2,3,4-tetrazol-5-yl)thio-butyrate
4-(1,2,3,4-Tetrazol-5-yl)thio-butyric acid
3-(1,2,3,4-Tetrazol-5-yl)methylthio-propionic acid
Methyl 3-(1,2,3,4-tetrazol-5-yl)methylthio-propionate

The compounds of the present invention can be prepared by various processes. For instance, compounds [Ia] which are the compounds [I] wherein $R^2$ is the group:

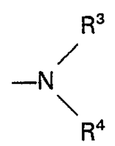

can be prepared by a process as shown in the following Reaction Scheme-I:

REACTION SCHEME-I

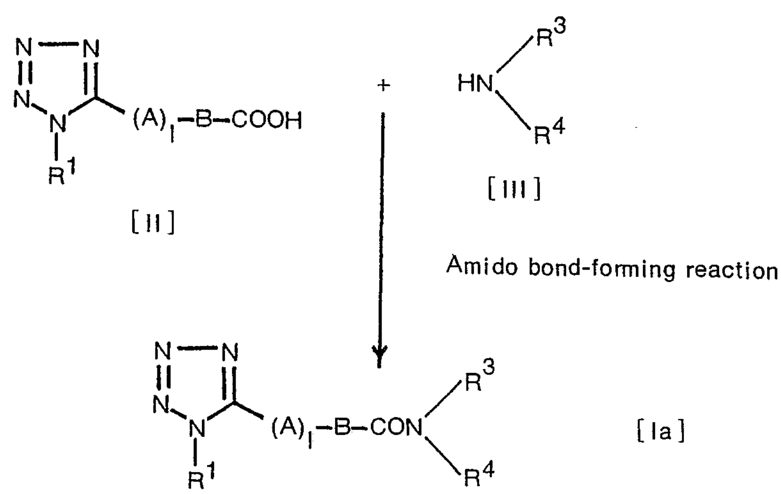


[II] [III] [Ia]

wherein $R^1$, $R^3$, $R^4$, A, B and l are as defined above.

The process as shown in Reaction Scheme-I is carried out by subjecting a carboxylic acid [II] and an amine [III] to an amido bond-forming reaction.

In the above process, a compound having an activated carboxyl group may be used instead of the carboxylic acid [II] and further a compound having an activated amino group instead of the amine [III]. The amido bond-forming reaction includes any conventional processes, such as (i) a mixed acid anhydride method, i.e. a process comprising reacting the carboxylic acid [II] with an alkyl halocarboxylate to give a mixed acid anhydride and reacting the mixed acid anhydride with the amine [III]; (ii) an active ester method, i.e. a process comprising converting the carboxylic acid [II] into an active ester, for example, p-nitrophenyl ester, N-hydroxysuccinimide ester, or N-hydroxybenzotriazole ester, and then reacting the active ester with the amine [III]; (iii) a carbodiimide method, i.e. a process comprising condensing the

carboxylic acid [II] with the amine [III] in the presence of a dehydrating agent such as dicyclohexylcarbodiimide or carbonyldiimidazole; (iv) a carboxylic acid halide method, i.e. a process comprising reacting a halide compound of the carboxylic acid [II] with the amine [III]; (v) a process comprising converting the carboxylic acid [II] into an acid anhydride compound thereof by using a dehydrating agent such as acetic anhydride and then reacting the resulting acid anhydride with the amine [III]; and (vi) a process comprising converting the carboxylic acid [II] into an ester with a lower alcohol and reacting the resulting ester with the amine [III] under a high pressure and at a high temperature. Among these process, the mixed acid anhydride method and carboxylic acid halide method are preferable. The alkyl halocarboxylate used in the mixed acid anhydride method includes, for instance, methyl chloroformate, methyl bromoformate, ethyl chloroformate, ethyl bromoformate and isobutyl chloroformate. The mixed acid anhydride may be prepared by the well-known Schotten-Baumann reaction and can be used to the subsequent reaction with the amine [III] without isolation from the reaction mixture. The Schotten-Baumann reaction can be done in the presence of a basic compound. The basic compound includes all compounds which are usually used in Schotten-Baumann reaction, for example, organic bases such as triethylamine, trimethylamine, pyridine, dimethylaniline, N-methylmorpholine, 1,5-diazabicyclo[4.3.0]nonene-5 (DBN), 1,5-diazabicyclo[5.4.0]-undecene-5 (DBU), or 1,4-diazabicyclo[2.2.2]octane (DABCO), and inorganic bases such as potassium carbonate, sodium carbonate, potassium hydrogen carbonate, or sodium hydrogen carbonate. The reaction is usually carried out at a temperature of −20 to 100°C, preferably 10 to 50°C, for 5 minutes to 10 hours.

The mixed acid anhydride method is usually carried out in an appropriate solvent. The solvent includes all solvent conventional in this method, for example, halogenated hydrocarbons such as methylene chloride, chloroform or dichloroethane, aromatic hydrocarbons such as benzene, toluene or xylene, ethers such as diethyl ether, tetrahydrofuran or dimethoxyethane, esters such as methyl acetate or ethyl acetate, aprotic polar solvents such as dimethylformamide, dimethylsulfoxide or hexamethylphosphoric triamide. The carboxylic acid [II], the alkyl halocarboxylate and the amine [III] are usually used in such an amount that the alkyl halocarboxylate and the amine [III] are each at least equimolar to the carboxylic acid [II], preferably 1 to 1.5 mole to 1 mole of the carboxylic acid [II].

The carboxylic acid halide method is carried out by reacting the carboxylic acid [II] with a halogenating agent to obtain a halide compound of the carboxylic acid [II] and then reacting the resulting carboxylic acid halide with the amine [III] after isolating and purifying the halide from the reaction mixture or without isolation.

The reaction of the carboxylic acid [II] and the halogenating agent is carried out in the presence or absence of a solvent. The solvent includes all solvents which do not give undesirable effect to the reaction, for example, aromatic hydrocarbons such as benzene, toluene or xylene, halogenated hydrocarbons such as chloroform, methylene chloride or carbon tetrachloride, ethers such as dioxane, tetrahydrofuran or diethyl ether, aprotic polar solvents such as dimethylformamide or dimethylsulfoxide. The halogenating agent includes any conventional halogenating agent which can convert the hydroxy group of the carboxyl group into halogen, for example, thionyl chloride, phosphorus oxychloride, phosphorus oxybromide, phosphorus pentachloride or phosphorus pentabromide.

The ratio of amount of the carboxylic acid [II] and the halogenating agent is not critical, but when the reaction is carried out in the absence of a solvent, the latter i.e. the acid [II], is used in a largely excess amount, and when the reaction is carried out in the presence of a solvent, the latter is used in an amount equivalent or more to the former, preferably 2 to 4 moles to 1 mole of the former. The reaction temperature and reaction time are not critical either, but the reaction is usually carried out at a temperature of room temperature to 100°C, preferably 50 to 80°C, for 30 minutes to 6 hours.

The reaction of the carboxylic acid halide with the amine [III] is usually carried out in the presence of a dehydrohalogenating agent. The dehydrohalogenating agent is usually basic compounds. The basic compounds used as the dehydrohalogenating agent include all conventional compounds, for example, inorganic bases such as sodium hydroxide, potassium hydroxide, sodium carbonate, potassium carbonate, sodium hydrogen carbonate, potassium hydrogen carbonate or silver carbonate, alkali metals such as sodium or potassium, alcoholates such as sodium methylate or sodium ethylate, organic bases such as triethylamine, pyridine, N,N-dimethylaminopyridine, 1,5-diazabicyclo[4.3.0]nonene-5 (DBN), 1,5-azabicyclo-[5.4.0]undecene-5 (DBU) or 1,4-diazabicyclo[2.2.2]octane (DABCO)]. The amine [III] may be used in a largely excess amount instead of using the dehydrohalogenating agent. The reaction may be carried out in the presence or absence of a solvent. The solvent includes any inert solvent which does not give undesirable effect to the reaction, for example, halogenated hydrocarbons such as chloroform, methylene chloride or carbon tetrachloride, ethers such as diethyl ether, tetrahydrofuran or dioxane, aromatic hydrocarbons such as benzene, toluene or xylene, esters such as methyl acetate or ethyl acetate, aprotic polar solvents such as N,N-dimethylformamide, dimethylsulfoxide or hexamethylphosphoric triamide.

The ratio of amount of the carboxylic acid halide and the amine [III] is not critical, but when the reaction is carried out in the absence of a solvent, the latter, i.e. the amine [III], is usually used in a largely excess amount, and when the reaction is carried out in the presence of a solvent, the latter is usually used in an amount of equivalent or more to the former, preferably 1 to 2 moles to 1 mole of the former. The reaction temperature and reaction time are not critical either, but the reaction is usually carried out at a temperature of −30 to 100°C, preferably 0 to 50°C, for 30 minutes to 12 hours.

The compounds [Ib] which are the compounds [I] wherein I is 1 can be prepared by a process as shown in the following Reaction Scheme-II:

REACTION SCHEME – II

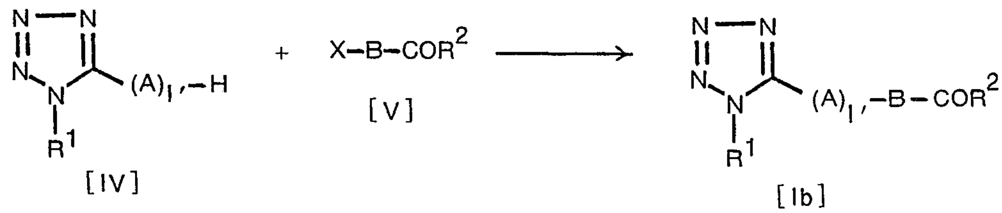


[IV] [V] [Ib]

wherein I′ is 1, X is a halogen, and $R^1$, $R^2$ and A are as defined above.

The dehydrohalogenation reaction of a 5-mercaptotetrazole derivative [IV] and a haloalkanecarboxylic acid derivative [V] in the above Reaction Scheme-II is usually carried out in the presence of the same dehydrohalogenating agent as used in the above Reaction Scheme-I. The reaction may be carried out in the presence or absence of a solvent. The solvent includes all inert solvents which do not give undesirable effect to the reaction, for example, alcohols such as methanol, ethanol, propanol, butanol or ethylene glycol, ethers such as diethyl ether, tetrahydrofuran, dioxane, monoglyme or diglyme, ketones such as acetone or methyl ethyl ketone, aromatic hydrocarbons such as benzene, toluene or xylene, esters such as methyl acetate or ethyl acetate, aprotic polar solvents such as N,N-dimethylformamide, dimethylsulfoxide or hexamethylphosphoric triamide. Besides, it is preferable to do the reaction in the presence of a metal iodide such as sodium iodide or potassium iodide.

The ratio of amount of the compound [IV] and the compound [V] is not critical, but when the reaction is carried out in the absence of a solvent, the latter, i.e. the compound [V], is used in a largely excess amount, and when the reaction is carried out in the presence of a solvent, the latter is used in an amount of 1 to 5 moles, preferably 1 to 2 moles, to 1 mole of the former. The reaction temperature and reaction time are not critical either, but the reaction is usually carried out at a temperature of −30 to 200°C, preferably 0 to 160°C, for 1 to 30 hours.

Compounds [Ic] which are the compounds [I] wherein I is 0 and $R^2$ is a lower alkoxy can be prepared by a process as shown in the following Reaction Scheme-III:

REACTION SCHEME – III

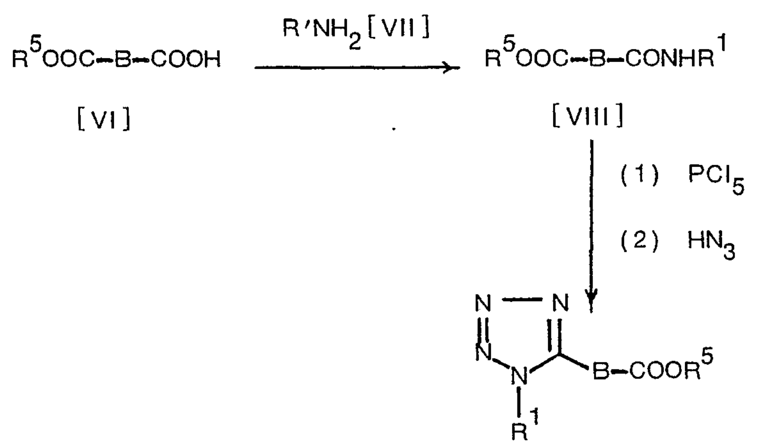


[VI] [VIII] [Ic]

wherein $R^1$ and B are as defined above, and $R^5$ is alkyl having 1 to 6 carbon atoms.

The reaction of a carboxylic acid [VI] and an amine [VII] in the above Reaction Scheme-III may preferably be carried out under the same conditions as shown in the mixed acid anhydride method and carboxylic acid halide method as mentioned in the above Reaction Scheme-I.

The reaction of a haloamide [VIII] with phosphorus pentachloride: $PCl_5$ is usually carried out in a solvent. The solvent includes all inert solvents which do not give undesirable effect to the reaction, for example, aromatic hydrocarbons such as benzene, toluene or xylene, halogenated aromatic hydrocarbons such as chlorobenzene or bromobenzene, ethers such as diethyl ether or dioxane, aliphatic hydrocarbons such as n-hexane or n-heptane. The ratio of amount of the haloamide [VIII] and $PCl_5$ is not critical, but usually, the latter, i.e. $PCl_5$, is used in an amount of 1 to 2 moles, preferably 1 to 1.2 mole, to 1 mole of the former. The reaction temperature and reaction time are not critical either, but the reaction is usually carried out at a temperature of −20 to 50°C, preferably 0 to 25°C, for 30 minutes to 5 hours.

The above reaction of the haloamide [VIII] with $PCl_5$, there is formed a haloimine derivative, and this

compound is subsequently reacted with hydrogen azide: $HN_3$ without isolation from the reaction mixture. The reaction is usually carried out in an appropriate solvent such as benzene, xylene, diethyl ether, or n-hexane. The hydrogen azide is usually used in an amount of 1 to 5 moles, preferably 1 to 3 moles, to 1 mole of the haloimine derivative. The reaction is usually carried out at a temperature of 0 to 150°C for 3 hours to 2 days.

Compounds [Id] which are the compounds [I] wherein A is a alkylenethio having 1 to 6 carbon atoms can be prepared by a process as shown in the following Reaction Scheme-IV:

REACTION SCHEME — IV

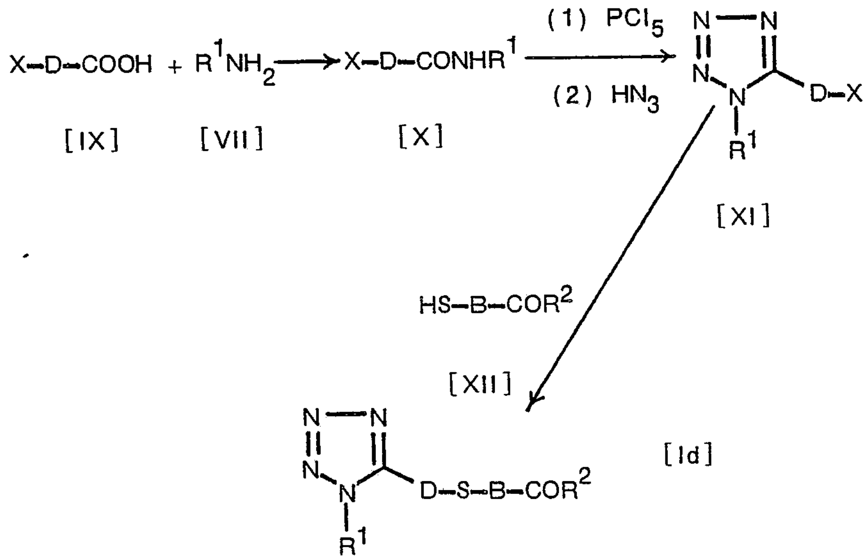


[IX] [VII] [X] [XI] [XII] [Id]

wherein X, $R^1$, $R^2$, and B are as defined above, and D is alkylene having 1 to 6 carbon atoms.

In Reaction Scheme-IV, the reaction of a compound [IX] and a compound [VII] and the reaction of a compound [X] and $PCl_5$ followed by the reaction with $HN_3$ are carried out under the same reaction conditions as in the reaction of the compound [VI] and the compound [VII] and the reaction of the compound [VIII] and $PCl_5$ followed by the reaction with $HN_3$ in the above Reaction Scheme-III, respectively. Besides, the reaction of a compound [XI] and a compound [XII] is carried out under the same reaction conditions as in the reaction of the compound [IV] and the compound [V] in the above Reaction Scheme-II.

Moreover, Compounds [Ie] which are the compounds [I] wherein $R^2$ is alkoxy having 1 to 6 carbon atoms and compounds [If] which are the compounds [I] wherein $R^2$ is hydroxy are reciprocally converted to each other by subjecting them to hydrolysis or esterification, as shown in the following Reaction Scheme-V:

REACTION SCHEME — V

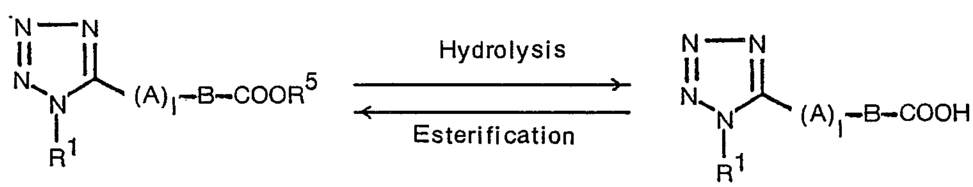


[Ie] [If]

wherein A, l, B, $R^1$, are as defined above.

Hydrolysis of the compound [Ie] can be carried out by conventional methods, for instance, in the presence of a basic compound such as sodium hydroxide, potassium hydroxide or barium hydroxide, or a mineral acid such as sulfuric acid, hydrochloric acid or boric acid. This hydrolysis is preferably carried out in an appropriate solvent. The solvent includes all conventional solvents which do not give undesirable effect to the reaction, and suitable examples are water and lower alcohols such as methanol, ethanol, isopropanol, or the like. The reaction temperature and reaction time are not critical, but the hydrolysis is usually carried out at a temperature of room temperature to 150°C, preferably 50 to 110°C, for 30 minutes to 10 hours.

Esterification of the compounds [If] can usually be effected by reacting them with a lower alcohol in the presence of a catalyst. The catalyst includes all catalysts which are usually used in the conventional esterification, for example, inorganic acids such as hydrogen chloride gas, concentrated sulfuric acid,

phosphoric acid, polyphosphoric acid, boron trifluoride, or perchloric acid; organic acids such as trifluoroacetic acid, trifluoromethanesulfonic acid, naphthalenesulfonic acid, p-toluenesulfonic acid, benzenesulfonic acid, or ethanesulfonic acid; acid anhydrides such as trifluoromethanesulfonic acid anhydride; thionyl chloride or acetone dimethylacetal. Cation exchange resins are also used as the catalyst. Amount of the catalyst is not critical but is selected within the range as usually used.

The esterification may be carried out in the presence or absence of a solvent. The solvent includes all solvents which are usually used in the conventional esterification reaction, for example, aromatic hydrocarbons such as benzene, toluene or xylene, halogenated hydrocarbons such as dichloromethane, dichloroethane, chloroform or carbon tetrachloride, ethers such as diethyl ether, tetrahydrofuran, dioxane or ethylene glycol monomethyl ether. The ratio of amount of the compounds [If] and the lower alcohol is not critical, but preferably, when the reaction is carried out in the absence of a solvent, the latter, i.e. the lower alcohol, is used in a largely excess amount, and when the reaction is carried out in the presence of a solvent, the latter is used in an amount of 1 to 5 moles, more preferably 1 to 2 moles, to 1 mole of the former. It is also preferably to do the esterification in the presence of a drying agent such as anhydrous copper sulfate, anhydrous calcium sulfate or phosphorus pentachloride, by which the produced water is taken out from the reaction system and the yield of the desired product is increased. The reaction temperature is not critical, but the reaction is usually carried out at a temperature of −20 to 200°C, preferably 0 to 150°C. Besides, the reaction time may vary with the kinds of the starting material and other reaction conditions but is usually in the range of 10 minutes to 20 hours.

In the esterification reaction, there may be used the compounds [If] wherein the carboxyl group is activated, for example, a carboxylic acid halide or carboxylic acid anhydride. When a carboxylic acid halide is used, the same conditions as used in the carboxylic acid halide method in Reaction Scheme-I are applicable. When a carboxylic acid anhydride is used, the compounds [If] are treated with a conventional dehydrating agent to obtain a carboxylic acid anhydride and then the resulting anhydride is reacted with a lower alcohol in an usual manner.

The starting compounds used in the above Reaction Schemes-I to V are partially known and are partially novel. For instance, the compounds [II] used in Reaction Scheme-I are novel and can be prepared by the processes as shown in Reaction Schemes-II, III, IV and V. Another starting compounds [III] are partially known and include novel compounds, which are prepared by the processes as shown in the following Reaction Schemes-VI and VII:

REACTION SCHEME – VI

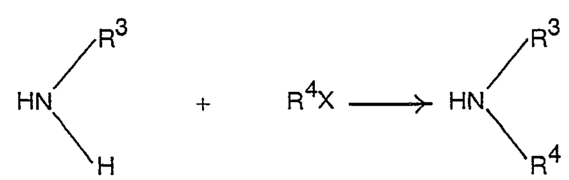

[XIII] [XIV] [III]

REACTION SCHEME – VII

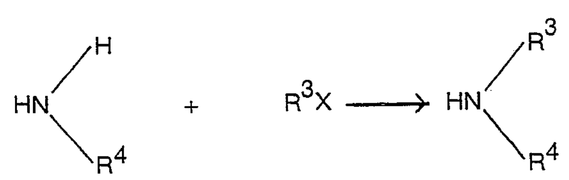

[XV] [XVI] [III]

wherein $R^3$ and $R^4$ are as defined above, and X is a halogen.

In the above Reaction Schemes-VI and VII, the reaction of an amine [XIII] and a halogen compound [XIV] and the reaction of an amine [XV] and a halogen compound [XVI] are carried out under the same reaction conditions as used in the dehydrohalogenation reaction in Reaction Scheme-II.

The compounds [IV] used in Reaction Scheme-II are known and another starting compounds [V] are

partially known and are partially novel. For example, compounds [Va] which are the compounds [V] wherein

$R^2$ is 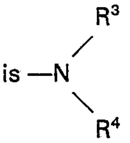

are prepared by a process as shown in the following Reaction Scheme-VIII:

REACTION SCHEME – VIII

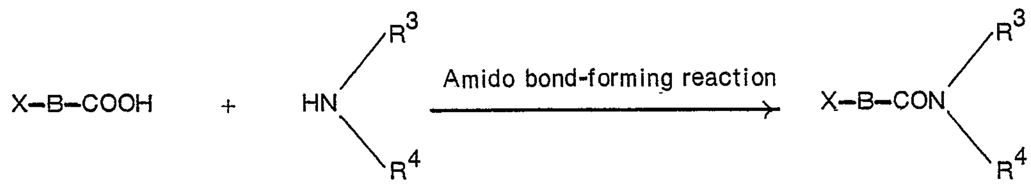


[XVII] [III] [Va]

wherein X, B, $R^3$ and $R^4$ are as defined above.

The reaction of a known halocarboxylic acid [XVII] and an amine [III] can be carried out under the same reaction conditions as used in the amido bond-forming reaction in Reaction Scheme-I.

The starting compounds used in Reaction Schemes-III and VI are known. The starting compounds used in Reaction Scheme-V can be prepared by the same process as used in Reaction Schemes-II to IV.

The compounds [I] having an acidic group may be converted into a salt thereof with a pharmaceutically acceptable basic compound. The basic compound includes metal hydroxides such as sodium hydroxide or potassium hydroxide, alkali metal alcoholates such as sodium methylate or potasssium ethylate. The compounds [I] having a basic group may also be converted into a salt with a pharmaceutically acceptable acid. The pharmaceutically acceptable acid includes inorganic acids such as sulfuric acid, nitric acid, hydrochloric acid or hydrobromic acid, and organic acids such as acetic acid, p-toluenesulfonic acid, ethanesulfonic acid, oxalic acid, maleic acid, succinic acid or benzoic acid.

The compounds obtained in the above processes can easily be isolated from the reaction mixture and purified by conventional methods. For instance, the isolation can be carried out by precipitation, extraction, recrystallization, distillation, column chromatography and preparative thin layer chromatography.

The compounds [I] or their pharmaceutically acceptable salts of the present invention are useful for the treatment of peptic and duodenal ulcers and usually used in the form of conventional pharmaceutical preparations. The pharmaceutical preparations can be prepared by using conventional diluents and carriers such as fillers, bulking agents, binding agents, wetting agents, disintegrators, surface active agents or lubricants. The preparations may be in various forms, such as tablets, pills, powders, solutions, suspensions, emulsions, granules, capsules, suppositories and injections (solution or suspension). The tablets can be prepared by using conventional carriers, such as excipients (e.g. lactose, sucrose, sodium chloride, glucose, urea, starches, calcium carbonate, kaolin, crystalline cellulose and silicic acid, binding agents (e.g. water, ethanol, propanol, simple syrup, aqueous glucose solution, aqueous solution of starches, aqueous gelatine solution, carboxymethyl cellulose, shellac, methyl cellulose, potassium phosphate and polyvinyl pyrrolidone, disintegrators (e.g. dry starches, sodium arginate, agar powder, laminaran powder, sodium hydrogen carbonate, calcium carbonate, polyoxyethylene sorbitan fatty acid esters, sodium laurylsulfate, monoglyceryl stearate, starches and lactose), disintegration inhibitors (e.g. sucrose, stearin, cacao butter and hydrogenated oils), absorption accelerators (e.g. quarternary ammonium salt and sodium laurylsulfate), humectants (e.g. glycerin and starches), adsorbents (e.g. starches, lactose, kaolin, bentonite, colloidal and silica), lubricants (e.g. purified talc, stearic acid salts, boric acid powder and polyethylene glycol), and the like. The tablets may be in the forms of sugar coated tablets, gelatin coated tablets, enteric coated tablets, film coated tablets and double or multiple layer tablets. The pills can be prepared by using conventional carriers, such as excepients (e.g. glucose, lactose, starches, cacao butter, hardened vegetable oils, kaolin and talc), binding agents (e.g. gum arabic powder, tragacanth powder, gelatin and ethanol), disintegrators (e.g. laminaran and agar). The suppositories can be prepared by using conventional carriers, such as polyethylene glycol, cacao butter, higher alcohols or their esters, gelatin or semi-synthetic glycerides. When the active compounds are prepared in the form of an injection, a solution or suspension containing the active compounds is sterilized and made isotonic to blood. The injections in the form of a solution, emulsion or suspension can be prepared by using conventional diluents, such as water, ethyl alcohol, propylene glycol ethoxylated isostearyl alcohol, polyoxy-isostearyl alcohol or polyoxyethylene sorbitan fatty esters. The injection preparation may be made isotonic by adding

thereto a sufficient amount of sodium chloride, glucose or glycerin and may optionally be incorporated with conventional solubilizers, buffer solutions, pain killers, colorants, preservatives, perfumes, flavors, sweetening agents, and other medicaments.

The anti-ulcer preparations of the present invention may contain a wide range of amount of the active compounds and contain usually 1 to 70% by weight, preferably 5 to 50% by weight, of the active compounds of the present invention based on the total weight of the preparations.

The administration route of the anti-ulcer preparations of the present invention is not restricted, and suitable administration route is determined by the forms of the preparations, age, sex and other conditions of patients to be treated and severity of disease. Tablets, pills, solutions, suspensions, emulsions, granules and capsules are usually administered in oral route. Injections are usually administered in intravenous route alone or optionally together with an appropriate adjuvant such as glucose or amino acids or may be administered alone in intramuscular, intracutaneous, subcutaneous, or intraperitoneal route.

The dose of the active compounds of the present invention may vary with the usage, age, sex and other conditions of patients to be treated, severity of disease, or the like, but is usually in the range of 0.6 to 50 mg/kg of body weight per day. The active compounds of the present invention is preferably contained in the anti-ulcer preparations in a dosage unit of 10 to 1000 mg.

Pharmacological test:

The pharmacological activities of the compounds (I) were tested by a Shay rat pylorus ligation method (cf. H. Shay et al: Gastroenterol., Vol. 5, page 43, 1945) which is the most popular method for testing of gastric juice secretion-inhibiting activity. The test was carried out using male Wistar rats, weighing about 170 g and fasting for 24 hours.

Test compounds were subcutaneously administered 30 minutes before the pylorus ligation, and the volume, total acidity and pepsin activity of the gastric juice were measured 4 hours after the ligation. As a control, a saline solution was administered instead of the test compound. The inhibitory ratio (%) of the test compounds was calculated when the inhibitory activities of the control were counted as zero (0). The results are shown in the following Table 1.

In the table, the inhibitory ratio (%) was evaluated as follows:

+ : 10 to less than 50%

++ : more than 50%.

Besides, the test compounds are as follows:

1. N,N-Diethyl-4-(1-methyl-1,2,3,4-tetrazol-5-yl)thio-butyramide
2. N-Ethyl-N-cyclohexyl-4-(1-methyl-1,2,3,4-tetrazol-5-yl)thio-butyramide
3. N-Ethyl-N-cyclohexyl-5-(1-methyl-1,2,3,4-tetrazol-5-yl)thio-valeramide
4. N-Ethyl-N-cyclohexyl-5-(1-phenyl-1,2,3,4-tetrazol-5-yl)valeramide
5. N,N-Diethyl-5-(1-cyclohexyl-1,2,3,4-tetrazol-5-yl)valeramide
6. N-Cyclohexyl-4-(1-methyl-1,2,3,4-tetrazol-5-yl)thio-butyramide
7. N-[4-(N,N-dimethylamino)phenyl]-4-(1-methyl-1,2,3,4-tetrazol-5-yl)thio-butyramide
8. N-Ethyl-N-(2-hydroxycyclohexyl)-4-(1-methyl-1,2,3,4-tetrazol-5-yl)thio-butyramide
9. N-Ethyl-N-phenyl-4-(1-methyl-1,2,3,4-tetrazol-5-yl)thio-butyramide
10. N-Ethyl-N-(2-pyridyl)-4-(1-methyl-1,2,3,4-tetrazol-5-yl)thio-butyramide
11. N-Cyclohexyl-N-[2-(3,4,-dimethoxyphenyl)ethyl]-4-(1-methyl-1,2,3,4-tetrazol-5-yl)thio-butyramide
12. N,N-Diethyl-3-[1-(4-ethylphenyl)-1,2,3,4-tetrazol-5-yl]methylthio-propionamide
13. N-Furfuryl-4-(1-methyl-1,2,3,4-tetrazol-5-yl)thio-butyramide

TABLE 1

| Test Compound No. | Dose (mg /kg, s.c.) | Inhibitory ratio | | |
|---|---|---|---|---|
| | | Volume of gastric juice | Total acidity | Pepsin activity |
| 1 | 100 | ++ | ++ | ++ |
| 2 | 100 | ++ | ++ | ++ |
| 3 | 100 | ++ | + | + |
| 4 | 100 | + | + | + |
| 5 | 100 | + | + | + |
| 6 | 100 | + | + | + |
| 7 | 100 | ++ | + | + |
| 8 | 100 | + | + | + |
| 9 | 100 | ++ | + | ++ |
| 10 | 100 | + | + | + |
| 11 | 100 | + | + | + |
| 12 | 100 | + | + | + |
| 13 | 100 | + | + | + |

Acute toxicity:

The test compounds were orally administered to male Wistar rats, and the half lethal dose ($LD_{50}$) was measured. The results are shown in Table 2.

TABLE 2

| Test Compound No. | $LD_{50}$ (mg/kg, p.o.) |
|---|---|
| 1 | > 500 |
| 2 | > 500 |
| 3 | > 500 |
| 4 | > 500 |
| 5 | > 500 |
| 6 | > 500 |
| 7 | > 500 |
| 8 | > 500 |
| 9 | > 500 |
| 10 | > 500 |
| 11 | > 500 |
| 12 | > 500 |
| 13 | > 500 |

The compounds of the present invention and preparation thereof are illustrated by the following Reference Examples and Examples, but are not limited thereto.

Reference Example 1

To ethyl acetate (400 ml) is added N-methylcyclohexylamine (26 ml), and to the mixture are added dropwise with stirring 4-chlorobutyryl chloride (25 ml) and triethylamine (33.5 ml) over a period of 20 minutes with keeping the inner temperature at 10 to 20°C by ice-cooling. The mixture is further stirred at room temperature for one hour. After the reaction, water is added to the reaction mixture. The organic layer is separated and washed with water, an aqueous saturated potassium carbonate solution, 10% aqueous hydrochloric acid and water in order, and then dried over anhydrous sodium sulfate. After filtering off sodium sulfate, the mother liquor is concentrated and distilled under reduced pressure to give N-methyl-N-cyclohexyl-4-chlorobutyramide (41.5 g), b.p. 133—136°C/2,66 mbar.

Reference Example 2

N-Ethyl-cyclohexylamine (2.6 g) is dissolved in dry benzene (20 ml). To the solution are added dropwise with stirring chloroacetyl chloride (2.6 g) and triethylamine (2.4 g) under ice-cooling. The mixture is stirred under ice-cooling for one hour and further at room temperature for one hour. The reaction mixture is poured onto ice-water and extracted with ether. The ether layer is washed with an aqueous sodium hydrogen carbonate solution and an aqueous saturated sodium chloride solution and dried over anhydrous magnesium sulfate. After distilling off the solvent, the resulting residue is distilled under reduced pressure to give N-ethyl-N-cyclohexyl-chloroacetamide (3 g), b.p. 118—120°C/0,27 mbar.

Reference Example 3 and 4

In the same manner as described in Reference Example 2, the following compounds are prepared.

N-Ethyl-N-cyclohexyl-4-chlorobutyramide, b.p. 120—130°C/2 mbar

N-Ethyl-N-cyclohexyl-3-chloropropionamide, b.p. 103—110°C/0,2 mbar.

Reference Example 5

To monomethyl adipate (6 g) is added thionyl chloride (10 ml), and the mixture is refluxed for one hour. Excess thionyl chloride is distilled off, and benzene is added to the residue and thionyl chloride is further removed by azeotropic distillation.

Separately, aniline (4.2 g) and potassium carbonate (5.2 g) are dissolved in acetone (100 ml) and water

(15 ml). To the mixture is added dropwise with stirring the chloride compound obtained above under ice-cooling. The mixture is stirred under ice-cooling for one hour and further at room temperature for 2 hours. After distilling off acetone, water is added to the residue, and the mixture is extracted with chloroform. The chloroform layer is washed with an aqueous saturated sodium chloride solution and dried over magnesium sulfate. Chloroform is distilled off to give methyl phenyladipinamate (9 g).

NMR: $\delta_{ppm}^{CDCl_3}$ 1.40—1.90 (4H, m), 2.10—2.50 (4H, m), 3.66 (3H, s), 6.80—7.70 (5H, m), 8.55 (1H, br.s)

Reference Examples 6 and 7

In the same manner as described in Reference Example 5, the following compounds are obtained.

Methyl N-cyclohexyladipinamate, faint brown prisms (recrystallized from methanol-water), m.p. 72—74°C

Methyl N-methyladipinamate, colorless liquid, b.p. 136—140°C/0,93 mbar.

Reference Example 8

A 40% aqueous methylamine solution (46.5 ml) is dissolved in acetone (300 ml). To the solution are added potassium carbonate (45.6 g) and water (100 ml), and thereto is added dropwise with stirring chloroacetyl chloride (24.3 ml) under ice-cooling. The mixture is stirred under ice-cooling for one hour and further at room temperature for 2 hours. After distilling off acetone, water is added to the residue, and the mixture is extracted with chloroform. The chloroform layer is washed with an aqueous saturated sodium chloride solution and dried over magnesium sulfate. After distilling off chloroform, the residue is distilled under reduced pressure to give N-methyl-chloroacetamide (15.5 g), colorless liquid, b.p. 107—109°C/35,9 mbar.

Reference Example 9

N-Methyl-chloroacetamide (10.8 g) is dissolved in benzene (100 ml), and thereto is added with stirring phosphorus pentachloride (10.8 g) at below 15°C. The mixture is stirred at room temperature for one hour and then allowed to stand overnight. The mixture is slowly refluxed for 2 hours. The reaction mixture is concentrated and thereto is added ice-water, and the mixture is extracted with chloroform. The chloroform layer is washed with water, a dilute aqueous sodium hydroxide solution and water, dried over magnesium sulfate and distilled to remove chloroform. The resulting residue is subjected to column chromatography (Wakogel C-200, made by Wako Pure Chemical Industry, eluting agent, chloroform: methanol = 50:1, V/V) to isolate 1-methyl-5-chloromethyl-1,2,3,4-tetrazole (8.5 g).

Example 1

4-(1-Methyl-1,2,3,4-tetrazol-5-yl)thio-butyric acid (2 g) is dissolved in dry tetrahydrofuran (30 ml), and thereto is added triethylamine (1.1 g). To the mixture is added dropwise with stirring isobutyl chloroformate (1.5 g) under ice-cooling. The mixture is stirred at room temperature for 30 minutes. To the mixture is further added dropwise with stirring diethylamine (0.9 g) at room temperature, and the mixture is stirred for 2 hours. The mixture is concentrated under reduced pressure, and the resulting residue is purified by subjecting it to column chromatography (Kieselgel 60, made by Merck & Co.). By eluting with n-hexane-ethyl acetate (1:1), there is obtained N,N-diethyl-4-(1-methyl-1,2,3,4-tetrazol-5-yl)thio-butyramide (1.4 g), colorless liquid, $n_D^{17.5}$ = 1.5227

NMR: $\delta_{ppm}^{CDCl_3}$ 1.04 (3H, t, J=7Hz), 1.12 (3H, t, J=7Hz), 1.90—2.70 (4H, m), 3.00—3.60 (6H, m), 3.88 (3H, s)

Elementary analysis for $C_{10}H_{19}N_5OS$:

Calcd. (%): C, 46.67; H, 7.44; N, 27.21

Found (%): C, 46.78; H, 7.51; N, 27.29

Examples 2 to 5

In the same manner as described in Example 1 the following compounds are obtained from appropriate starting materials.

(2) N-Ethyl-N-cyclohexyl-4-(1-methyl-1,2,3,4-tetrazol-5-yl)thio-butyramide, colorless liquid, $n_D^{17.5}$ = 1.5327

NMR: $\delta_{ppm}^{CDCl_3}$ 0.90—1.40 (3H, m), 1.20—2.00 (10H, m), 2.00—2.80 (4H, m), 3.00—3.60 (6H, m), 3.95 (3H, s), 3.50—4.50 (1H, m)

(3) N-Ethyl-N-phenyl-4-(1-methyl-1,2,3,4-tetrazol-5-yl)thio-butyramide, pale yellow liquid, $n_D^{26}$ = 1.5534

NMR: $\delta_{ppm}^{CDCl_3}$ 1.12 (3H, t, J=7Hz), 1.70—2.40 (4H, m), 3.32 (2H, t, J=7Hz), 3.74 (2H, q, J=14Hz, 7Hz), 3.90 (3H, s), 7.00—7.60 (5H, m)

(4) 5-(3-Morpholinocarbonylpropylthio)-1-methyl-1,2,3,4-tetrazole, white needles (recrystallized from petroleum ether-ethanol), m.p. 71—73°C

(5) 5-[3-(4-Acetylpiperadinocarbonyl)propylthio]-1-methyl-1,2,3,4-tetrazole, white crystalline powder (recrystallized from ligroine-acetone), m.p. 90—91.5°C

Example 6

4-(1-Methyl-1,2,3,4-tetrazol-5-yl)thio-butyric acid (45 mmole) is dissolved in tetrahydrofuran (50 ml), and thereto is added DBU (50 mmole). To the mixture is added dropwise with stirring isobutyl chloroformate (50 mmole) under ice-cooling and the mixture is stirred at room temperature for 30 minutes. To the

mixture is added dropwise 2-(3,4-dimethoxyphenyl)ethylamine (54 mmole) and the mixture is further stirred at room temperature for 2 hours. After the solvent is distilled off under reduced pressure, the resulting residue is extracted with chloroform. The chloroform layer is washed with 5% aqueous hydrochloric acid, an aqueous saturated sodium hydrogen carbonate solution and an aqueous saturated sodium chloride solution and dried over anhydrous sodium sulfate. After distilling off chloroform, the residue is subjected to column chromatography (Wakogel C—200, eluting agent, chloroform) to isolate N-[2-(3,4-dimethoxyphenyl)ethyl]-4-(1-methyl-1,2,3,4-tetrazol-5-yl)thio-butyramide (yield: 41%) which is recrystallized from hexane-chloroform, colorless flaks, m.p. 70.5—71.5°C.

Elementary analysis for $C_{16}H_{23}N_5O_3S$:

Calcd. (%): C, 52.59; H, 6.34; N, 19.16
Found (%): C, 52.51; H, 6.16; N, 19.10

Examples 7 to 58

In the same manner as described in Example 6, the following compounds are prepared from appropriate starting materials.

(7) N-Hexyl-4-(1-methyl-1,2,3,4-tetrazol-5-yl)-thio-butyramide, colorless flaks (recrystallized from hexane-ether), m.p. 41—42°C

(8) N-Cyclohexyl-4-(1-methyl-1,2,3,4-tetrazol-5-yl)thio-butyramide, colourless needles (hexane-ethyl acetate), m.p. 116.5—117.5°C

(9) N-Cyclooctyl-4-(1-methyl-1,2,3,4-tretrazol-5-yl)thio-butyramide, colorless liquid, $n_D^{14}$ = 1.5323

(10) N-Cyclododecanyl-4-(1-methyl-1,2,3,4-tetrazol-yl)thio-butyramide, colorless needles (hexane-ethyl acetate), m.p. 119—120°C

(11) N-Butyl-4-(1-methyl-1,2,3,4-tetrazol-5-yl)thio-butyramide, colorless liquid, $n_D^{16.5}$ = 1.5198

(12) N-(2-Hydroxyethyl)-4-(1-methyl-1,2,3,4-tetrazol-5-yl)thio-butyramide, colourless liquid, $n_D^{14.5}$ =1.5350

(13) N-Ethyl-N-benzyl-4-(1-methyl-1,2,3,4-tetrazol-yl)thio-butyramide, colorless liquid, $n_D^{19}$ = 1.5596

(14) N-Butyl-N-cyclohexyl-4-(1-methyl-1,2,3,4-tetrazol-5-yl)thio-butyramide, colorless liquid, $n_D^{19}$ = 1.5222

(15) N,N-Dibutyl-4-(1-methyl-1,2,3,4-tetrazol-5-yl)thio-butyramide, colorless liquid, $n_D^{19}$ = 1.5049

(16) N,N-Dibenzyl-4-(1-methyl-1,2,3,4-tetrazol-5-yl)thio-butyramide, colorless liquid, $n_D^{19}$ = 1.5773

(17) N,N-Diisopropyl-4-(1-methyl-1,2,3,4-tetrazol-5-yl)thio-butyramide, colorless liquid, $n_D^{19.5}$ = 1.5111

(18) N,N-Dicyclohexyl-4-(1-methyl-1,2,3,4-tetrazol-5-yl)thio-butyramide, colorless needles (hexane), m.p. 91—92°C

(19) N-Benzyl-N-tert-butyl-4-(1-methyl-1,2,3,4-tetrazol-5-yl)thio-butyramide, colorless needles (hexane), m.p. 86.5—87.5°C

(20) N-Cyclohexyl-N-[2-(3,4-dimethoxyphenyl)ethyl]-4-(1-methyl-1,2,3,4-tetrazol-5-yl)thio-butyramide, colorless liquid, $n_D^{16}$ = 1.5470

(21) N-Methyl-N-(2-thienylmethyl)-4-(1-methyl-1,2,3,4-tetrazol-5-yl)thio-butyramide, colorless liquid, $n_D^{16}$ = 1.5706

(22) N-Benzyl-N-[2-(3,4-dimethoxyphenyl)ethyl]-4-(1-methyl-1,2,3,4-tetrazol-5-yl)thio-butyramide, colorless liquid, $n_D^{16}$ = 1.5659

(23) N-[2-(3,4-Dimethoxyphenyl)ethyl]-N-(2-hydroxyethyl)-4-(1-methyl-1,2,3,4-tetrazol-5-yl)thio-butyramide, colorless liquid, $n_D^{14.5}$ = 1.5372

(24) N,N-Dihexyl-4-(1-methyl-1,2,3,4-tetrazol-5-yl)thio-butyramide, colorless liquid, $n_D^{15}$ = 1.5011

(25) N-Ethyl-N-[2-(3,4-dimethoxyphenyl)ethyl]-4-(1-methyl-1,2,3,4-tetrazol-5-yl)thio-butyramide, colorless liquid, $n_D^{14}$ = 1.5451

(26) N-tert-Butyl-4-(1-methyl-1,2,3,4-tetrazol-5-yl)thio-butyramide, colorless plates (hexane-ethyl acetate), m.p. 71—73°C

(27) N-Ethyl-4-(1-methyl-1,2,3,4-tetrazol-5-yl)thio-butyramide, colorless liquid, $n_D^{15}$ = 1.5319

(28) N-Benzyl-4-(1-methyl-1,2,3,4-tetrazol-5-yl)thio-butyramide, colorless needles (hexane-ethyl acetate), m.p. 65—66°C

(29) N-Hexyl-N-cyclohexyl-4-(1-methyl-1,2,3,4-tetrazol-5-yl)thio-butyramide, colorless liquid, $n_D^{16}$ = 1.5182

(30) N-Cyclohexyl-N-(2-hydroxyethyl)-4-(1-methyl-1,2,3,4-tetrazol-5-yl)thio-butyramide, colorless liquid, $n_D^{14.5}$ = 1.5372

(31) N-Phenyl-4-(1-methyl-1,2,3,4-tetrazol-5-yl)thio-butyramide, colorless needles (hexane-ethyl acetate), m.p. 106—107°C

(32) N-(2-Pyridyl)-4-(1-methyl-1,2,3,4-tetrazol-5-yl)thio-butyramide, colorless needles (hexane-ethyl acetate), m.p. 95—96°C

(33) N-(3-Pyridyl)-4-(1-methyl-1,2,3,4-tetrazol-5-yl)thio-butyramide, colorless plates (ethyl acetate), m.p. 110.5—113°C

(34) N-(2-Pyrimidyl)-4-(1-methyl-1,2,3,4-tetrazol-5-yl)thio-butyramide, colorless granules (hexane-ethyl acetate), m.p. 108—110°C

(35) N-Furfuryl-4-(1-methyl-1,2,3,4-tetrazol-5-yl)thio-butyramide, colorless flaks (hexane-ethyl acetate), m.p. 71—73°C

(36) N-(4-Aminosulfonylphenyl)-4-(1-methyl-1,2,3,4-tetrazol-5-yl)thio-butyramide, colorless needles (methanol), m.p. 169.5—170.5°C

(37) N-[4-(N,N-Dimethylamino)phenyl]-4-(1-methyl-1,2,3,4-tetrazol-5-yl)thio-butyramide, colorless prisms (hexane-ethyl acetate), m.p. 144—147°C

(38) N-(2-Methyl-3-chlorophenyl)-4-(1-methyl-1,2,3,4-tetrazol-5-yl)thio-butyramide, colorless needles (hexane-ethyl acetate), m.p. 104.5—105.5°C

(39) N-(4-Nitrophenyl)-4-(1-methyl-1,2,3,4-tetrazol-5-yl)thio-butyramide, colorless needles (ethyl acetate), m.p. 194—195°C

(40) N-(2-Methoxyphenyl)-4-(1-methyl-1,2,3,4-tetrazol-5-yl)thio-butyramide, colorless needles (hexane-ethyl acetate), m.p. 79.5—82°C

(41) N-Methyl-N-(2-tetrahydropyranyl)-4-(1-methyl-1,2,3,4-tetrazol-5-yl)thio-butyramide, colorless liquid, $n_D^{14} = 1.5273$

(42) N-Ethyl-N-(2-pyridyl)-4-(1-methyl-1,2,3,4-tetrazol-5-yl)thio-butyramide, colorless liquid, $n_D^{16} = 1.5623$

(43) N-Ethyl-N-(3-pyridyl)-4-(1-methyl-1,2,3,4-tetrazol-5-yl)thio-butyramide, colorless liquid, $n_D^{16} = 1.5618$

(44) N-Ethyl-N-cyclopentyl-4-(1-methyl-1,2,3,4-tetrazol-5-yl)thio-butyramide, colorless liquid, $n_D^{9} = 1.5384$

(45) N-Ethyl-N-cyclohexylmethyl-4-(1-methyl-1,2,3,4-tetrazol-5-yl)thio-butyramide, colorless liquid, $n_D^{11} = 1.5293$

(46) N-Isopropyol-N-cyclohexyl-4-(1-methyl-1,2,3,4-tetrazol-5-yl)thio-butyramide, colorless liquid, $n_D^{15} = 1.5238$

(47) N-Ethyl-N-(4-hydroxycyclohexyl)-4-(1-methyl-1,2,3,4-tetrazol-5-yl)thio-butyramide, colorless liquid, $n_D^{9} = 1.5363$

(48) N-Ethyl-N-(2-hydroxycylcohexyl)-4-(1-methyl-1,2,3,4-tetrazol-5-yl)thio-butyramide, colorless needles (hexane-ethyl acetate), m.p. 132—133°C

(49) N-Ethyl-N-(2-acetyloxycyclohexyl)-4-(1-methyl-1,2,3,4-tetrazol-5-yl)thio-butyramide, colorless liquid, $n_D^{11} = 1.5218$

(50) N,N-Dipropyl-4-(1-methyl-1,2,3,4-tetrazol-5-yl)thio-butyramide, colorless liquid $n_D^{15} = 1.5151$

(51) N-Butyl-N-phenyl-4-(1-methyl-1,2,3,4-tetrazol-5-yl)thio-butyramide, colorless liquid, $n_D^{15} = 1.5509$

(52) N-Methyl-N-cyclohexyl-4-(1-methyl-1,2,3,4-tetrazol-5-yl)thio-butyramide, pale yellow liquid

NMR: $\delta_{ppm}^{CCl_4}$ 1.00—2.00 (10H, br.), 1.80—2.70 (4H, m), 2.73 (3H, d, J=6Hz), 3.28 (2H, t, J=6Hz), 3.85 (3H, s), 3.20—4.50 (1H, m)

(53) N,N-Dimethyl-4-(1-methyl-1,2,3,4-tetrazol-5-yl)thio-butyramide, colorless liquid, $n_D^{16} = 1.5327$

(54) N-Ethyl-N-cyclooctyl-4-(1-methyl-1,2,3,4-tetrazol-5-yl)thio-butyramide, colorless liquid, $n_D^{16} = 1.5309$

(55) 5-[3-(4-Methylpiperazinocarbonyl)propylthio]-1-methyl-1,2,3,4-tetrazol, colorless flaks (hexane-ethyl acetate), m.p. 65—68°C

(56) 5-(3-Piperidinocarbonylpropylthio)-1-methyl-1,2,3,4-tetrazole, colorless liquid, $n_D^{25} = 1.5310$

(57) N-Ethyl-N-cycloheptyl-4-(1-methyl-1,2,3,4-tetrazol-5-yl)thio-butyramide, colorless liquid, $n_D^{18} = 1.5290$

Example 58

4-(1-Phenyl-1,2,3,4-tetrazol-5-yl)thio-butyric acid (45 mmole) is dissolved in tetrahydrofuran (50 ml) and thereto is added DBU (50 mmole). To the mixture is added dropwise with stirring isobutyl chloroformate (50 mmole) under ice-cooling. The mixture is stirred at room temperature for 30 minutes. To the mixture is added dropwise N-ethylcyclohexylamine (54 mmole) and the mixture is further stirred at room temperature for 2 hours. After distilling off the solvent under reduced pressure, the residue is extracted with chloroform. The chloroform layer is washed with 5% aqueous hydrochloric acid, and aqueous saturated sodium hydrogen carbonate solution and an aqueous saturated sodium chloride solution and dried over anhydrous sodium sulfate. Chloroform is distilled off and the resulting residue is subjected to column chromatography (Wakogel C—200, eluting solvent, chloroform) to isolate N-ethyl-N-cyclohexyl-4-(1-phenyl-1,2,3,4-tetrazol-5-yl)thio-butyramide (yield: 43%), colorless liquid, $n_D^{18} = 1.5590$.

Elementary analysis for $C_{19}H_{27}N_5OS$:

Calcd. (%): C, 61.10; H, 7.29; N, 18.75

Found (%): C, 61.28; H, 7.38; N, 18.86

Example 59

In the same manner as described in Example 58 except that N,N-diethylamine is used instead of N-ethyl-N-cyclohexylamine, there is obtained N,N-diethyl-4-(1-phenyl-1,2,3,4-tetrazol-5-yl)thio-butyramide, colorless liquid, $n_D^{18} = 1.5592$.

Example 60

2-(1-Methyl-1,2,3,4-tetrazol-5-yl)thio-acetic acid (45 mmole) is dissolved in tetrahydrofuran (50 ml) and thereto is added DBU (50 mmole). To the mixture is added dropwise with stirring isobutyl chloroformate

(50 mmole) under ice-cooling and the mixture is stirred at room temperature for 30 minutes. To the mixture is added dropwise N-ethyl-cyclohexylamine under ice-cooling, and the mixture is further stirred at room temperature for 2 hours. After distilling off the solvent under reduced pressure, the resulting residue is extracted with chloroform. The chloroform layer is washed with 5% aqueous hydrochloric acid, an aqueous saturated sodium hydrogen carbonate solution and an aqueous saturated sodium chloride solution and dried over anhydrous sodium sulfate. Chloroform is distilled off and the residue is subjected to column chromatography (Wakogel C—200, eluting solvent, benzene:chloroform = 4:1, V/V) to isolate N-ethyl-N-cyclohexyl-2-(1-methyl-1,2,3,4-tetrazol-5-yl)thio-acetamide (yield: 52%) which is recrystallized from ether-petroleum ether, white prisms, m.p. 69—71°C.

Elementary analysis for $C_{12}H_{21}N_5OS$:
Calcd. (%): C, 50.86; H, 7.47; N, 24.71
Found (%): C, 50.75; H, 7.55; N, 24.78

Examples 61 and 62

In the same manner as described in Example 60, the following compounds are prepared by using appropriate starting materials.

(61) N-Ethyl-N-cyclohexyl-3-(1-methyl-1,2,3,4-tetrazol-5-yl)thio-propionamide, pale yellow liquid, $n_D^{26}$ = 1.5273

(62) N-Ethyl-N-cyclohexyl-5-(1-methyl-1,2,3,4-tetrazol-5-yl)thio-valeramide, pale yellow liquid, $n_D^{25}$ = 1.5227

Example 63

4-(1-Cyclohexyl-1,2,3,4-tetrazol-5-yl)thiobutyric acid (45 mmole) is dissolved in tetrahydrofuran (50 ml) and thereto is added DBU (50 mmole). To the mixture is added dropwise with stirring isobutyl chloroformate under ice-cooling, and the mixture is stirred at room temperature for 30 minutes. To the mixture is added dropwise N-ethyl-cyclohexylamine (54 mmole), and the mixture is further stirred at room temperature for 2 hours. After distilling off the solvent under reduced pressure, the residue is extracted with chloroform. The chloroform layer is washed with 5% aqueous hydrochloric acid, an aqueous saturated sodium hydrogen carbonate and an aqueous saturated sodium chloride solution and dried over anhydrous sodium sulfate. Chloroform is distilled off and the resulting residue is subjected to column chromatography (Wakogel C—200, eluting solvent, benzene:chloroform = 4:1, V/V) to isolate N-ethyl-N-cyclohexyl-4-(1-cyclohexyl-1,2,3,4-tetrazol-5-yl)thiobutyramide (yield: 47%), colorless liquid, $n_D^{18}$ = 1.5290.

Elementary analysis for $C_{19}H_{33}N_5OS$:
Calcd. (%): C. 60.12; H, 8.76; N, 18.45
Found (%): C, 59.95; H, 8.62; N, 18.55

Example 64

To methylene chloride (50 ml) are added 4-(1-methyl-1,2,3,4-tetrazol-5-yl)thio-butyric acid (45 mmole) and N-methylmorpholine (50 mmole). To the mixture is added dropwise with stirring methyl chloroformate (50 mmole) with keeping the inner temperature at 10—20°C by ice-cooling, and thereafter, the mixture is stirred at room temperature for 30 minutes. To the mixture is added 2-methoxycyclohexylamine (54 mmole), and the mixture is stirred at the same temperature for 4 hours. After the reaction, water is added to the reaction mixture. The organic layer is separated and washed with dilute aqueous sodium hydroxide solution, diluted hydrochloric acid and water in order, and dried over sodium sulfate. Inorganic materials are filtered off, and the mother liquor is concentrated. The resulting residue is subjected to column chromatography (Wakogel C—200, eluting solvent, benzene:chloroform = 4:1, V/V) to isolate N-(4-methoxycyclohexyl)-4-(1-methyl-1,2,3,4-tetrazol-5-yl)thio-butyramide (yield: 43%), colorless liquid, $n_D^{11}$ = 1.5263.

Elementary analysis for $C_{13}H_{23}N_5O_2S$:
Calcd. (%): C, 52.50; H, 7.79; N, 23.55
Found (%): C, 52.56; H, 7.71; N, 23.63

Example 65

To tetrahydrofuran (50 ml) are added 4-(1-methyl-1,2,3,4-tetrazol-5-yl)thio-butyric acid (45 mmole) and pyridine (50 mmole), and thereto is added dropwise with stirring methyl bromoformate (50 mmole) with keeping the inner temperature at 5—15°C by ice-cooling, and thereafter, the mixture is stirred at room temperature for one hour. To the mixture is added 4-methylcyclohexylamine (55 mmole), and the mixture is further stirred for 3 hours. After the reaction, the solvent is distilled off under reduced pressure, and the residue is dissolved in chloroform. The chloroform layer is washed with dilute aqueous sodium hydroxide solution, diluted hydrochloric acid and water in order, and dried over sodium sulfate. Inorganic materials are filtered off and the mother liquor is concentrated. The resulting residue is subjected to column

chromatography (Wakogel C—200, eluting solvent, benzene:chloroform = 4:1, V/V) to isolate N-(4-methylcyclohexyl)-4-(1-methyl-1,2,3,4-tetrazol-5-yl)thio-butyramide (yield: 45%).

Elementary analysis for $C_{13}H_{23}N_5OS$:
Calcd. (%): C, 52.50; H, 7.79; N, 23.55
Found (%): C, 52.31; H, 7.65; N, 23.80

Example 66

4-(1-Methyl-1,2,3,4-tetrazol-5-yl)thio-butyric acid (45 mmole) is dissolved in tetrahydrofuran (50 ml), and thereto is added DBU (50 mmole). To the mixture is added dropwise with stirring isobutyl chloroformate (50 mmole) under ice-cooling, and the mixture is stirred at room temperature for 30 minutes. To the mixture is added dropwise 4-(N,N-dimethylamino)cyclohexylamine (54 mmole), and the mixture is further stirred at room temperature for 2 hours. After distilling off the solvent under reduced pressure, the resulting residue is extracted with chloroform. The chloroform layer is washed with 5% aqueous hydrochloric acid, an aqueous saturated sodium hydrogen carbonate solution and an aqueous saturated sodium chloride solution and then dried over anhydrous sodium sulfate. Chloroform is distilled off, and the resulting residue is subjected to silica gel column chromatography (Wakogel C—200, eluting solvent, benzene:chloroform = 4:1, V/V) to isolate N-[4-(N,N-dimethylamino)cyclohexyl]-4-(1-methyl-1,2,3,4-tetrazol-5-yl)thiobutyramide (yield: 47%).

Elementary analysis for $C_{14}H_{26}N_6OS$:
Calcd. (%): C, 51.51; H, 8.03; N, 25.74
Found (%): C, 51.60; H, 8.22; N, 26.05

Example 67

4-[1-(2-Methoxycyclohexyl)-1,2,3,4-tetrazol-5-yl]thio-butyric acid (45 mmole) is dissolved in tetrahydrofuran (50 ml), and thereto is added DBU (50 mmole). To the mixture is added dropwise with stirring isobutyl chloroformate (50 mmole) under ice-cooling, and the mixture is stirred at room temperature for 30 minutes. To the mixture is added dropwise N-ethyl-cyclohexylamine (54 mmole), and the mixture is further stirred at room temperature for 2 hours. The solvent is distilled off under reduced pressure, and the residue is extracted with chloroform. The chloroform layer is washed with 5% aqueous hydrochloric acid, an aqueous saturated sodium hydrogen carbonate solution and an aqueous saturated sodium chloride solution and then dried over anhydrous sodium sulfate. After distilling off chloroform, the resulting residue is subjected to column chromatography (Wakogel C—200, eluting solvent, benzene:chloroform = 4:1, V/V) to isolate N-ethyl-N-cyclohexyl-4-[1-(2-methoxycyclohexyl)-1,2,3,4-tetrazol-5-yl]thiobutyramide (yield: 45%).

Elementary analysis for $C_{20}H_{35}N_5O_2S$:
Calcd. (%): C, 58.65; H, 8.61; N, 17.10
Found (%): C, 58.25; H,8.34; N, 17.20

Example 68

4-[1-(4-Ethylphenyl)-1,2,3,4-tetrazol-5-yl]thio-butyric acid (45 mmole) is dissolved in tetrahydrofuran (50 ml), and thereto is added DBU (50 mmole). To the mixture is added dropwise with stirring isobutyl chloroformate (50 mmol) under ice-cooling, and the mixture is stirred at room temperature for 30 minutes. To the mixture is added dropwise N-ethyl-cyclohexylamine (54 mmole), and the mixture is further stirred at room temperature for 2 hours. The solvent is distilled off under reduced pressure and the resulting residue is extracted with chloroform. The chloroform layer is washed with 5% aqueous hydrochloric acid, an aqueous saturated sodium hydrogen carbonate solution and an aqueous saturated sodium chloride solution and then dried over anhydrous sodium sulfate. Chloroform is distilled off and the resulting residue is subjected to column chromatography (Wakogel C—200, eluting solvent, benzene:chloroform = 4:1, V/V) to isolate N-ethyl-N-cyclohexyl-4-[1-(4-ethylphenyl)-1,2,3,4-tetrazol-5-yl]thio-butyramide (yield: 43%), colorless liquid, $n_D^{19}$ = 1.5533.

Elementary analysis for $C_{21}H_{31}N_5OS$:
Calcd. (%): C, 62.81; H, 7.78; N, 17.44
Found (%): C, 63.05; H, 7.84; N, 17.81

Example 69

5-(1-Phenyl-1,2,3,4-tetrazol-5-yl)valeric acid (2.5 g) is dissolved in dimethylformamide (50 ml), and thereto is added triethylamine (1.1 g). To the mixture is added dropwise with stirring isobutyl chloroformate (1.5 g) under ice-cooling, and the mixture is stirred at room temperature for 30 minutes. To the mixture is added dropwise with stirring diethylamine (0.9 g) at room temperature, and the mixture is stirred for 2 hours. After distilling off dimethylformamide under reduced pressure, acetone is added to the resulting residue, and the insoluble materials are filtered off. The mother liquor is concentrated and then purified by column chromatography (Kieselgel 60, made by Merck & Co.). By eluting with chloroform, there

is obtained N,N-diethyl-5-(1-phenyl-1,2,3,4-tetrazol-5-yl)valeramide (1.2 g), colorless liquid, $n_D^{25}$ = 1.5303.

Elementary analysis for $C_{16}H_{23}N_5O$:

Calcd. (%): C, 56.40; H, 6.63; N, 21.92

Found (%): C, 56.69; H, 6.80; N, 21.71

Example 70

5-(1-Cyclohexyl-1,2,3,4-tetrazol-5-yl)valeric acid (2.5 g) is dissolved in tetrahydrofuran (50 ml), and thereto is added triethylamine (1.1 g). To the mixture is added dropwise with stirring isobutyl chloroformate (1.5 g) under ice-cooling, and the mixture is stirred at room temperature for 30 minutes. To the mixture is added dropwise with stirring N-ethyl-cyclohexylamine (1.5 g) at room temperature, and the mixture is further stirred for 2 hours. After distilling off tetrahydrofuran, water is added to the residue, and the mixture is extracted with chloroform. The chloroform layer is washed with an aqueous saturated sodium chloride solution and dried over magnesium sulfate. Chloroform is distilled off, and the resulting residue is subjected to column chromatography (Kieselgel 60, made by Merck & Co., eluting solvent, chloroform) to isolate N-ethyl-N-cyclohexyl-5-(1-cyclohexyl-1,2,3,4-tetrazol-5-yl)valeramide (2.6 g), colorless liquid, $n_D^{25}$ = 1.4970.

Elementary analysis for $C_{20}H_{35}N_5O$:

Calcd. (%): C, 66.44; H, 9.76; N, 19.37

Found (%): C, 66.78; H, 9.56; N, 19.52

Example 71

5-(1-Methyl-1,2,3,4-tetrazol-5-yl)valeric acid (1.8 g) is dissolved in dimethylformamide (50 ml), and thereto is added triethylamine (1.1 g). To the mixture is added dropwise with stirring isobutyl chloroformate (1.5 g) under ice-cooling, and the mixture is stirred at room temperature for 30 minutes. To the mixture is added dropwise with stirring diethylamine (0.9 g) at room temperature, and the mixture is stirred for 2 hours. Dimethylformamide is distilled off under reduced pressure, and the residue is purified by column chromatography (Wakogel C-200). After eluting with benzene-chloroform (1:1), the eluate is distilled under reduced pressure to give N,N-diethyl-5-(1-methyl-1,2,3,4-tetrazol-5-yl)valeramide (0.7 g), colorless liquid, b.p. 190—200°C (bath temperature)/0.09 mbar, $n_D^{25}$ = 1.4907.

Elementary analysis for $C_{11}H_{21}N_5O$:

Calcd. (%): C, 55.20; H, 8.85; H, 29.27

Found (%): C, 55.48; H, 8.98; N, 29.43

Examples 72 to 76

In the same manner as described in Example 71, the following compounds are prepared by using appropriate starting materials.

(72) N,N-Diethyl-5-(1-cyclohexyl-1,2,3,4-tetrazol-5-yl)valeramide, colorless liquid, $n_D^{25}$ = 1.4970

(73) N,N-Diethyl-5-(1,2,3,4-tetrazol-5-yl)valeramide, colorless liquid, $n_D^{25}$ = 1.4867

(74) N-Ethyl-N-cyclohexyl-5-(1-methyl-1,2,3,4-tetrazol-5-yl)valeramide, colorless liquid, $n_D^{18}$ = 1.5085

(75) N-Ethyl-N-cyclohexyl-5-(1-cyclohexyl-1,2,3,4-tetrazol-5-yl)valeramide, white crystalline powder (ether), m.p. 92—95°C

(76) N-Ethyl-N-cyclohexyl-5-(1-phenyl-1,2,3,4-tetrazol-5-yl)valeramide, white prisms (ether), m.p. 73—75°C.

Example 77

3-(1-Methyl-1,2,3,4-tetrazol-5-yl)methylthiopropionic acid (2 g) is dissolved in tetrahydrofuran (50 ml), and thereto is added triethylamine (1.1 g). To the mixture is added dropwise with stirring isobutyl chloroformate (1.5 g) under ice-cooling, and the mixture is stirred at room temperature for 30 minutes. To the mixture is added dropwise with stirring diethylamine (0.9 g) at the same temperature, and the mixture is further stirred for 3 hours. After distilling off the solvent, the resulting residue is subjected to column chromatography (Kieselgel 60, made by Merck & Co., eluting solvent, chloroform:methanol = 50:1, V/V) to isolate N,N-diethyl-3-(1-methyl-1,2,3,4-tetrazol-5-yl)methylthio-propionamide (1.5 g), colorless liquid, $n_D^{25}$ = 1.5200.

Elementary analysis for $C_{10}H_{19}N_5OS$:

Calcd. (%): C, 46.67; H, 7.44; N, 27.21

Found (%): C, 46.85; H, 7.61 N, 27.39

Examples 78 to 80

In the same manner as described in Example 77, the following compounds are prepared by using appropriate starting materials.

(78) N,N-Diethyl-3-(1-ethyl-1,2,3,4-tetrazol-5-yl)methylthio-propanamide, colorless needles (recrystallized from ether), m.p. 58—59°C

(79) N,N-Diethyl-3-[1-(4-ethylphenyl)-1,2,3,4-tetrazol-5-yl)]methylthiopropanamide, colorless liquid, $n_D^{25}$ = 1.5499

(80) N-Ethyl-N-cyclohexyl-3-(1-ethyl-1,2,3,4-tetrazol-5-yl)methylthio-propionamide, pale yellow liquid, $n_D^{18}$ = 1.5277.

Example 81

Thionyl chloride (10 ml) is added to 4-(1-methyl-1,2,3,4-tetrazol-5-yl)thio-butyric acid (2 g) and the mixture is refluxed for 1 hour. After excess thionyl chloride is distilled off under reduced pressure, dry benzene is added to the residue and the remaining small amount of thionyl chloride is removed as the benzene azeotrope. The residue is dissolved in dry benzene (50 ml) and thereto is added dropwise with stirring N-methylcyclohexylamine (2.8 g) under ice-cooling. The mixture is stirred at room temperature for 1 hour and benzene is added to the reaction mixture. The mixture is washed with dilute hydrochloric acid, aqueous saturated sodium bicarbonate solution and then, saturated aqueous sodium chloride solution and dried over sodium sulfate. After benzene is distilled off, the residue is subjected to column chromatography (Wakogel C-200) by eluting the column with benzene-chloroform (4:1, V/V) to give N-methyl-N-cyclohexyl-4-(1-methyl-1,2,3,4-tetrazol-5-yl)-thiobutyramide (2.3 g), pale yellow liquid.

NMR: $\delta_{ppm}^{CCl_4}$ 1.00—2.00 (10H, br), 1.80—2.70 (4H, m), 2.73 (3H, d, J=6Hz), 3.28 (2H, t, J=6Hz), 3.85 (3H, s), 3.20—4.50 (1H, m)

Elementary analysis for $C_{13}H_{23}N_5OS$:

| | | | |
|---|---|---|---|
| Calcd. (%): | C, 52.50; | H, 7.79; | N, 23.55 |
| Found (%): | C, 52.69; | H, 7.83; | N, 23.51 |

Examples 82 to 100

According to the same procedure as described in Example 81, the following compounds are obtained.

(82) N-Ethyl-N-cyclohexyl-4-(1-methyl-1,2,3,4-tetrazol-5-yl)thio-butyramide, colorless liquid, $n_D^{17.5}$ = 1.5327

(83) N-Ethyl-N-phenyl-4-(1-methyl-1,2,3,4-tetrazol-5-yl)thio-butyramide, pale yellow liquid, $n_D^{26}$ = 1.5534

(84) 5-(3-Morpholinocarbonylpropylthio)-1-methyl-1,2,3,4-tetrazole, white needles (recrystallized from petroleum ether-ethanol), m.p. 71—73°C

(85) 5-[3-(4-Acethylpiperazinocarbonyl)propylthio]-1-methyl-1,2,3,4-tetrazole, white crystalline powder (recrystallized from ligroine-acetone), m.p. 90—91.5°C

(86) N-[2-(3,4-Dimethoxyphenylethyl]-4-(1-methyl-1,2,3,4-tetrazol-5-yl)thio-butyramide, colorless flakes (recrystallized from hexane-ethyl acetate), m.p. 70.5—71.5°C

(87) N-(2-Hydroxyethyl)-4-(1-methyl-1,2,3,4-tetrazol-5-yl)thio-butyramide, colorless liquid, $n_D^{14.5}$ = 1.5350

(88) N-Ethyl-N-benzyl-4-(1-methyl-1,2,3,4-tetrazol-5-yl)thio-butyramide, colorless liquid, $n_D^{19}$ = 1.5596

(89) N-Cyclohexyl-N-[2-(3,4-dimethoxyphenyl)-ethyl]-4-(1-methyl-1,2,3,4-tetrazol-5-yl)thio-butyramide, colorless liquid, $n_D^{16}$ = 1.5470

(90) N-Methyl-N-(2-thienylmethyl)-4-(1-methyl-1,2,3,4-tetrazol-5-yl)thio-butyramide, colorless liquid, $n_D^{16}$ = 1.5706

(91) N-(2-Pyridyl)-4-(1-methyl-1,2,3,4-tetrazol-5-yl)thio-butyramide, colorless needles (hexane-ethyl acetate), m.p. 95—96°C

(92) N-Furfuryl-4-(1-methyl-1,2,3,4-tetrazol-5-yl)thio-butyramide, colorless flakes (hexane-ethyl acetate), m.p. 71—73°C

(93) N-[4-(N,N-Dimethylamino)phenyl]-4-(1-methyl-1,2,3,4-tetrazol-5-yl)thio-butyramide, colorless prisms (hexane-ethyl acetate), m.p. 144—147°C

(94) N-(2-Methyl-3-chlorophenyl)-4-(1-methyl-1,2,3,4-tetrazol-5-yl)thio-butyramide, colorless needles (hexane-ethyl acetate), m.p. 104.5—105.5°C

(95) N-Methyl-N-(2-tetrahydropyranyl)-4-(1-methyl-1,2,3,4-tetrazol-5-yl)thio-butyramide, colorless liquid, $n_D^{14}$ = 1.5273

(96) N-Ethyl-N-cyclohexylmethyl-4-(1-methyl-1,2,3,4-tetrazol-5-yl)thio-butyramide, colorless liquid, $n_D^{11}$ = 1.5293

(97) N-Ethyl-N-(4-hydroxycyclohexyl)-4-(1-methyl-1,2,3,4-tetrazol-5-yl)thio-butylamide, colorless liquid, $n_D^{9}$ = 1.5363

(98) N-Ethyl-N-(2-acetyloxycyclohexyl)-4-(1-methyl-1,2,3,4-tetrazol-5-yl)thio-butyramide, colorless liquid, $n_D^{11}$ = 1.5218

(99) 5-(3-Piperidinocarbonylpropylthio)-1-methyl-1,2,3,4-tetrazole, colorless liquid, $n_D^{25}$ = 1.5310

(100) N-Ethyl-N-cyclohexyl-4-(1-phenyl-1,2,3,4-tetrazol-5-yl)thio-butyramide, colorless liquid, $n_D^{18}$ = 1.5590.

Example 101

Thionyl chloride (10 ml) is added to 5-(1-phenyl-1,2,3,4-tetrazol-5-yl)valeric acid (1.8 g) and the mixture is refluxed for 1 hour. After excess thionyl chloride is distilled off under reduced pressure, dry benzene is added to the residue and the remaining small amount of thionyl chloride is removed as the benzene azeotrope. The resulting residue is dissolved in dry pyridine (50 ml) and thereto is added dropwise with

stirring N,N-Diethylamine (1.5 g) under ice-cooling. The mixture is stirred at room temperature for 1 hour and benzene is added to the reaction mixture. The mixture is washed with diluted hydrochloric acid, aqueous saturated sodium bicarbonate solution and aqueous saturated sodium chloride solution and dried over sodium sulfate. After benzene is distilled off, the residue is subjected to column chromatography (Kieselgel 60 produced by Merck) by eluting the column with chloroform to give N,N-diethyl-5-(1-phenyl-1,2,3,4-tetrazol-5-yl)valeramide (2.1 g), colorless liquid, $n_D^{25}$ = 1.5303.

Elementary analysis for $C_{16}H_{23}N_5O$:

Calcd. (%): C, 63.76; H, 7.69; N, 23.24
Found (%): C, 63.88; H, 7.79; N, 23.31

Examples 102—104

According to the same procedure as described in Example 101, the following compounds are obtained.

(102) N-Ethyl-N-cyclohexyl-5-(1-cyclohexyl-1,2,3,4-tetrazol-5-yl)valeramide, white crystalline powder (recrystallized from ether), m.p. 92—95°C

(103) N-Ethyl-N-cyclohexyl-5-(1-methyl-1,2,3,4-tetrazol-5-yl)valeramide, colorless liquid, $n_D^{18}$ = 1.5085

(104) N-Ethyl-N-cyclohexyl-5-(1-phenyl-1,2,3,4-tetrazol-5-yl)valeramide, white prisms (ether), m.p. 73—75°C.

Example 105

4-(1-Methyl-1,2,3,4-tetrazol-5-yl)thio-butyric acid (2 g) and N-ethyl-N-cyclohexylamine (1.4 g) are added to a mixed solvent of dioxane (20 ml) and methylene chloride (20 ml) and thereto is added dropwise with stirring a solution of N,N'-dicyclohexylcarbodiimide (2.1 g) in methylene chloride (5 ml), which is maintained at the temperature of 10 to 20°C, with stirring while cooling externally with ice. After addition of the solution, stirring is continued at the same temperature for 5 hours. Crystals separated out from the reaction mixture are filtered off and the filtrate is concentrated under reduced pressure. The resulting residue is dissolved in methylene chloride (100 ml). The organic layer is washed with 5% aqueous hydrochloric acid, 5% aqueous sodium bicarbonate solution and water and dried over sodium sulfate. The solvent is distilled off under reduced pressure and the resulting residue is subjected to column chromatography (Wakogel C-200) by eluting the column with chloroform to give N-ethyl-N-cyclohexyl-4-(1-methyl-1,2,3,4-tetrazol-5-yl)-thio-butyramide (0.8 g), colorless liquid, $n_D^{17.5}$ = 1.5327.

Elementary analysis for $C_{14}H_{25}N_5OS$:

Calcd. (%): C, 53.99; H, 8.09; N, 22.49
Found (%): C, 54.12; H, 8.14; N, 22.56

Examples 106 to 115

According to the same procedure as described in Example 105, the following compounds are obtained:

(106) N-Ethyl-N-phenyl-4-(1-methyl-1,2,3,4-tetrazol-5-yl)thiobutyramide, pale yellow liquid, $n_D^{26}$ = 1.5534

(107) 5-(3-Morpholinocarbonylpropylthio)-1-methyl-1,2,3,4-tetrazole, white needles (recrystallized from petroleum ether-ethanol), m.p. 71—73°C

(108) N-[2-(3,4-Dimethoxyphenyl)ethyl]-4-(1-methyl-1,2,3,4-tetrazol-5-yl)thio-butyramide, colorless flakes (recrystallized from hexane-ethyl acetate), m.p. 70.5—71.5°C

(109) N-Ethyl-N-benzyl-4-(1-methyl-1,2,3,4-tetrazol-5-yl)thio-butyramide, colorless liquid, $n_D^{19}$ = 1.5596

(110) N-Methyl-N-(2-thienylmethyl)-4-(1-methyl-1,2,3,4-tetrazol-5-yl)thiobutyramide, colorless liquid, $n_D^{16}$ = 1.5706

(111) N-Furfuryl-4-(1-methyl-1,2,3,4-tetrazol-5-yl)thio-butyramide, colorless flakes, m.p. 71—73°C

(112) N-Ethyl-N-cyclohexylmethyl-4-(1-methyl-1,2,3,4-tetrazol-5-yl)thio-butyramide, colorless liquid, $n_D^{11}$ = 1.5293

(113) N-Ethyl-N-cyclohexyl-4-(1-phenyl-1,2,3,4-tetrazol-5-yl)thio-butyramide, colorless liquid, $n_D^{18}$ = 1.5590

(114) N-Ethyl-N-cyclohexyl-5-(1-cyclohexyl-1,2,3,4-tetrazol-5-yl)valeramide, white crystalline powder (ether), m.p. 92—95°C

(115) N-Ethyl-N-cyclohexyl-5-(1-phenyl-1,2,3,4-tetrazol-5-yl)valeramide, white prisms (ether), m.p. 73—75°C.

Example 116

p-Nitrophenyl 4-(1-methyl-1,2,3,4-tetrazol-5-yl)thio-butyrate (3.2 g) is dissolved in dimethylformamide (40 ml) and thereto is added cyclohexylamine (2.0 g). The reaction mixture is allowed to stand at room temperature overnight and then, concentrated under reduced pressure. The resulting residue is subjected to column chromatography (Wakogel C-200) by eluting the column with benzene-chloroform (4:1, V/V) and

recrystallized from hexane-ethyl acetate to give N-cyclohexyl-4-(1-methyl-1,2,3,4-tetrazol-5-yl)thio-butyramide (1.8 g), colorless needles, m.p. 116.5—117.5°C

Elementary analysis for $C_{12}H_{21}N_5OS$:

Calcd. (%): C, 50.86; H, 7.47; N, 24.71

Found (%): C, 50.85; H, 7.37; N, 24.65

Examples 117 to 123

According to the same procedure as described in Example 116, the following compounds are obtained.

(117) N-Ethyl-N-cyclohexyl-4-(1-methyl-1,2,3,4-tetrazol-5-yl)thio-butyramide, colorless liquid, $n_D^{17.5} = 1.5327$

(118) N-Ethyl-N-phenyl-4-(1-methyl-1,2,3,4-tetrazol-5-yl)thiobutyramide, pale yellow liquid, $n_D^{26} = 1.5534$

(119) N-[2-(3,4-Dimethoxyphenyl)ethyl-4-(1-methyl-1,2,3,4-tetrazol-5-yl)thio-butyramide, colorless flakes (hexane-ethyl acetate), m.p. 70.5—71.5°C

(120) N-Furfuryl-4-(1-methyl-1,2,3,4-tetrazol-5-yl)thio-butyramide, colorless flakes (hexane-ethyl acetate), m.p. 71—73°C

(121) N-Ethyl-N-cyclohexylmethyl-4-(1-methyl-1,2,3,4-tetrazol-5-yl)thio-butyramide, colorless liquid, $n_D^{11} = 1.5293$

(122) N-Ethyl-N-cyclohexyl-5-(1-phenyl-1,2,3,4-tetrazol-5-yl)valeramide, white prisms (ether), m.p. 73—75°C

(123) N-Ethyl-N-cyclohexyl-5-(1-cyclohexyl-1,2,3,4-tetrazol-5-yl)valeramide, white crystalline powder (ether), m.p. 92—95°C

Example 124

Methyl 4-(1-methyl-1,2,3,4-tetrazol-5-yl)thio-butyrate (1.4 g), sodium ethylate (0.5 g) and N-ethylcyclohexylamine (5 ml) are added to ethanol (50 ml) and the mixture is reacted in an autoclave at 140—150°C under the pressure of 110 atmospheric pressure for 6 hours. After cooling, the reaction mixture is concentrated under reduced pressure and the resulting residue is dissolved in chloroform (200 ml). The solution is washed with 1% aqueous potassium carbonate solution, diluted hydrochloric acid and water and dried over sodium sulfate. After the solvent is distilled off, the resulting residue is subjected to column chromatography (Wakogel C-200) by eluting the column with chloroform to give N-ethyl-N-cyclohexyl-4-(1-methyl-1,2,3,4-tetrazol-5-yl)thio-butyramide (0.6 g), colorless liquid, $n_D^{17.5} = 1.5327$.

Elementary analysis for $C_{14}H_{25}N_5OS$:

Calcd. (%): C, 53.99; H, 8.09; N, 22.49

Found (%): C, 54.19; H, 8.21; N, 22.68

Examples 125 to 127

According to the same procedure as described in Example 124, the following compounds are obtained.

(125) N-[2-(3,4-Dimethoxyphenyl)ethyl]-4-(1-methyl-1,2,3,4-tetrazol-5-yl)thio-butyramide, colorless flakes (hexane-ethyl acetate), m.p. 70.5—71.5°C

(126) N-Phenyl-4-(1-methyl-1,2,3,4-tetrazol-5-yl)thio-butyramide, colorless needles (hexane-ethyl acetate), m.p. 106—107°C

(127) N-Ethyl-N-cyclohexyl-5-(1-cyclohexyl-1,2,3,4-tetrazol-5-yl)valeramide, white crystalline powder (ether), m.p. 92—95°C.

Example 128

N-Ethyl-N-cyclohexyl-chloroacetamide (3 g) and 1-methyl-5-mercapto-1,2,3,4-tetrazole (1.8 g) are dissolved in acetone (50 ml) and thereto is added potassium carbonate. The mixture is refluxed for 3 hours and acetone is distilled off under reduced pressure. After addition of water, the residue is extracted with ether. The ether solution is washed with aqueous saturated sodium chloride solution and dried over magnesium sulfate. After ether is distilled off, the resulting residue is recrystallized from ether-petroleum ether to give N-ethyl-N-cyclohexyl-2-(1-methyl-1,2,3,4-tetrazol-5-yl)thio-acetamide (1.5 g), white prisms, m.p. 69—71°C.

Elementary analysis for $C_{12}H_{21}N_5OS$:

Calcd. (%): C, 50.86; H, 7.47; N, 24.71

Found (%): C, 50.78; H, 7.57; N, 24.75

Example 129

N-Methyl-N-cyclohexyl-4-chlorobutyramide (6.6 g) is dissolved in dry benzene (50 ml) and thereto are added 1-methyl-5-mercapto-1,2,3,4-tetrazole (3.6 g), potassium carbonate (4.5 g) and sodium iodide (0.2 g). The mixture is refluxed for 5 hours and diluted with benzene. The reaction mixture is washed with water, aqueous saturated sodium bicarbonate solution and aqueous saturated sodium chloride solution and dried over sodium sulfate. After benzene is distilled off, the resulting residue is subjected to column chromatography (Wakogel C-200) by eluting the column with benzene-chloroform (4:1, V/V) to give N-methyl-N-cyclohexyl-4-(1-methyl-1,2,3,4-tetrazol-5-yl)thio-butyramide (5 g), pale yellow liquid.

NMR: $\delta^{CCl_4}_{ppm}$ 1.00—2.00 (10H, br), 1.80—2.70 (4H, m), 2.73 (3H, d, J=6Hz), 3.28 (2H, t, J=6Hz), 3.85 (3H, s), 3.20—4.50 (1H, m).

Elementary analysis for $C_{13}H_{23}N_5OS$:

Calcd. (%): C, 52.50; H, 7.79; N, 23.55
Found (%): C, 52.72; H, 7.85; N, 23.61

Example 130

1-Methyl-5-mercapto-1,2,3,4-tetrazole (1.6 g), potassium hydroxide (0.9 g), sodium iodide (3.2 g) and 3-morpholinocarbonylpropyl chloride (5.1 g) are added to ethanol (30 ml) and the mixture is stirred at 70—80°C for 4.5 hours. After completion of the reaction, the reaction mixture is poured into aqueous saturated sodium chloride solution and the precipitating crystals are separated out by filtration and washed with water. The crude crystals thus obtained are recrystallized from ether-petroleum ether to give 5-(3-morpholino-carbonylpropylthio)-1-methyl-1,2,3,4-tetrazole (2.2 g), white needles, m.p. 71—73°C.

Elementary analysis for $C_{10}H_{17}O_2S$:

Calcd. (%): C, 44.27; H, 6.37; N, 25.81
Found (%): C, 44.10; H, 6.30; N, 25.59

Example 131

1-Methyl-5-mercapto-1,2,3,4-tetrazole (1.6 g), sodium ethylate (1.4 g), sodium iodide (1.6 g) and N-cyclohexyl-3-chlorobutyramide (4.8 g) are added to ethanol (30 ml) and the mixture is refluxed with stirring for 6 hours. After completion of the reaction, the reaction mixture is poured into aqueous saturated sodium chloride solution. The precipitating crystals are separated out by filtration and washed with water. The crude crystals thus obtained are recrystallized from hexane-ethyl acetate to give N-cyclohexyl-4-(1-methyl-1,2,3,4-tetrazol-5-yl)thio-butyramide (2.1 g), colorless needles, m.p. 116.5—117.5°C.

Elementary analysis for $C_{12}H_{21}N_5OS$:

Calcd. (%): C, 50.86; H, 7.47; N, 24.71
Found (%): C, 50.87; H, 7.35; N, 24.63

Examples 132 to 186

According to the same procedure as described in Examples 128 to 131, the following compounds are obtained.

(132) N,N-Diethyl-4-(1-methyl-1,2,3,4-tetrazol-5-yl)thio-butyramide, colorless liquid, $n_D^{17.5}$ = 1.5227

(133) N-Ethyl-N-cyclohexyl-4-(1-methyl-1,2,3,4-tetrazol-5-yl)thio-butyramide, colorless liquid, $n_D^{17.5}$ = 1.5327

(134) N-Ethyl-N-phenyl-4-(1-methyl-1,2,3,4-tetrazol-5-yl)thio-butyramide, pale yellow liquid, $n_D^{26}$ = 1.5534

(135) 5-[3-(4-Acetylpiperazinocarbonyl)propylthio]-1-methyl-1,2,3,4-tetrazole, white crystalline powder (recrystallized from ligroine-acetone), m.p. 90—91.5°C

(136) N-[2-(3,4-Dimethoxyphenyl)ethyl]-4-(1-methyl-1,2,3,4-tetrazol-5-yl)thio-butyramide, colorless flakes (hexane-ethyl acetate), m.p. 70.5—71.5°C

(137) N-Hexyl-4-(1-methyl-1,2,3,4-tetrazol-5-yl)thio-butyramide, colorless (recrystallized from hexane-ether), m.p. 41—42°C

(138) N-Cyclooctyl-4-(1-methyl-1,2,3,4-tetrazol-5-yl)thio-butyramide, colorless liquid, $n_D^{14}$ = 1.5323

(139) N-Cyclododecanyl-4-(1-methyl-1,2,3,4-tetrazol-5-yl)thio-butyramide, colorless needles (hexane-ethyl acetate), m.p. 119—120°C

(140) N-Butyl-4-(1-methyl-1,2,3,4-tetrazol-5-yl)thio-butyramide, colorless liquid, $n_D^{16.5}$ = 1.5198

(141) N-(2-Hydroxyethyl-4-(1-methyl-1,2,3,4-tetrazol-5-yl)thio-butyramide, colorless liquid, $n_D^{14.5}$ = 1.5350

(142) N-Ethyl-N-benzyl-4-(1-methyl-1,2,3,4-tetrazol-5-yl)thio-butyramide, colorless liquid, $n_D^{19}$ = 1.5596

(143) N-Butyl-N-cyclohexyl-4-(1-methyl-1,2,3,4-tetrazol-5-yl)thio-butyramide, colorless liquid, $n_D^{19}$ = 1.5222

(144) N,N-Dibutyl-4-(1-methyl-1,2,3,4-tetrazol-5-yl)thio-butyramide, colorless liquid, $n_D^{19}$ = 1.5049

(145) N,N-Dibenzyl-4-(1-methyl-1,2,3,4-tetrazol-5-yl)thio-butyramide, colorless liquid, $n_D^{19}$ = 1.5773

(146) N,N-Diisopropyl-4-(1-methyl-1,2,3,4-tetrazol-5-yl)thio-butyramide, colorless liquid, $n_D^{19.5}$ = 1.5111

(147) N,N-Dicyclohexyl-4-(1-methyl-1,2,3,4-tetrazol-5-yl)thio-butyramide, colorless needles (hexane), m.p. 91—92°C

(148) N-Benzyl-N-tert-butyl-4-(1-methyl-1,2,3,4-tetrazol-5-yl)thio-butyramide, colorless needles (hexane), m.p. 86.5—87.5°C

(149) N - Cyclohexyl - N - [2 - (3,4 - dimethoxyphenyl)ethyl] - 4 - (1 - methyl - 1,2,3,4 - tetrazol - 5 - yl)thiobutyramide, colorless liquid, $n_D^{16}$ = 1.5470

(150) N-Methyl-N-(2-thienylmethyl)-4-(1-methyl-1,2,3,4-tetrazol-5-yl)thio-butyramide, colorless liquid, $n_D^{16}$ = 1.5706

(151) N-Benzyl-N-[2-(3,4-dimethoxyphenyl)ethyl]-4-(1-methyl-1,2,3,4-tetrazol-5-yl)thio-butyramide, colorless liquid, $n_D^{16}$ = 1.5659

(152) N,N-Dihexyl-4-(1-methyl-1,2,3,4-tetrazol-5-yl)thio-butyramide, colorless liquid, $n_D^{15}$ = 1.5011

(153) N-Ethyl-N-[2-(3,4-dimethoxyphenyl)ethyl]-4-(1-methyl-1,2,3,4-tetrazol-5-yl)thio-butyramide, colorless liquid, $n_D^{14}$ = 1.5451

(154) N-tert-Butyl-4-(1-methyl-1,2,3,4-tetrazol-5-yl)thio-butyramide, colorless flakes (hexane-ethyl acetate), m.p. 71—73°C

(155) N-Ethyl-4-(1-methyl-1,2,3,4-tetrazol-5-yl)thio-butyramide, colorless liquid, $n_D^{15}$ = 1.5319

(156) N-Benzyl-4-(1-methyl-1,2,3,4-tetrazol-5-yl)thio-butyramide, colorless needles (hexane-ethyl acetate), m.p. 65—66°C

(157) N-Hexyl-N-cyclohexyl-4-(1-methyl-1,2,3,4-tetrazol-5-yl)thio-butyramide, colorless liquid, $n_D^{16}$ = 1.5182

(158) N-Cyclohexyl-N-(2-hydroxyethyl)-4-(1-methyl-1,2,3,4-tetrazol-5-yl)thio-butyramide, colorless liquid, $n_D^{14.5}$ = 1.5372

(159) N-Phenyl-4-(1-methyl-1,2,3,4-tetrazol-5-yl)thio-butyramide, colorless needles (hexane-ethyl acetate), m.p. 106—107°C

(160) N-(2-Pyridyl)-4-(1-methyl-1,2,3,4-tetrazol-5-yl)thio-butyramide, colorless needles (hexane-ethyl acetate), m.p. 95—96°C

(161) N-(3-Pyridyl)-4-(1-methyl-1,2,3,4-tetrazol-5-yl)thio-butyramide, colorless plates (ethyl acetate), m.p. 110.5—113°C

(162) N-(2-Pyrimidyl)-4-(1-methyl-1,2,3,4-tetrazol-5-yl)thio-butyramide, colorless granules (hexane-ethyl acetate), m.p. 108—110°C

(163) N-Furfuryl-4-(1-methyl-1,2,3,4-tetrazol-5-yl)thio-butyramide, colorless flakes (hexane-ethyl acetate), m.p. 71—73°C

(164) N-(4-Aminosulfonylphenyl)-4-(1-methyl-1,2,3,4-tetrazol-5-yl)thio-butyramide, colorless needles (methanol), m.p. 169.5—170.5°C

(165) N-[4-(N,N-Dimethylamino)phenyl]-4-(1-methyl-1,2,3,4-tetrazol-5-yl)thio-butyramide, colorless prisms (hexane-ethyl acetate), m.p. 144—147°C

(166) N-(2-Methyl-3-chlorophenyl)-4-(1-methyl-1,2,3,4-tetrazol-5-yl)thio-butyramide, colorless needles (hexane-ethyl acetate), m.p. 104.5—105.5°C

(167) N-(4-Nitrophenyl)-4-(1-methyl-1,2,3,4-tetrazol-5-yl)thio-butyramide, colorless needles (ethyl acetate), m.p. 194—195°C

(168) N-(2-Methoxyphenyl)-4-(1-methyl-1,2,3,4-tetrazol-5-yl)thio-butyramide, colorless needles (hexane-ethyl acetate), m.p. 79.5—82°C

(169) N-Methyl-N-(2-tetrahydropyranyl)-4-(1-methyl-1,2,3,4-tetrazol-5-yl)thio-butyramide, colorless liquid, $n_D^{14}$ = 1.5273

(170) N-Ethyl-N-(2-pyridyl)-4-(1-methyl-1,2,3,4-tetrazol-5-yl)thio-butyramide, colorless liquid, $n_D^{16}$ = 1.5623

(171) N-Ethyl-N-(3-pyridyl)-4-(1-methyl-1,2,3,4-tetrazol-5-yl)thio-butyramide, colorless liquid, $n_D^{16}$ = 1.5618

(172) N-Ethyl-N-cyclopentyl-4-(1-methyl-1,2,3,4-tetrazol-5-yl)thio-butyramide, colorless liquid, $n_D^{9}$ = 1.5384

(173) N-Ethyl-N-cyclohexylmethyl-4-(1-methyl-1,2,3,4-tetrazol-5-yl)thio-butyramide, colorless liquid, $n_D^{11}$ = 1.5293

(174) N-Isopropyl-N-cyclohexyl-4-(1-methyl-1,2,3,4-tetrazol-5-yl)thio-butyramide, colorless liquid, $n_D^{15}$ = 1.5238

(175) N-Ethyl-N-(4-hydroxycyclohexyl)-4-(1-methyl-1,2,3,4-tetrazol-5-yl)thio-butyramide, colorless liquid, $n_D^{9}$ = 1.5363

(176) N-Ethyl-N-(2-hydroxycyclohexyl)-4-(1-methyl-1,2,3,4-tetrazol-5-yl)thio-butyramide, colorless needles (hexane-ethyl acetate), m.p. 132—133°C

(177) N-Ethyl-N-(2-acetyloxycyclohexyl)-4-(1-methyl-1,2,3,4-tetrazol-5-yl)thio-butyramide, colorless liquid, $n_D^{11}$ = 1.5218

(178) N,N-Dipropyl-4-(1-methyl-1,2,3,4-tetrazol-5-yl)thio-butyramide, colorless liquid, $n_D^{15}$ = 1.5151

(179) N-Butyl-N-phenyl-4-(1-methyl-1,2,3,4-tetrazol-5-yl)thio-butyramide, colorless liquid, $n_D^{15}$ = 1.5509

(180) N,N-Dimethyl-4-(1-methyl-1,2,3,4-tetrazol-5-yl)thio-butyramide, colorless liquid, $n_D^{16}$ = 1.5327

(181) N-Ethyl-N-cyclooctyl-4-(1-methyl-1,2,3,4-tetrazol-5-yl)thio-butyramide, colorless liquid, $n_D^{16}$ = 1.5309

(182) 5-[3-(4-Methylpiperazinocarbonyl)propylthio]-1-methyl-1,2,3,4-tetrazole, colorless granules (hexane-ethyl acetate), m.p. 65—68°C

(183) 5-(3-Piperidinocarbonylpropylthio)-1-methyl-1,2,3,4-tetrazole, colorless liquid, $n_D^{25}$ = 1.5310

(184) N-Ethyl-N-cyclohexyl-3-(1-methyl-1,2,3,4-tetrazol-5-yl)thio-propionamide, pale yellow liquid, $n_D^{26}$ = 1.5273

(185) N-Ethyl-N-cyclohexyl-5-(1-methyl-1,2,3,4-tetrazol-5-yl)thio-valeramide, pale yellow liquid, $n_D^{25}$ = 1.5227

(186) N-Ethyl-N-cyclohexyl-4-(1-phenyl-1,2,3,4-tetrazol-5-yl)thio-butyramide, colorless liquid, $n_D^{18}$ = 1.5590.

Example 187

1-Methyl-5-mercaptomethyl-1,2,3,4-tetrazole (0.1 mol) is dissolved in acetone (100 ml) and thereto are added N,N-diethyl-3-bromopropionamide (0.12 mol) and potassium carbonate (0.12 mol). The mixture is refluxed for 4 hours. After acetone is distilled off under reduced pressure, water is added to the residue and the aqueous mixture is extracted with chloroform. The chloroform layer is washed with aqueous saturated sodium chloride solution and dried over magnesium sulfate. After the solvent is distilled off, the resulting residue is subjected to column chromatography (Kiesselgel 60 produced by Merck) by eluting the column with chloroform-methanol (50:1, V/V) to give N,N-diethyl-3-(1-methyl-1,2,3,4-tetrazol-5-yl)methylthio-propionamide (yield 48%), colorless liquid, $n_D^{25}$ = 1.5200

Elementary analysis for $C_{10}H_{19}N_5OS$:

Calcd. (%): C, 46.67; H, 7.44; N, 27.21
Found (%): C, 46.72; H, 7.53; N, 27.29

Examples 188 and 189

According to the same procedure as described in Example 187, the following compounds are obtained.

(188) N,N-Diethyl-3-[1-(4-ethylphenyl)-1,2,3,4-tetrazol-5-yl]methylthio-propionamide, colorless liquid, $n_D^{25}$ = 1.5499

(189) N-Ethyl-N-cyclohexyl-3-(1-ethyl-1,2,3,4-tetrazol-5-yl)methylthio-propionamide, pale yellow liquid, $n_D^{18}$ = 1.5277

Example 190

1-Methyl-5-mercapto-1,2,3,4-tetrazole (11.6 g) is dissolved in acetone (100 ml) and thereto are added methyl 4-bromobutyrate (21.7 g) and potassium carbonate (15 g). The mixture is refluxed for 4 hours. After acetone is distilled off under reduced pressure, water is added to the resulting residue and the aqueous mixture is extracted with chloroform. The chloroform solution is washed with aqueous saturated sodium chloride solution and dried over magnesium sulfate. After chloroform is distilled off, the residue is subjected to column chromatography (Wakogel C-200) by eluting the column with benzene-ether (5:1, V/V). The eluate is distilled under reduced pressure to give methyl 4-(1-methyl-1,2,3,4-tetrazol-5-yl)thio-butyrate (20 g), colorless liquid, b.p. 175—177°C/1,06 mbar, $n_D^{26}$ = 1.5083.

Elementary analysis for $C_7H_{12}N_4OS$:

Calcd. (%): C, 38.88; H, 5.59; N, 25.91
Found (%): C, 38.98; H, 5.67; N, 25.83

Examples 191 and 192

Substitution of 1-phenyl- or 1-cyclohexyl-5-mercapto-1,2,3,4-tetrazole for 1-methyl-5-mercapto-1,2,3,4-tetrazole in the procedure of Example 190 produces the following compounds.

(191) Methyl 4-(1-phenyl-1,2,3,4-tetrazol-5-yl)thio-butyrate, colorless liquid, $n_D^{18}$ = 1.5654

(192) Methyl 4-(1-cyclohexyl-1,2,3,4-tetrazol-5-yl)thio-butyrate, pale yellow liquid, $n_D^{18}$ = 1.5162

Example 193

1-Methyl-5-mercapto-1,2,3,4-tetrazole (0.1 mol) is dissolved in acetone (100 ml) and thereto is added dropwise a solution of 4-bromobutyric acid (0.12 mol) and sodium hydroxide (0.25 mol) in water (50 ml). The mixture is warmed with stirring at 50—60°C for 4 hours and acetone is distilled off under reduced pressure. The resulting residue is acidified with diluted hydrochloric acid, saturamed with sodium chloride and extracted with chloroform. The chloroform solution is washed with aqueous saturated sodium chloride solution and dried over magnesium sulfate. The solvent is distilled off and the residue is subjected to column chromatography (Kieselgel 60 produced by Merck) by eluting the column with chloroform-methanol (10:1, V/V) to give 4-(1-methyl-1,2,3,4-tetrazol-5-yl)thio-butyric acid (yield 23%) colorless liquid.

NMR: $\delta_{ppm}^{CDCl_3}$ 1.80—2.80 (6H, m), 3.42 (2H, t, J=7Hz), 3.96 (3H, s), 11.18 (1H, s)

Elementary analysis for $C_6H_{10}N_4O_2S$:

Calcd. (%): C, 35.63; H, 4.98; N, 27.70
Found (%): C, 35.79; H, 5.12; N, 27.84

Examples 194 and 195

Substitution of 1-phenyl- or 1-cyclohexyl-5-mercapto-1,2,3,4-tetrazole for 1-methyl-5-mercapto-1,2,3,4-tetrazole in the procedure of Example 193 produces the following compounds.

(194) 4-(1-Phenyl-1,2,3,4-tetrazol-5-yl)thio-butyric acid, pale yellow liquid, NMR: $\delta_{ppm}^{CDCl_3}$ 2.00—2.70 (4H, m), 3.44 (2H, t, J=7Hz), 7.55 (5H, s), 10.98 (1H, br.s)

(195) 4-(1-Cyclohexyl-1,2,3,4-tetrazol-5-yl)thio-butyric acid, colorless prisms (ether), m.p. 67.5—69.5°C.

Example 196

2-Mercaptopropionic acid (1.6 g) is dissolved in 1N aqueous sodium hydroxide solution (45 ml) and thereto is added dropwise a solution of 1-methyl-5-chloromethyl-1,2,3,4-tetrazole (15.2 g) in acetone (20 ml) with stirring under ice-cooling. Stirring is continued for 3 hours under ice-cooling. Acetone is distilled off and the residue is acidified with concentrated hydrochloric acid, saturated with sodium chloride and then, extracted with chloroform. The chloroform solution is dried over magnesium sulfate. After chloroform is distilled off, the residue is subjected to column chromatography (Kieselgel 60, made by Merck) by eluting the column with chloroform-methanol (10:1, V/V) to give 3-(1-methyl-1,2,3,4-tetrazol-5-yl)methylthio-propionic acid (2.1 g) as a colorless liquid.

NMR: $\delta^{CDCl_3}_{ppm}$ 2.40—3.00 (4H, m), 4.05 (2H, s), 4.12 (3H, s), 9.79 (1H, s).

Elementary analysis for $C_6H_{10}N_4O_2S$:

Calcd. (%): C, 35.63; H, 4.98; N, 27.70
Found (%): C, 35.81; H, 4.83; N, 27.83

Examples 197 and 198

According to the same procedure as described in Example 196, the following compounds are obtained by using appropriate starting materials.

(197) 3-(1-Ethyl-1,2,3,4-tetrazol-5-yl)methylthio-propionic acid, colorless prisms (acetone-ether), m.p. 68—70°C

(198) 3-[1-(4-Ethylphenyl)-1,2,3,4-tetrazol-5-yl]methylthio-propionic acid, colorless liquid, NMR: $\delta^{CDCl_3}_{ppm}$ 1.28 (3H, t, J=7Hz), 2.40—3.10 (6H, m), 3.97 (2H, s), 7.43 (4H, br.s), 10.02 (1H, br.s).

Example 199

Methyl N-phenyladipinamate (9 g) is dissolved in benzene (70 ml) and thereto is added phosphorus pentachloride (9 g) with stirring at below 15°C. The mixture is stirred at room temperature for 1.5 hours and concentrated to 1/3 by volume under reduced pressure. A solution of hydrogen azide in benzene (0.0172 mol/10 ml, 44.2 ml) is added dropwise to the resulting iminochloride solution with stirring under ice-cooling. The mixture is stirred at room temperature for 1 hour, allowed to stand overnight and thereafter gently refluxed for 2 hours. The reaction mixture is concentrated, and thereto is added ice-water (50 ml) and the mixture is extracted with chloroform. The chloroform solution is washed with water, aqueous dilute sodium hydroxide solution and water and then dried over magnesium sulfate. Chloroform is distilled off and the residue is subjected to column chromatography (Wakogel C-200) by eluting the column with benzene-ether (5:1, V/V) to give methyl 5-(1-phenyl-1,2,3,4-tetrazol-5-yl)valerate (7.3 g) as a colorless liquid.

NMR: $\delta^{CDCl_3}_{ppm}$ 1.50—2.10 (4H, m), 2.30 (2H, t, J=6Hz), 2.90 (2H, t, J=6Hz), 3.62 (3H, s), 7.30—7.70 (5H, m).

Elementary analysis for $C_{13}H_{16}N_4O_2$:

Calcd. (%): C, 59.98; H, 6.20; N, 21.53
Found (%): C, 60.19; H, 6.32; N, 21.71

Examples 200 to 202

According to the same procedure as described in Example 199, the following compounds are obtained by using appropriate starting materials.

(200) Methyl 5-(1-methyl-1,2,3,4-tetrazol-5-yl)valerate, colorless liquid, NMR: $\delta^{CDCl_3}_{ppm}$ 1.50—2.20 (4H, m), 2.36 (2H, t, J=6Hz), 2.90 (2H, t, J=6Hz), 3.65 (3H, s), 4.05 (3H, s).

(201) Methyl 5-(1-cyclohexyl-1,2,3,4-tetrazol-5-yl)valerate, colorless liquid, NMR: $\delta^{CDCl_3}_{ppm}$ 1.00—2.20 (14H, m), 2.38 (2H, t, J=6Hz), 2.90 (2H, t, J=6Hz), 3.70 (3H, s), 3.90—4.50 (1H, m)

(202) Methyl 5-(1,2,3,4-tetrazol-5-yl)valerate, colorless liquid, NMR: $\delta^{CDCl_3}_{ppm}$ 1.50—2.20 (4H, m), 2.34 (2H, t, J=6Hz), 3.02 (2H, t, J=6Hz), 3.60 (3H, s).

Example 203

20% aqueous Hydrochloric acid (100 ml) is added to methyl 5-(1-phenyl-1,2,3,4-tetrazol-5-yl)valerate (9.3 g) and the mixture is refluxed for 4 hours. After cooling, water is added and the mixture is extracted with chloroform. The chloroform solution is extracted with aqueous saturated sodium bicarbonate solution. The aqueous layer is acidified with concentrated hydrochloric acid and again extracted with chloroform. The latter chloroform extract is washed with aqueous saturated sodium chloride solution and dried over magnesium sulfate. Chloroform is distilled off and to give 5-(1-phenyl-1,2,3,4-tetrazol-5-yl)valeric acid (8.2 g), pale yellow liquid.

NMR: $\delta^{CDCl_3}_{ppm}$ 1.40—2.10 (4H, m), 2.33 (2H, t, J=6Hz), 2.92 (2H, t, J=6Hz), 7.30—7.70 (5H, m), 10.56 (1H, s).

Elementary analysis for $C_{12}H_{14}N_4O_2$:

Calcd. (%): C, 58.52; H, 5.73; N, 22.75
Found (%): C, 58.61; H, 5.85; N, 22.88

Examples 204 to 206

According to the same procedure as described in Example 203, the following compounds are obtained by using the compounds of Examples 200 to 202 as the starting material.

(204) 5-(1-Methyl-1,2,3,4-tetrazol-5-yl)valeric acid, colorless prisms (ether-ethanol), m.p. 107—109°C, NMR: $\delta_{ppm}^{CDCl_3}$ 1.40—2.20 (4H, m), 2.28 (2H, t, J=2.87 (2H, t, J=6Hz), 4.02 (3H, s), 9.30 (1H, br.s)

(205) 5-(1-Cyclohexyl-1,2,3,4-tetrazol-5-yl)valeric acid, colorless prisms (recrystallized from ethanol-water), m.p. 100—102°C

(206) 5-(1,2,3,4-Tetrazol-5-yl)valeric acid, colorless liquid, NMR: $\delta_{ppm}^{d_6-DMSO}$ 1.30—2.00 (4H, m), 2.30 (2H, t, J=6Hz), 2.87 (2H, t, J=6Hz).

Example 207

20% aqueous Hydrochloric acid (150 ml) is added to methyl 4-(1-methyl-1,2,3,4-tetrazol-5-yl)thio-valerate (17 g) and the mixture is refluxed for 2 hours. After cooling, the reaction mixture is diluted with water and extracted with chloroform. The chloroform solution is washed with aqueous saturated sodium chloride solution and dried over magnesium sulfate. Chloroform is distilled off to give 4-(1-methyl-1,2,3,4-tetrazol-5-yl)thio-valeric acid, colorless liquid, $n_D^{26}$ = 1.5133.

NMR: $\delta_{ppm}^{CDCl_3}$ 1.80—2.80 (6H, m), 3.42 (2H, t, J=7Hz), 3.96 (3H, s), 11.18 (1H, s).

Elementary analysis for $C_6H_{10}N_4O_2S$:

Calcd. (%): C, 35.63; H, 4.98; N, 27.70
Found (%): C, 35.76; H, 5.11; N, 27.81

Examples 208 and 209

Substitution of methyl 4-(1-phenyl- or cyclohexyl-1,2,3,4-tetrazol-5-yl)thio-valerate for methyl 4-(1-methyl-1,2,3,4-tetrazol-5-yl)thio-valerate in the procedure of Example 207 produces the following compounds.

(208) 4-(1-Phenyl-1,2,3,4-tetrazol-5-yl)thiovaleric acid, pale yellow liquid, NMR: $\delta_{ppm}^{CDCl_3}$ 2.00—2.70 (4H, m), 3.44 (2H, t, J=7Hz), 7.55 (5H, s), 10.98 (1H, br.s)

(209) 4-(1-Cyclohexyl-1,2,3,4-tetrazol-5-yl)-thio-valeric acid, colorless prisms (ether), m.p. 67.5—69.5°C.

Example 210

Thionyl chloride (400 mg) is added to methanol (10 ml) with stirring while externally cooling with ice and stirring is continued for 10 minutes. 4-(1-Phenyl-1,2,3,4-tetrazol-5-yl)thio-butyric acid (500 mg) is added to the solution and the mixture is stirred at room temperature for 2 hours. The reaction mixture is poured into aqueous saturated sodium chloride solution (100 ml) and extracted with chloroform. The chloroform layer is washed with aqueous saturated sodium bicarbonate solution and water and dried over sodium sulfate. Chloroform is distilled off and the residue is subjected to column chromatography (Wakogel C-200) by eluting the column with benzene-ether (5:1, V/V) to give methyl 4-(1-phenyl-1,2,3,4-tetrazol-5-yl)thio-butyrate (350 mg), colorless liquid, $n_D^{18}$ = 1.5654.

Elementary analysis for $C_{12}H_{14}N_4O_2S$:

Calcd. (%): C, 38.88; H, 5.59; N, 25.91
Found (%): C, 38.95; H, 5.61; N, 25.81

Example 211

Thionyl chloride (10 ml) is added to 4-(1-methyl-1,2,3,4-tetrazol-5-yl)thio-butyric acid (2 g) and the mixture is refluxed for 1 hour. After excess thionyl chloride is distilled off under reduced pressure, dry benzene is added to the residue and the remaining small amount of thionyl chloride is removed as the benzene azeotrope. The residue thus obtained is dissolved in dry benzene (50 ml) and thereto is added pyridine (2 ml). Methanol (2 ml) is added dropwise with stirring while externally cooling with ice. After the mixture is stirred at room temperature for 1 hour, the benzene layer is washed with dilute hydrochloric acid, aqueous saturated sodium bicarbonate solution and aqueous saturated sodium chloride solution and dried over sodium sulfate. Benzene is distilled off and the residue is subjected to column chromatography (Wakogel C-200) by eluting the column with benzene-ether (5:1, V/V). The eluate is distilled under reduced pressure to give methyl 4-(1-methyl-1,2,3,4-tetrazol-5-yl)thio-butyrate (1.2 g), colorless liquid, b.p. 175—177°C/1,06 mbar.

Elementary analysis for $C_7H_{12}N_4O_2S$:

Calcd. (%): C, 38.88; H, 5.59; N, 25.91
Found (%): C, 38.92; H, 5.53; N, 25.83

Example 212

In the same manner as described in Example 63, there is obtained N,N-diethyl-4-(1-cyclohexyl-1,2,3,4-tetrazol-5-yl)thio-butyramide, colorless liquid, $n_D^{20}$ = 1.5237.

Example 213

In the same manner as described in Example 190, there is obtained methyl 4-[1-(4-ethylphenyl)-1,2,3,4-tetrazol-5-yl)thio-butyrate, pale yellow liquid, $n_D^{14}$ = 1.5581.

Example 214

In the same manner as described in Example 193, there is obtained 4-[1-(4-ethylphenyl)-1,2,3,4-tetrazol-5-yl)thio-butyric acid, pale yellow liquid, NMR: $\delta^{CDCl_3}_{ppm}$ 1.28 (3H, t, J=7.5Hz), 2.00—2.90 (6H, m), 3.44 (2H, t, J=7Hz), 7.36 (2H, d, J=9Hz), 7.48 (2H, d, J=9Hz), 10.60 (1H, br.s).

Preparation 1

| Ingredients | Amounts |
|---|---|
| N-Ethyl-N-(2-hydroxycyclohexyl)-4-(1-methyl-1,2,3,4-tetrazol-5-yl)thio-butyramide | 150 g |
| Avicel® (trade mark of microcrystalline cellulose produced by Asahi-kasei Co., Ltd.) | 40 g |
| Corn starch | 30 g |
| Magnesium stearate | 2 g |
| Hydroxypropylmethyl cellulose | 10 g |
| Polyethylene glycol-6000 | 3 g |
| Castor oil | 40 g |
| Methanol | 40 g |

The compound of the present invention, Avicel®, corn starch and magnesium stearate are mixed, ground and then, compressed into tablets with a punch having a diameter of 10 mm. The tablets thus obtained are coated with the film coating solution consisting of hydroxy-propylmethyl cellulose, polyethylene glycol-6000, castor oil and methanol to obtain film coated tablets.

Preparation 2

| Ingredients | Amounts |
|---|---|
| N-(2-Pyridyl-4-(1-methyl-1,2,3,4-tetrazol-5-yl) thio-butyramide | 150 g |
| Citric acid | 1 g |
| Lactose | 33.5 g |
| Dipotassium hydrogen phosphate | 70 g |
| Pluronic® F-68 | 30 g |
| Sodium lauryl sulfate | 15 g |
| Polyvinyl pyrrolidone | 15 g |
| Polyethylene glycol (Carbowax® 1500) | 4.5 g |
| Polyethylene glycol (Carbowax® 6000) | 45 g |
| Corn starch | 30 g |
| Dry sodium lauryl sulfate | 3 g |
| Dry magnesium stearate | 3 g |
| Ethanol | appropriate amount |

The compound of the present invention, citric acid, lactose, dipotassium hydrogen phosphate, Pluronic® F-68 and sodium lauryl sulfate are mixed and passed through No. 60 screen. The resulting

mixture is granulated with an alcoholic solution containing polyvinyl pyrrolidone, Carbowax® 1500 and Carbowax® 6000. The powdery mixture is made into paste by adding alcohol, if necessary. Corn starch is added to the wet mass and mixing is continued until uniform granules are formed. The wet granules are passed through No. 10 screen, placed in a tray and dried in an oven at 100°C for 12—14 hours. The dried granules are passed through No. 16 screen, mixed with dry sodium lauryl sulfate and dry magnesium stearate and then, compressed into a desired shape.

The core tablets thus obtained are treated with varnish and thereto is spreaded talc in order to prevent absorption of moisture. Subcoating is applied about the resulting tablets. The tablets are again treated enough times with varnish for oral administration. Further, subcoating and smooth layers are applied about the tablets so as to make them round and smooth. Coloring layer is applied so as to be the tablets in desired color. After drying, polishing produces the tablets having homogeneous gloss.

Preparation 3

| Ingredients | Amounts |
|---|---|
| N-Ethyl-N-cyclohexyl-4-(1-methyl-1,2,3,4-tetrazol-5-yl)thio-butyramide | 5 g |
| Polyethylene glycol (molecular weight 4000) | 0.3 g |
| Sodium chloride | 0.9 g |
| Polyoxyethylene sorbitan monooleate | 0.4 g |
| Sodium metabisulfite | 0.1 g |
| Methyl-paraben | 0.18 g |
| Propyl-paraben | 0.02 g |
| Distilled water for injection | 100 ml |

The above parabens, sodium metabisulfite and sodium chloride are dissolved in an about half amount of the distilled water with stirring at 80°C. The resulting solution is cooled to 40°C and the above compound of the present invention, polyethylene glycol and polyoxyethylene sorbitan monooleate are dissolved in the solution. The remaining distilled water is added to the solution to adjust the volume. The solution is sterilized by filtration with a suitable filter paper to obtain a preparation for injection.

## Claims

1. A tetrazole derivative of the formula:

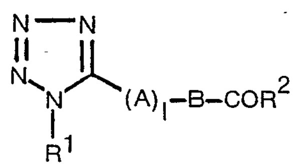

wherein $R^1$ is hydrogen, an alkyl having 1 to 6 carbon atoms, a cycloalkyl having 3 to 12 carbon atoms or phenyl; A is sulfur or an alkylene-thio wherein the alkylene moiety has 1 to 6 carbon atoms and the thio group bonds to the group B; l is 0 or 1; B is an alkylene having 1 to 6 carbon atoms; $R^2$ is hydroxy, an alkoxy having 1 to 6 carbon atoms, or a group:

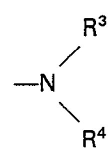

wherein $R^3$ and $R^4$ are the same or different and are each hydrogen, an alkyl having 1 to 6 carbon atoms, a cycloalkyl having 3 to 12 carbon atoms, phenyl, a cycloalkylalkyl wherein the cycloalkyl moiety has 3 to 8 carbon atoms and the alkyl moiety has 1 to 6 carbon atoms, a phenyl-alkyl wherein the alkyl moiety has 1 to 6 carbon atoms, a hydroxyalkyl having 1 to 6 carbon atoms, a saturated or unsaturated heterocyclic group selected from the group consisting of pyridyl, pyrrolyl, pyrrolidinyl, 3-pyrrolinyl, dihydropyridyl,

piperidinyl, 2-pyrazolinyl, 2-imidazolinyl, imidazolyl, imidazolidinyl, pyrazolyl, pyrimidinyl, pyridazinyl, pyrazinyl, piperazinyl, oxazolyl, oxazolidinyl, isoxazolyl, 4H-1,4-oxazinyl, morpholinyl, thiazolyl, thiazolidinyl, isothiazolyl, 4H-1,4-thiazinyl, furyl, tetrahydrofuryl, 2H-pyranyl, 4H-pyranyl, 3,4-dihydro-2H-pyranyl, tetrahydropyranyl, thienyl, tetrahydrothienyl, thianyl and 1,4-dithianyl, or an alkyl having 1 to 6 carbon atoms which is substituted by said saturated or unsaturated heterocyclic group, or the $R^3$ and $R^4$ may combine together with the nitrogen atom to which they are joined with or without being intervened with oxygen or nitrogen to form a saturated or unsaturated heterocyclic group selected from the group consisting of piperidino, pyrrolidino, piperazino, 1-imidazolyl, 1-pyrazolyl, 4,5-dihydropyrazol-1-yl, 1-pyrrolyl, 4H-1,4-oxazin-4-yl, and 3-tetrahydrooxazolyl which heterocyclic group may be substituted with an alkyl having 1 to 6 carbon atoms or an alkanoyl having 1 to 6 carbon atoms; said cycloakyl, phenyl and phenyl-alkyl in the substituents $R^1$, $R^3$ and $R^4$ may have on the cycloalkyl or phenyl ring one or two substituents selected from the group consisting of an alkoxy having 1 to 6 carbon atoms, an alkyl having 1 to 6 carbon atoms, a halogen, an N,N-dialkylamino wherein each alkyl moiety has 1 to 6 carbon atoms, nitro, aminosulfonyl, hydroxy and an alkanoyloxy having 1 to 6 carbon atoms; provided that

a) when l is 0 and $R^1$ is hydrogen or an alkyl having 1 to 6 carbon atoms, $R^2$ is other than the groups selected from hydroxy, an alkoxy having 1 to 6 carbon atoms and amino;

b) when l is 0 and $R^1$ is n-butyl, B is not methylene and $R^2$ is not ethylamino;

c) when l is 0 and $R^1$ is phenyl, B is methylene, $R^2$ is other than the groups selected from hydroxy, ethoxy, amino, methylamino and benzylamino;

d) when l is 0 and $R^1$ is phenyl and B is ethylmethylene, $R^2$ is not hydroxy, ethoxy or amino;

e) when l is 0 and $R^1$ is cyclohexyl and B is ethylene or methylmethylene, $R^2$ is not amino;

f) when l is 0 and $R^1$ is cyclohexyl and B is dimethylmethylene, $R^2$ is other than the groups selected from amino, diethylamino, benzylamino and morpholino; and

g) when l is 1 and $R^1$ is hydrogen, an alkyl group having 1 to 6 carbon atoms, a cycloalkyl group having 3 to 12 carbon atoms, or phenyl and A is S and B is alkylene having 1 to 6 carbon atoms and $R^2$ is a

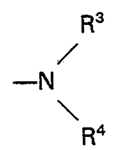

group, $R^3$ and $R^4$ are not combined together with the nitrogen atom to which they are joined to form a 1-imidazolyl- or 1-pyrrolyl-group, and a pharmaceutically acceptable salt thereof.

2. A compound according to claim 1, wherein $R^2$ is hydroxy or alkoxy having 1 to 6 carbon atoms.

3. A compound according to claim 1, wherein $R^2$ is a group $-NR^3R^4$, wherein $R^3$ and $R^4$ are as defined above.

4. A compound according to claim 3, wherein l is 0.

5. A compound according to claims 3 and 4, wherein $R^3$ and $R^4$ are the same or different and are each an alkyl group having 1 to 6 carbon atoms or a cycloalkyl group having 3 to 12 carbon atoms which may have one or two substituents selected from the group consisting of an alkoxy having 1 to 6 carbon atoms, an alkyl having 1 to 6 carbon atoms, a halogen, an N,N-dialkylamino wherein each alkyl moiety has 1 to 6 carbon atoms, nitro, aminosulfonyl, hydroxy and an alkanoyloxy having 1 to 6 carbon atoms.

6. A compound according to claims 3 to 5, which compound is selected from N-ethyl-N-cyclohexyl-5-(1-phenyl-1,2,3,4-tetrazole-5-yl)valeramide and N-ethyl-N-cyclohexyl-5-(1-cyclohexyl-1,2,3,4-tetrazole-5-yl)-valeramide.

7. A compound according to claim 3, wherein l is 1.

8. A compound according to claim 7, wherein A is an alkylenethio-group having 1 to 6 carbon atoms in the alkylene moiety, the thio-group binding to the group B.

9. A compound according to claims 7 and 8, wherein $R^3$ and $R^4$ are the same or different and are each an alkyl group having 1 to 6 carbon atoms or a cycloalkyl group having 3 to 12 carbon atoms which may have one or two substituents selected from the group consisting of an alkoxy having 1 to 6 carbon atoms, an alkyl having 1 to 6 carbon atoms, a halogen, an N,N-dialkylamino wherein each alkyl moiety has 1 to 6 carbon atoms, nitro, aminosulfonyl, hydroxy and an alkanoyloxy having 1 to 6 carbon atoms.

10. A compound according to claim 7, wherein A is sulfur.

11. A compound according to claim 7, wherein A is a thio group and $R^2$ is a disubstituted amino group, which compound is selected from N,N-dimethyl-4-(1-methyl-1,2,3,4-tetrazol-5-yl)-thio-butyramide, N,N-diethyl-4-(1-methyl-1,2,3,4-tetrazol-5-yl)-thio-butyramide, N-ethyl-N-(2-hydroxycyclohexyl)-4-(1-methyl-1,2,3,4-tetrazol-5-yl)thio-butyramide, N-ethyl-N-(4-hydroxycyclohexyl)-4-(1-methyl-1,2,3,4-tetrazol-5-yl)-thiobutyramide, N-ethyl-N-cyclohexyl-3-(1-methyl-1,2,3,4-tetrazol-5-yl)thio-propionamide, N-ethyl-N-cyclohexyl-5-(1-methyl-1,2,3,4-tetrazol-5-yl)thiovaleramide, N,N-diethyl-4-(1-phenyl-1,2,3,4-tetrazol-5-yl)-thio-butyramide, N-ethyl-N-cyclohexyl-4-(1-phenyl-1,2,3,4-tetrazol-5-yl)thio-butyramide and N-ethyl-N-cyclohexyl-4-(1-cyclohexyl-1,2,3,4-tetrazol-5-yl)-thio-butyramide.

12. A compound according to claim 2, which compound is methyl-4-(1-methyl-1,2,3,4-tetrazole-5-yl)-thio-butyrate.

13. A compound according to claim 3, which compound is N-[2-(3,4-dimethoxyphenyl)ethyl]-4-(1-methyl-1,2,3,4-tetrazol-5-yl)thio-butyramide.

14. An anti-ulcer composition, which comprises as an essential active ingredient a therapeutically effective amount of a tetrazole derivative of claims 1 to 13 with a conventional pharmaceutically acceptable carrier or diluent.

15. A process for the preparation of a tetrazole derivative of the formula:

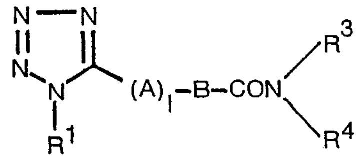

[Ia]

wherein $R^1$, A, l, B, $R^3$ and $R^4$ are as defined in claim 1 or a pharmaceutically acceptable salt, which comprises reacting a carboxylic acid compound of the formula:

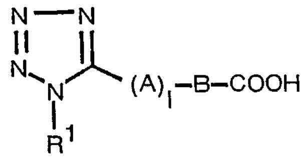

[II]

wherein $R^1$, A, l and B are as defined above, or its reactive derivative with an amine of the formula:

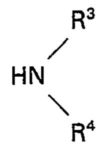

[III]

wherein $R^3$ and $R^4$ are as defined above, or its reactive derivative.

16. A process for the preparation of a tetrazole derivative of the formula:

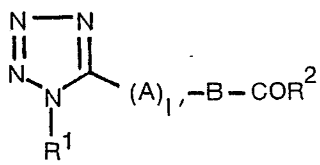

[Ib]

wherein $R^1$, A and B are as defined in claim 1, l′ is 1 and $R^2$ is hydroxy, an alkoxy having 1 to 6 carbon atoms, or a group

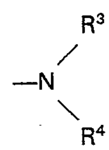

wherein $R^3$ and $R^4$ are as defined in claim 1 or a pharmaceutically acceptable salt thereof, which comprises reacting a 5-mercaptotetrazole derivative of the formula:

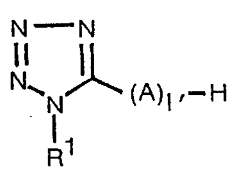

[IV]

wherein $R^1$, A and l′ are as defined above, with a halo-alkanecarboxylic acid derivative of the formula:

$$X\text{—}B\text{—}COR^2 \quad [V]$$

wherein X is a halogen, and B and $R^2$ are as defined above.

17. A process for the preparation of a tetrazol derivative of the formula:

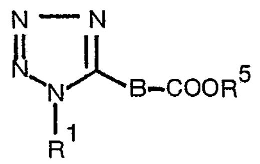

[Ic]

wherein $R^1$ is hydrogen, an alkyl having 1 to 6 carbon atoms, a cycloalkyl having 3 to 12 carbon atoms or phenyl; B is an alkylene having 1 to 6 carbon atoms, and $R^5$ is an alkyl having 1 to 6 carbon atoms, said cycloalkyl and phenyl may have on the ring one or two substituents selected from the group consisting of an alkoxy having 1 to 6 carbon atoms, an alkyl having 1 to 6 carbon atoms, halogen, an N,N-dialkylamino wherein each alkyl moiety has 1 to 6 carbon atoms, nitro, aminosulfonyl, hydroxy and an alkanoyloxy having 1 to 6 carbon atoms, or a pharmaceutically acceptable salt thereof, which comprises reacting a carboxylic acid amide of the formula:

$$R^5COO—B—CONHR^1 \qquad [VIII]$$

wherein $R^5$, B and $R^1$ are as defined above, with phosphorus pentachloride and subsequently with hydrogen azide.

18. A process for the preparation of a tetrazole derivative of the formula:

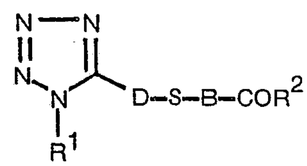

[Id]

wherein $R^1$ and B are as defined in claim 1, D is alkylene having 1 to 6 carbon atoms and $R^2$ is as defined in claim 16 or a pharmaceutically acceptable salt thereof, which comprises reacting a tetrazole compound of the formula:

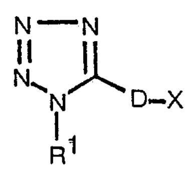

[XI]

wherein $R^1$ and D are as defined above and X is a halogen, with a mercapto compound of the formula:

$$HS—B—COR^2 \qquad [XII]$$

wherein B and $R^2$ are as defined above.

19. A process for the preparation of a tetrazole derivative of the formula:

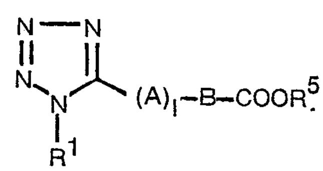

[Ie]

wherein $R^1$, A, l, and B are as defined in claim 1, $R^5$ is an alkyl having 1 to 6 carbon atoms, which comprises subjecting a tetrazole derivative of the formula:

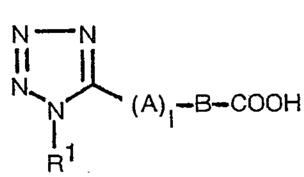

[If]

wherein $R^1$, A, l and B are as defined above, to esterification.

20. A process for the preparation of a tetrazole derivative of the formula:

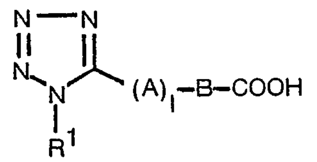

[If]

wherein $R^1$, A, l and B are as defined in claim 1, which comprises subjecting a tetrazole derivative of the formula:

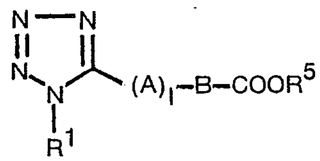

[Ie]

wherein $R^1$, A, l and B as as defined above, and $R^5$ is an alkyl having 1 to 6 carbon atoms, to hydrolysis.

**Patentansprüche**

1. Tetrazol-Derivat der Formel

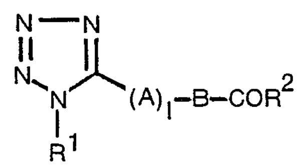

in der

$R^1$ Wasserstoff, Alkyl mit 1 bis 6 Kohlenstoff-Atomen, Cycloalkyl mit 3 bis 12 Kohlenstoff-Atomen oder Phenyl ist,

A Schwefel oder Alkylenthio ist, worin die Alkylen-Struktureinheit 1 bis 6 Kohlenstoff-Atome besitzt und die Thio-Gruppe die Gruppe B bindet,

l 0 oder 1 ist,

B ein Alkylen-Ring mit 1 bis 6 Kohlenstoff-Atomen ist,

$R^2$ Hydroxy, Alkoxy mit 1 bis 6 Kohlenstoff-Atomen oder eine Gruppe

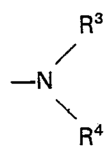

ist, in der

$R^3$ und $R^4$ gleich oder verschieden sind und jeweils Wasserstoff, Alkyl mit 1 bis 6 Kohlenstoff-Atomen, Cycloalkyl mit 3 bis 12 Kohlenstoff-Atomen, Phenyl, Cycloalkylalkyl, worin die Cycloalkyl-Struktureinheit 3 bis 8 Kohlenstoff-Atome und die Alkyl-Struktureinheit 1 bis 6 Kohlenstoff-Atome besitzt, Phenylalkyl, worin die Alkyl-Struktureinheit 1 bis 6 Kohlenstoff-Atome besitzt, Hydroxyalkyl mit 1 bis 6 Kohlenstoff-Atomen, eine gesättigte oder ungesättigte heterocyclische Gruppe ausgewählt aus der aus Pyridyl, Pyrrolyl, Pyrrolidinyl, 3-Pyrrolinyl, Dihydropyridyl, Piperidinyl, 2-Pyrazolinyl, 2-Imidazolinyl, Imidazolyl, Imidazolidinyl, Pyrazolyl, Pyrimidinyl, Pyridazinyl, Pyrazinyl, Piperazinyl, Oxazolyl, Oxazoldinyl, Isoxazolyl, 4H-1,4-Oxazinyl, Morpholinyl, Thiazolyl, Thiazolidinyl, Isothiazolyl, 4H-1,4-Thiazinyl, Furyl, Tetrahydrofuryl, 2H-Pyranyl, 4H-Pyranyl, 3,4-Dihydro-2H-pyranyl, Tetrahydropyranyl, Thienyl, Tetrahydrothienyl, Thianyl und 1,4-Di-thianyl bestehenden Gruppe, oder Alkyl mit 1 bis 6 Kohlenstoff-Atomen, das durch die genannte gesättigte oder ungesättigte Gruppe substituiert ist, sind, oder $R^3$ und $R^4$ können zusammen mit dem Stickstoff-Atom, an das sie gebunden sind, mit oder dazwischenliegendem Sauerstoff oder Stickstoff zu einer gesättigten oder ungesättigten heterocyclischen Gruppe kombinieren, die aus der aus Piperidino, Pyrrolidino, Piperazino, 1-Imidazolyl, 1-Pyrazolyl, 4,5-Dihydropyrazol-1-yl, 1-Pyrrolyl, 4H-1,4-Oxazin-4-yl. und 3-Tetrahydrooxazolyl bestehenden Gruppe ausgewählt ist, wobei diese heterocyclische Gruppe durch ein Alkyl mit 1 bis 6 Kohlenstoff-Atomen oder ein Alkanoyl mit 1 bis 6 Kohlenstoff-Atomen substituiert sein kann, und wobei die Cycloalkyl-, Phenyl oder Phenylalkyl-Gruppen in den Substituenten $R^1$, $R^3$ und $R^4$ an dem Cycloalkyl- oder Phenyl-Ring einen oder zwei Substituenten ausgewählt aus der aus Alkoxy mit 1 bis 6 Kohlenstoff-Atomen, Alkyl mit 1 bis 6 Kohlenstoff-Atomen, Halogen, N,N-Dialkylamino, worin jede Alkyl-

Strukturereinheit 1 bis 6 Kohlenstoff-Atome besitzt, Nitro, Aminosulfonyl, Hydroxy und Alkanoyloxy mit 1 bis 6 Kohlenstoff-Atomen bestehenden Gruppe tragen können,
mit der Maßgabe, daß

a) wenn l 0 ist und $R^1$ Wasserstoff oder Alkyl mit 1 bis 6 Kohlenstoff-Atomen ist, $R^2$ von den aus Hydroxy, Alkoxy mit 1 bis 6 Kohlenstoff-Atomen und Amino ausgewählten Gruppen verschieden ist,

b) wenn l 0 ist und $R^1$ n-Butyl ist, B nicht Methylen und $R^2$ nicht Ethylamino ist,

c) wenn l 0 ist und $R^1$ Phenyl ist und B Methylen ist, $R^2$ von den aus Hydroxy, Ethoxy, Amino, Methylamino und Benzylamino ausgewählten Gruppen verschieden ist,

d) wenn l 0 ist und $R^1$ Phenyl ist und B Ethylmethylen ist, $R^2$ nicht Hydroxy, Ethoxy oder Amino ist,

e) wenn l 0 ist und $R^1$ Cyclohexyl ist und B Ethylen oder Methylmethylen ist, $R^2$ nicht Amino ist,

f) wenn l 0 ist und $R^1$ Cyclohexyl ist und B Dimethylmethylen ist, $R^2$ von den aus Amino, Diethylamino, Benzylamino und Morpholino ausgewählten Gruppen verschieden ist, und

g) wenn l 1 ist und $R^1$ Wasserstoff, eine Alkyl-Gruppe mit 1 bis 6 Kohlenstoff-Atomen, eine Cycloalkyl-Gruppe mit 3 bis 12 Kohlenstoff-Atomen oder Phenyl ist und A S ist und B Alkylen mit 1 bis 6 Kohlenstoff-Atomen ist und $R^2$ eine Gruppe,

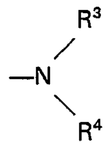

ist,

$R^3$ und $R^4$ nicht zusammen mit dem Stickstoff-Atom, an das sie gebunden sind, unter Bildung einer 1-Imidazolyl-Gruppe oder 1-Pyrrolyl-Gruppe kombiniert sind,
und pharmazeutisch unbedenkliches Salz desselben.

2. Verbindung nach Anspruch 1, dadurch gekennzeichnet, daß $R^2$ Hydroxy oder Alkoxy mit 1 bis 6 Kohlenstoff-Atomen ist.

3. Verbindung nach Anspruch 1, dadurch gekennzeichnet, daß $R^2$ eine Gruppe $—NR^3R^4$ ist, in der $R^3$ und $R^4$ die im Vorstehenden angegebenen Bedeutungen haben.

4. Verbindung nach Anspruch 3, dadurch gekennzeichnet, daß l 0 ist.

5. Verbindung nach den Ansprüchen 3 und 4, dadurch gekennzeichnet, daß $R^3$ und $R^4$ gleich oder verschieden sind und jeweils ein Alkyl-Gruppe mit 1 bis 6 Kohlenstoff-Atomen oder eine Cycloalkyl-Gruppe mit 3 bis 12 Kohlenstoff-Atomen, sind, die einen oder zwei Substituenten ausgewählt aus der aus Alkyloxy mit 1 bis 6 Kohlenstoff-Atomen, Alkyl mit 1 bis 6 Kohlenstoff-Atomen, Halogen, N,N-Dialkylamino, worin jede Alkyl-Struktureinheit 1 bis 6 Kohlenstoff-Atome besitzt, Nitro, Aminosulfonyl, Hydroxy und Alkanoyloxy mit 1 bis 6 Kohlenstoff-Atomen bestehenden Gruppe aufweisen kann.

6. Verbindung nach den Ansprüchen 3 bis 5, die ausgewählt ist aus N-Ethyl-N-cyclohexyl-5-(1-phenyl-1,2,3,4-tetrazol-5-yl)valeramid und N-Ethyl-N-cyclohexyl-5-(1-cyclohexyl-1,2,3,4-tetrazol-5-yl)valeramid.

7. Verbindung nach Anspruch 3, dadurch gekennzeichnet, daß l 1 ist.

8. Verbindung nach Anspruch 7, dadurch gekennzeichnet, daß A eine Alkylenthio-Gruppe mit 1 bis 6 Kohlenstoff-Atomen in der Alkylen-Struktureinheit ist, wobei die Thio-Gruppe mit der Gruppe B verbunden ist.

9. Verbindung nach den Ansprüchen 7 und 8, dadurch gekennzeichnet, daß $R^3$ und $R^4$ gleich oder verschieden sind und jeweils ein Alkyl-Gruppe mit 1 bis 6 Kohlenstoff-Atomen oder eine Cycloalkyl-Gruppe mit 3 bis 12 Kohlenstoff-Atomen sind, die einen oder zwei Substituenten ausgewählt aus der aus Alkyloxy mit 1 bis 6 Kohlenstoff-Atomen, Alkyl mit 1 bis 6 Kohlenstoff-Atomen, Halogen, N,N-Dialkylamino, worin jede Alkyl-Struktureinheit 1 bis 6 Kohlenstoff-Atome besitzt, Nitro, Aminosulfonyl, Hydroxy und Alkanoyloxy mit 1 bis 6 Kohlenstoff-Atomen bestehenden Gruppe aufweisen kann.

10. Verbindung nach Anspruch 7, dadurch gekennzeichnet, daß A Schwefel ist.

11. Verbindung nach Anspruch 7, dadurch gekennzeichnet, daß A eine Thio-Gruppe ist und $R^2$ eine disubstituierte Amino-Gruppe ist, wobei die Verbindung ausgewählt ist aus N,N-Dimethyl-4-(1-methyl-1,2,3,4-tetrazolyl-5-yl)thiobutyramid, N,N-Diethyl-4-(1-methyl-1,2,3,4-tetrazolyl-5-yl)thiobutyramid, N-Ethyl-N-(2-hydroxycyclohexyl)-4-(1-methyl-1,2,3,4-tetrazolyl-5-yl)thiobutyramid, N-Ethyl-N-(4-hydroxycyclohexyl)-4-(1-methyl-1,2,3,4-tetrazolyl-5-yl)thiobutyramid, N-Ethyl-N-cyclohexyl-3-(1-methyl-1,2,3,4-tetrazolyl-5-yl)thiopropionamid, N-Ethyl-N-cyclohexyl-5-(1-methyl-1,2,3,4-tetrazolyl-5-yl)thiovaleramid, N,N-Diethyl-4-(1-phenyl-1,2,3,4-tetrazolyl-5-yl)thiobutyramid, N-Ethyl-N-cyclohexyl-4-(1-phenyl-1,2,3,4-tetrazolyl-5-yl)thiobutyramid und N-Ethyl-N-cyclohexyl-3-(1-cyclohexyl-1,2,3,4-tetrazolyl-5-yl)thiopropion-amid.

12. Verbindung nach Anspruch 2, die Methyl-4-(1-methyl-1,2,3,4-tetrazol-5-yl)thiobutyrat ist.

13. Verbindung nach Anspruch 3, die N-[2-(3,4-Dimethoxyphenyl)ethyl]-4-(1-methyl-1,2,3,4-tetrazol-5-yl)thiobutyramid ist.

14. Anti-Ulcus-Zusammensetzung, enthaltend als wesentlichen aktiven Inhaltsstoff eine therapeutisch wirksame Menge eines Tetrazol-Derivats nach Anspruch 1 bis 13 mit einem herkömmlichen pharmazeutisch unbedenklichen Träger oder Verdünnungsmittel.

15. Verfahren zur Herstellung eines Tetrazol-Derivats der Formel

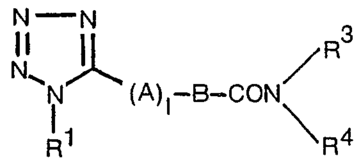

[Ia]

in der $R^1$, A, l, B, $R^3$ und $R^4$ die in Anspruch 1 angegebenen Bedeutungen haben, oder eines pharmazeutisch unbedenklichen Derivats desselben, dadurch gekennzeichnet, daß eine Carbonsäure-Verbindung der Formel

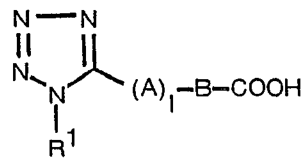

[II]

in der $R^1$, A, l und B die im Vorstehenden angegebenen Bedeutungen haben, oder ein reaktionsfähiges Derivat derselben mit einem Amin der Formel

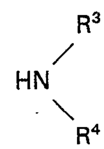

[III]

in der $R^3$ und $R^4$ die im Vorstehenden angegebenen Bedeutungen haben, oder einem reaktionsfähigen Derivat desselben umgesetzt wird.

16. Verfahren zur Herstellung eines Tetrazol-Derivats der Formel

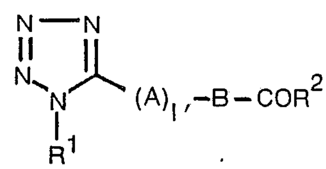

[Ib]

in der $R^1$, A und B die in Anspruch 1 angegebenen Bedeutungen haben, l' 1 ist und $R^2$ Hydroxy, Alkoxy mit 1 bis 6 Kohlenstoff-Atomen oder eine Gruppe

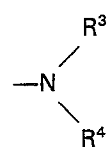

ist, in der $R^3$ und $R^4$ die in Anspruch 1 angegebenen Bedeutungen haben, oder eines pharmazeutisch unbedenklichen Derivats desselben, dadurch gekennzeichnet, daß ein 5-Mercaptotetrazol-Derivat der Formel

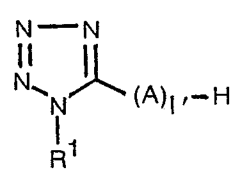

[IV]

in der $R^1$, A und l' die im Vorstehenden angegebenen Bedeutungen haben, mit einem Halogenoalkancarbonsäure-Derivat der Formel

$$X—B—COR^2 \qquad [V]$$

in der X Halogen ist und B und $R^2$ die im Vorstehenden angegebenen Bedeutungen haben, umgesetzt wird.

17. Verfahren zur Herstellung eines Tetrazol-Derivats der Formel

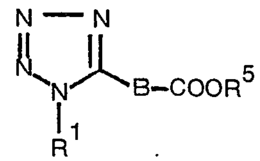

[Ic]

in der $R^1$ Wasserstoff, Alkyl mit 1 bis 6 Kohlenstoff-Atomen, Cycloalkyl mit 3 bis 12 Kohlenstoff-Atomen oder Phenyl ist, B ein Alkylen mit 1 bis 6 Kohlenstoff-Atomen ist und $R^5$ Alkyl mit 1 bis 6 Kohlenstoff-Atomen ist, wobei das Cycloalkyl und Phenyl an dem Ring einen oder zwei Substituenten ausgewählt aus der aus Alkoxy mit 1 bis 6 Kohlenstoff-Atomen, Alkyl mit 1 bis 6 Kohlenstoff-Atomen, Halogen, N,N-Dialkylamino, worin jede Alkyl-Struktureinheit 1 bis 6 Kohlenstoff-Atome besitzt, Nitro, Aminosulfonyl, Hydroxy und Alkanoyloxy mit 1 bis 6 Kohlenstoff-Atomen bestehenden Gruppe tragen können, oder eines pharmazeutisch unbedenklichen Salzes desselben, dadurch gekennzeichnet, daß ein Carbonsäureamid der Formel

$R^5OOC—B—CONHR^1$ [VIII]

in der $R^5$, B und $R^1$ die im Vorstehenden angegebenen Bedeutungen haben, mit Phosphorpentachlorid und anschließend mit Hydrogenazid umgesetzt wird.

18. Verfahren zur Herstellung eines Tetrazol-Derivats der Formel

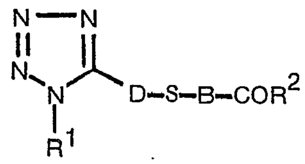

[Id]

in der $R^1$ und B die in Anspruch 1 angegebenen Bedeutungen haben, D Alkylen mit 1 bis 6 Kohlenstoff-Atomen ist und $R^2$ die in Anspruch 16 angegebene Bedeutung hat, oder eines pharmazeutisch unbedenklichen Salzes desselben, dadurch gekennzeichnet, daß eine Tetrazol-Verbindung der Formel

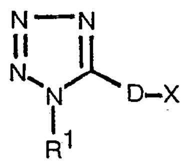

[XI]

in der $R^1$ und D die im Vorstehenden angegebenen Bedeutungen haben und X Halogen ist, mit einer Mercapto-Verbindung der Formel

$HS—B—COR^2$ [XII]

in der B und $R^2$ die im Vorstehenden angegebenen Bedeutungen haben, umgesetzt wird.

19. Verfahren zur Herstellung eines Tetrazol-Derivats der Formel

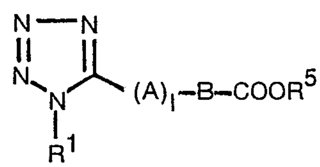

[Ie]

in der $R^1$, A, l und B die in Anspruch 1 angegebenen Bedeutungen haben und $R^5$ Alkyl mit 1 bis 6 Kohlenstoff-Atomen ist, dadurch gekennzeichnet, daß ein Tetrazol-Derivat der Formel

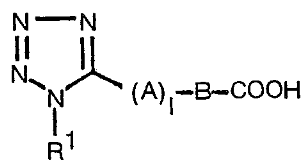

[If]

in der $R^1$, A, l, und B die im Vorstehenden angegebenen Bedeutungen haben, der Veresterung unterworfen wird.

20. Verfahren zur Herstellung eines Tetrazol-Derivats der Formel

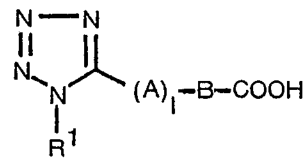

[If]

in der $R^1$, A, l und B die in Anspruch 1 angegebenen Bedeutungen haben, dadurch gekennzeichnet, daß ein Tetrazol-Derivat der Formel

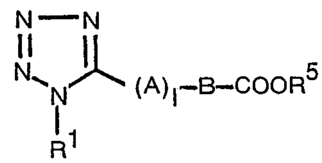

[Ie]

in der $R^1$, A, l und B die im Vorstehenden angegebenen Bedeutungen haben und $R^5$ Alkyl mit 1 bis 6 Kohlenstoff-Atomen ist, der Hydrolyse unterworfen wird.

**Revendications**

1. Dérivé du tétrazole de la formule:

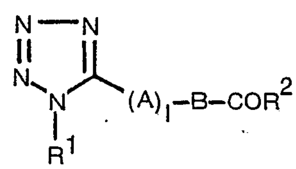

dans laquelle $R^1$ représente un atome d'hydrogène, un radical alkyle comportant de 1 à 6 atomes de carbone, un radical cycloalkyle comportant de 3 à 12 atomes de carbone ou le radical phényle; A représente un atome de soufre ou un radical alkylènethio dont le groupement alkylène comporte de 1 à 6 atomes de carbone et le groupe thio est lié au radical B; 1 est égal à 0 ou à 1; B représente un radical alkylène comportant de 1 à 6 atomes de carbone; $R^2$ représente le radical hydroxyle, un radical alcoxy comportant de 1 à 6 atomes de carbone ou un radical:

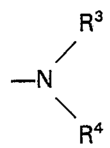

où $R^3$ et $R^4$ qui peuvent avoir des significations identiques ou différentes, représentent chacun un atome d'hydrogène, un radical alkyle comportant de 1 à 6 atomes de carbone, un radical cycloalkyle comportant de 3 à 12 atomes de carbone, le radical phényle, un radical cycloalkylalkyle dont le groupement cycloalkyle comporte de 3 à 8 atomes de carbone et le groupement alkyle comporte de 1 à 6 atomes de carbone, un radical phénylalkyle dont le groupement alkyle comporte de 1 à 6 atomes de carbone, un radical hydroxyalkyle possédant de 1 à 6 atomes de carbone, un radical hétérocyclique, saturé ou insaturé, choisi dans le groupe formé par les radicaux qui suivent: pyridyle, pyrrolyle, pyrrolidinyle, 3-pyrrolinyle, dihydropyridyle, pipéridinyle, 2-pyrazolinyle, 2-imidazolinyle, imidazolyle, imidazolidinyle, pyrazolyle, pyrimidinyle, pyridazinyle, pyrazinyle, pipérazinyle, oxazolyle, oxazolidinyle, isoxazolyle, 4H-1,4-oxazinyle, morpholinyle, thiazolyle, thiazolidinyle, isothiazolyle, 4H-1,4-thiazinyle, furyle, tétrahydrofuryle, 2H-pyranyle, 4H-pyranyle, 3,4-dihydro-2H-pyranyle, tétrahydropyranyle, thiényle, tétrahydrothiényle, thianyle et 1,4-dithianyle ou un radical alkyle comportant de 1 à 6 atomes de carbone, qui est substitué par le radical hétérocyclique saturé ou insaturé précité, ou bien les symboles $R^3$ et $R^4$ peuvent mutuellement se combiner à l'atome d'azote auquel ils sont attachés, avec ou sans atome d'azote ou d'oxygène intercalaire, pour former un radical hétérocyclique, saturé ou insaturé, choisi dans le groupe formé par les radicaux pipéridino, pyrrolidino, pipérazino, 1-imidazolyle, 1-pyrazolyle, 4,5-dihydropyrazol-1-yle, 1-pyrrolyle, 4H-1,4-oxazin-4-yle et 3-tétrahydrooxazolyle, lequel radical hétérocyclique peut être substitué par un groupe alkyle comportant de 1 à 6 atomes de carbone ou un groupe alcanoyle comportant de 1 à 6 atomes de carbone; lesdits radicaux cycloalkyle, phényle et phényl-alkyle dans les substituants $R^1$, $R^3$ et $R^4$, pouvant posséder, sur le cycle cycloalkyle ou phényle, un ou deux substituants choisis dans le groupe formé par les radicaux alcoxy comportant de 1 à 6 atomes de carbone, les radicaux alkyle comportant de 1 à 6 atomes de

carbone, les halogènes, les radicaux N,N-dialkylamino dont le groupement alkyle comporte de 1 à 6 atomes de carbone, les radicaux nitro, aminosulfonyl, hydroxyle et alcanoyloxy comportant de 1 à 6 atomes de carbone; avec la condition que:

a) lorsque 1 est égal à 0 et $R^1$ représente un atome d'hydrogène ou un radical alkyle comportant de 1 à 6 atomes de carbone, $R^2$ possède une signification autre que les radicaux choisis parmi les groupes hydroxyle, alcoxy possédant de 1 à 6 atomes de carbone et amino;

b) lorsque 1 est égal à 0 et $R^1$ représente un radical n-butyle, B ne représente pas le groupe méthylène et $R^2$ ne représente pas le radical éthylamino;

c) lorsque 1 est égal à 0 et $R^1$ représente le radical phényle et B représente le radical méthylène, $R^2$ soit différent des radicaux choisis parmi les groupes hydroxyle, éthoxy, amino, méthylamino et benzylamino;

d) lorsque 1 est égal à 0 et $R^1$ représente le radical phényle et B représente le radical éthylméthylène, $R^2$ ne désigne pas de radical hydroxyle, éthoxy ou amino;

e) lorsque 1 est égal à 0 et $R^1$ représente le radical cyclohexyle et B représente le radical éthylène ou méthylméthylène, $R^2$ ne désigne pas de radical amino;

f) lorsque 1 est égal à 0 et $R^1$ représente le radical cyclohexyle et B représente le radical diméthylméthylène, $R^2$ diffère des radicaux choisis parmi les groupes amino, diéthylamino, benzylamino et morpholino; et

g) lorsque 1 est égal à 1 et $R^1$ représente un atome d'hydrogène, un radical alkyle comportant de 1 à 6 atomes de carbone, un radical cycloalkyle comportant de 3 à 12 atomes de carbone ou le radical phényle et A représente S et B représente un radical alkylène comportant de 1 à 6 atomes de carbone et $R^2$ représente un radical

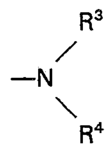 ,

$R^3$ et $R^4$ ne soient pas mutuellement combinés à l'atome d'azote auquel ils sont attachés pour former un radical 1-imidazolyle ou 1-pyrrolyle,
ainsi qu'un sel pharmaceutiquement acceptable d'un tel dérivé.

2. Composé suivant la revendication 1, caractérisé en ce que $R^2$ représente un radical hydroxyle ou alcoxy comportant de 1 à 6 atomes de carbone.

3. Composé suivant la revendication 1, caractérisé en ce que $R^2$ représente un radical $—NR^3R^4$, où $R^3$ et $R^4$ possèdent les significations qui leur ont été précédemment attribuées.

4. Composé suivant la revendication 3, caractérisé en ce que 1 est égal à 0.

5. Composé suivant l'une quelconque des revendications 3 et 4, caractérisé en ce que $R^3$ et $R^4$ possédent des significations indentiques ou différentes et représentent chacun un radical alkyle comportant de 1 à 6 atomes de carbone, ou un radical cycloalkyle comportant de 3 à 12 atomes de carbone, qui peut posséder un ou deux substituants choisis dans le groupe formé par les radicaux alcoxy comportant de 1 à 6 atomes de carbone, alkyle comportant de 1 à 6 atomes de carbone, les halogènes, les radicaux N,N-dialkylamino dont chaque groupement alkyle comporte de 1 à 6 atomes de carbone, nitro, aminosulfonyle, hydroxyle et alcanoyloxy comportant de 1 à 6 atomes de carbone.

6. Composé suivant l'une quelconque des revendications 3 à 5, caractérisé en ce qu'il est choisi parmi le N-éthyl-N-cyclohexyl-5-(1-phényl)-1,2,3,4-tétrazole-5-yl)valéramide et le N-éthyl-N-cyclohexyl-5-(1-cyclohexyl-1,2,3,4-tétrazole-5-yl)valéramide.

7. Composé suivant la revendication 3, caractérisé en ce que 1 est égal à 1.

8. Composé suivant la revendication 7, caractérisé en ce que A représente un radical alkylènethio dont le groupement alkylène comporte de 1 à 6 atomes de carbone, le radical thio étant lié au radical B.

9. Composé suivant l'une quelconque des revendications 7 et 8, caractérisé en ce que $R^3$ et $R^4$ qui possèdent des significations identiques ou différentes, représentent chacun un radical alkyle possédant de 1 à 6 atomes de carbone ou un radical cycloalkyle comportant de 3 à 12 atomes de carbone, qui peut posséder un ou deux substituants choisis dans le groupe formé par les radicaux alcoxy possédant de 1 à 6 atomes de carbone, alkyle comportant de 1 à 6 atomes de carbone, les halogènes, les radicaux N,N-dialkylamino dont chaque groupement alkyle comporte de 1 à 6 atomes de carbone, nitro, aminosulfonyle, hydroxyle et alcanoyloxy comportant de 1 à 6 atomes de carbone.

10. Composé suivant la revendication 7, caractérisé en ce que A représente un atome de soufre.

11. Composé suivant la revendication 7, caractérisé en ce que A représente un radical thio et $R^2$ désigne un radical amino bisubstitué, caractérisé en ce qu'il est choisi parmi les substances qui suivent: N,N-diméthyl-4-(1-méthy-1,2,3,4-tétrazol-5-yl)-thiobutyramide, N,N-diéthyl-4-(1-méthyl-1,2,3,4-tétrazol-5-yl)-thio-butyramide, N-éthyl-N-(2-hydroxycyclohexyl)-4-(1-méthyl-1,2,3,4-tétrazol-5-yl)-thio-butyramide, N-éthyl-N-(4-hydroxycyclohexyl)-4-(1-méthyl-1,2,3,4-tétrazol-5-yl)-thiobutyramide, N-éthyl-N-cyclohexyl-3-(1-méthyl-1,2,3,4-tétrazol-5-yl)-thio-propionamide, N-éthyl-N-cyclohexyl-5-(1-méthyl-1,2,3,4-tétrazol-5-yl)-thio-valéramide, N,N-diéthyl-4-(1-phényl-1,2,3,4-tétrazol-5-yl)-thio-butyramide, N-éthyl-N-cyclohexyl-4-(1-phényl-1,2,3,4-tétrazol-5-yl)-thio-butyramide et N-éthyl-N-cyclohexyl-4-(1-cyclohexyl-1,2,3,4-tétrazol-5-yl)-thio-butyramide.

12. Composé suivant la revendication 2, caractérisé en ce qu'il est le 4-(1-méthyl-1,2,3,4-tétrazole-5-yl)-thio-butyrate de méthyle.

13. Composé suivant la revendication 3, caractérisé en ce qu'il est le N-[2-(3,4-diméthoxyphényl)éthyl]-4-(1-méthyl-1,2,3,4-tétrazol-5-yl)thio-butyramide.

14. Composition antiulcères, caractérisée en ce qu'elle contient, à titre d'ingrédient actif essentiel, une quantité thérapeutiquement efficace d'un dérivé du tétrazole suivant l'une quelconque des revendications 1 à 13, ainsi qu'un diluant ou excipient ou véhicule pharmaceutiquement acceptable classique.

15. Procédé de préparation d'une dérivé du tétrazole de la formule:

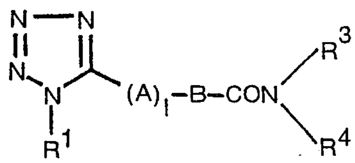

[Ia]

dans laquelle $R^1$, A, l, B, $R^3$ et $R^4$ possèdent les significations qui leur ont été attribuées dans la revendication 1, ou d'un sel pharmaceutiquement acceptable d'un tel dérivé, caractérisé en ce que l'on fait réagir un acide carboxylique qui répond à la formule:

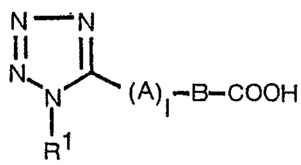

[II]

dans laquelle $R^1$, A, l et B possèdent les significations qui leur ont été précédemment attribuées, ou son dérivé réactif, sur une amine de la formule:

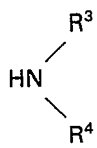

(III)

dans laquelle $R^3$ et $R^4$ possèdent les significations qui leur ont été précédemment attribuées, ou son dérivé réactif.

16. Procédé de préparation d'une dérivé du tétrazole de la formule:

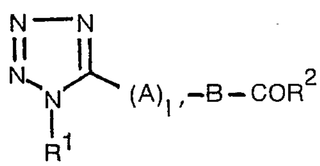

[Ib]

dans laquelle $R^1$, A et B possèdent les significations qui leur ont été attribuées dans la revendication 1, l' est égal à 1 et $R^2$ représente le radical hydroxyle, un radical alcoxy comportant de 1 à 6 atomes de carbone, ou un radical

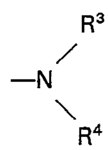

où $R^3$ et $R^4$ possèdent les significations qui leur ont été attribuées dans la revendication 1, ou d'un sel pharmaceutiquement acceptable d'un tel composé, caractérisé en ce que l'on fait réagir un dérivé ou 5-mercaptotétrazole de la formule:

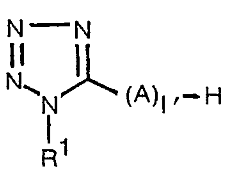

[IV]

dans laquelle $R^1$, A et l' possèdent les significations qui leur ont été précédemment attribuées, sur un dérivé d'un acide haloalcanecarboxylique de la formule:

$$X—B—COR^2 \qquad (V)$$

dans laquelle X représente un atome d'halogène, et B et $R^2$ possèdent les significations qui leur ont été précédemment attribuées.

17. Procédé de préparation d'un dérivé du tétrazole de la formule suivante:

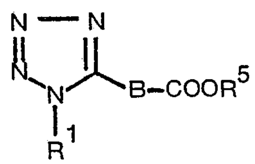
[Ic]

dans laquelle $R^1$ représente un atome d'hydrogène, un radical alkyle comportant de 1 à 6 atomes de carbone, un radical cycloalkyle comportant de 3 à 12 atomes de carbone ou le radical phényle; B représente un radical alkylène comportant de 1 à 6 atomes de carbone et $R^5$ représente un radical alkyle comportant de 1 à 6 atomes de carbone, lesdits radicaux cycloalkyle et phényle pouvant posséder, sur le cycle, un ou deux substituants choisis dans le groupe formé par les radicaux alcoxy comportant de 1 à 6 atomes de carbone, alkyle comportant de 1 à 6 atomes de carbone, les halogènes, les radicaux N,N-dialkylamino dont chaque groupement alkyle comporte de 1 à 6 atomes de carbone, nitro, aminosulfonyl, hydroxyle et alcanoyloxy comportant de 1 à 6 atomes de carbone, ou d'un sel pharmaceutiquement acceptable d'un tel dérivé, caractérisé en ce que l'on fait réagir un amide d'acide carboxylique de la formule:

$$R^5OOC—B—CONHR^1 \qquad (VIII)$$

dans laquelle $R^5$, B et $R^1$ possèdent les significations qui leur ont été précédemment attributées, sur le pentachlorure de phosphore et ensuite sur l'acide azothydrique.

18. Procédé de préparation d'un dérivé du tétrazole de la formule:

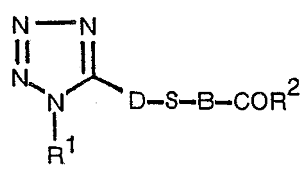
[Id]

dans laquelle $R^1$ et B possèdent les significations qui leur ont été attribuées dans la revendication 1, D représente un radical alkylène comportant de 1 à 6 atomes de carbone et $R^2$ possède les significations qui lui ont été attribuées dans la revendication 5 ou d'un sel pharmaceutiquement acceptable d'un tel dérivé, caractérisé en ce que l'on fait réagir un composé du tétrazole de la formule:

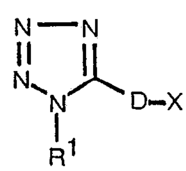
[XI]

dans laquelle $R^1$ et D possèdent les significations qui leur ont été précédemment attribuées et X représente un atome d'halogène, sur un composé du type mercapto de la formule:

$$HS—B—COR^2 \qquad (XII)$$

dans laquelle B et $R^2$ possèdent les significations qui leur ont été précédemment attribuées.

19. Procédé de préparation d'un dérivé du tétrazole de la formule:

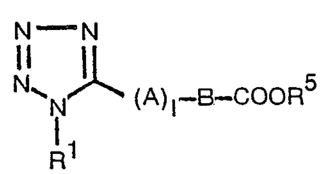
[Ie]

dans laquelle $R^1$, A, I et B possèdent les significations qui leur ont été attribuées dans la revendication 1, $R^5$

représente un radical alkyle comportant de 1 à 6 atomes de carbone, caractérisé en ce que l'on soumet un dérivé du tétrazole de la formule:

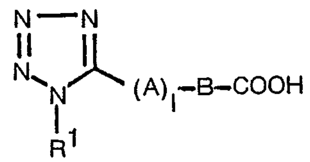

[If]

dans laquelle $R^1$, A, l et B possèdent les significations qui leur ont été précédemment attribuées, à une estérification.

20. Procédé de préparation d'un dérivé du tétrazole de la formule:

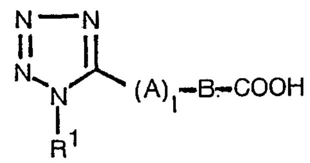

[If]

dans laquelle $R^1$, A, l et B possèdent les significations qui leur ont été attribuées dans la revendication 1, caractérisé en ce que l'on soumet un dérivé du tétrazole de la formule:

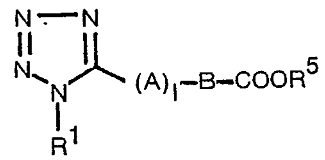

[Ie]

dans laquelle $R^1$, A, l et B possèdent les significations qui leur ont été précédemment attribuées, et $R^5$ représente un groupe alkyle comportant de 1 à 6 atomes de carbone, à une hydrolyse.